# EUROPEAN PATENT SPECIFICATION

(11) **EP 3 775 200 B1**
(45) Date of publication and mention of the grant of the patent: **29.12.2021**
(21) Application number: 19786850.8
(22) Date of filing: 09.10.2019
(51) Int. Cl.: C12N 15/10, C12Q 1/6806, C40B 40/08

(54) **NUCLEIC ACID LIBRARIES, PEPTIDE LIBRARIES AND USES THEREOF**
NUKLEINSÄUREBIBLIOTHEKEN, PEPTIDBIBLIOTHEKEN UND VERWENDUNGEN DAVON
BANQUES D'ACIDES NUCLÉIQUES, BANQUES DE PEPTIDES ET UTILISATIONS ASSOCIÉES

(30) Priority: 09.10.2018 GB 201816440
(43) Date of publication of application: 17.02.2021
(73) Proprietor: Phoremost Limited, Cambridge Cambridgeshire CB22 3AT (GB)
(72) Inventor: MUELLNER, Markus, Cambridgeshire CB22 3AT (GB); YARKER, Joanne L., Cambridgeshire CB22 3AT (GB)
(74) Representative: Stratagem IPM Limited
(86) International application number: PCT/GB2019/052859
(87) International publication number: WO 2020/074891

(56) References cited:
- WO-A1-2010/129310
- WO-A1-2017/109499
- WO-A2-2015/095355

## Description

The invention relates to libraries of nucleic acids that encode a plurality of peptides that represent fragments of naturally occurring proteins. Each peptide may be selected from, and collectively the plurality of peptides can be representative of, a proteome of a species or an organism, or such peptides may be selected from more than one proteome, and hence collectively the plurality can be representative of a metaproteome. The peptides may also be selected from proteins that are differentially expressed between cell or tissue types. The invention also relates to libraries of such peptides and to methods involving such libraries of nucleic acids and/or peptides. Described herein are computer readable media or data-processing systems comprising information relating to such libraries.

The identification of new therapeutic targets is a key starting point for drug discovery. Drug discovery efforts have traditionally been focused upon identifying classically-druggable targets, such as kinases, G-protein coupled receptors (GPCRs) and ion channels. However, such chemically facile targets do not always represent the most biologically relevant targets for therapeutic intervention. Drugging protein:protein interactions (PPIs) is of particular interest because these represent the predominant type of target involved in defective signalling pathways utilised by cancer cells and a large set of potentially actionable interfaces in human disease. Unfortunately, systematic attempts to drug PPIs and other 'undruggable' targets have been limited by technological restrictions, in large part due to limitations in current high-throughput DNA and RNA-based genomics technologies in being able to identify new druggable space at the proteome level.

Current genomics-based technologies that can identify candidate drug targets linked to disease biology using unbiased 'phenotypic' assays, have typically been performed using gene knock-outs (e.g., CRISPR), or at the transcriptomic level using RNAi. These approaches yield important information on which targets may represent important nodes in disease progression and therapeutic intervention in disease, but suffer a serious limitation: because they screen at the genetic, rather than protein-level, they cannot identify how to drug those targets or determine if those represent druggable candidates as an inherent part of the process. This is because such genetic screens remove target proteins rather than inhibiting them. To gain such crucial additional information on druggability, a new high-throughput proteome-level screening technology would need to be used; one that can handle the higher complexity of screening protein function (>300,000 unique protein transcripts and millions of unique PPIs) compared to gene function (~30,000 genes and their splice variants).

Recently, the systematic identification of novel drug target sites directly in the human proteome has gained a level of tractability and attention with the introduction of DNA-encoded, protein-fragment expression libraries that can be screened in high throughput in phenotypic assays (such as described in WO 2013/116903); often dubbed 'Protein-interference' (Protein-i). Such protein-fragment libraries, typically derived from diverse bacterial genomes, are composed of small self-folding sub-domains that form the evolutionary building blocks of larger proteins. When assembled into libraries for intracellular expression in mammalian cells, they represent a highly diverse collection of 3-dimensional shapes for docking to target proteins and exploring candidate novel druggable sites across the human proteome. Crucially, these protein fragments are small enough to describe discrete spatial sites in target proteins, and thus can be recapitulated with small-molecule drugs subsequently designed to match that shape. Moreover, because protein-fragment libraries describe many more shapes than current small-molecule libraries, this offers a more robust approach to informing the rational design of future small molecule drugs to novel validated targets.

While bacterial-derived protein-fragment libraries have been shown to be successful in Protein-i screening and are straightforward to generate by fragmenting and cloning into expression libraries due to bacterial genomes being composed mostly of coding sequence, they may, however, be under-powered in possessing a large fraction of protein-fragments that can functionally interact with mammalian (e.g. human) proteins, compared to using fragments of a mammalian or human proteome itself.

However, creating protein-fragment libraries directly from a mammalian (e.g. human) genome is complicated by the fact that DNA of higher organisms contains mostly non-coding sequences (>95% of human DNA is estimated to be non-coding) and a much larger absolute number of coding sequences and thus generally require a large degree of manual bespoke cloning to assemble fragments thereof into expression libraries for phenotypic screening.

Those bacterial-derived protein-fragment libraries described to date (e.g. in WO2013/116903) are obtained by mechanically shearing genomes and randomly inserting fragments into vectors. This leads to many fragments of random size that are either in frame (1:6 chance) or out of frame (5:6 chance) with the original gene in the bacterium. The same strategy would not work for eukaryotic organisms since most of their DNA is non-coding. In addition, bacterial-derived protein-fragment libraries such as these have no "inventory" i.e. because the sequences were randomly cloned it is not possible to say exactly what is contained within a given library other than by very deep sequencing.

These practical limitations have led to significant inertia in mining a potentially rich alternative vein of directly relevant protein-fold structural diversity in target-identification and validation screens in human cells.

Other screening approaches are described, for example, in WO 2001/086297. Here random short (40-mer and 20-mer) peptide phage display libraries are generated and used to find peptides that bind to a pre-selected target or a known, pre-identified consensus motif. This relies on existing disease targets being known/recognised and does not facilitate the identification of new targets. WO 2007/097923 discloses libraries, and means to produce such libraries, of peptide structures that are representative of the repertoire of protein structures existing in nature. However, such libraries are selected to comprise those peptides that are capable of folding or assuming their native conformations independently of artificial scaffolds or flanking sequences in the proteins from which they are derived.

WO 2010/129310 describes libraries of nucleic acids encoding peptides from proteins comprising an entire natural proteome (or known bioactive peptides) that in each case are expressed and secreted to the outside of the cell. The use of such libraries to isolate biologically-active secreted peptides ("BASPs") is described therein, as well as how such libraries are constructed, starting from high-throughput oligonucleotide synthesis but without disclosure of the sequences synthesised or the peptides encoded in such libraries. Indeed, little information is described therein on the amino acid sequences or other particular (e.g. advantageous) features of the peptides encoded, or of the nucleic acids encoding such peptides, nor on the method by which such peptides are selected for inclusion in (or exclusion from) such libraries, or the design (and features, e.g., of the sequence) of the nucleic acids selected to be synthesised for such libraries. Little further information is provided on such important matters of library design (e.g. in-silico construction) in the corresponding scientific publication for this technology (Natarajan et al, 2014; PNAS 111:E474).

There are also several known phage display libraries. WO 2015/095355 relates to detection of an antibody against a pathogen. It describes a phage display library comprising viral protein sequences. A related paper: Xu et al, 2015; Science 348, describes the VirScan technology and it is said to combine DNA microarray synthesis and bacteriophage display to create a uniform, synthetic representation of peptide epitopes comprising the human virome. An earlier publication by the same group, Larman et al, 2011, Nat Biotechnol 29:535, describes a similar approach but relates to a T7 "peptidome" phage display library which comprises peptides from a human genome (i.e. 36 amino acid peptides from approx. 24,000 unique ORFs from a human genome).

WO 2017/109499 relates to a method of identifying a peptide interaction site on a target protein wherein the target protein modulates the phenotype of a mammalian cell, using short expressed peptides (SEPs) such as short open reading frame (sORF) encoded peptides.

Accordingly, it is one object of the present invention to provide a library which encodes protein fragments/peptides wherein such a library can be used in screening methods including but not limited to PPI screens. In other objects, the present invention provides alternative, improved, simpler, cheaper and/or integrated means or methods that address one or more of these or other problems. An object underlying the present invention is solved by the subject matter as disclosed or defined anywhere herein, for example by the subject matter of the attached claims.

The figures show:
**Figure 1****:** depicts a screen for SEPs expressed in the HuPEx library that are able to overcome 6-thioguanine toxicity. Cells carrying a library of inserts that express SEPs are treated with 500nM 6-thioguanine for 6 days. Enrichment between 6-thioguanine treatment (n=3) and DMSO control (n=3) is shown.
**Figure 2****:** depicts a screen for SEPs across all three libraries (HuPEx, BugPEx, OmePEx) for SEPs able to selectively kill cells lacking the PTEN tumour suppressor gene.
**Figure 3****:** depicts the effect on MCF10A PTEN KO cells of a peptide (7-924) identified from the screen described in Example 4(2) expressed by pMOST25 compared to empty vector and a positive control (shRNA against NLK).
**Figure 4****:** depicts the experimental principle of the MNNG-induced parthantos phenotypic screen of Examples 9 and 10.
**Figure 5****:** depicts the relative abundance of DNA sequences encoding for SEPs from the HuPEx library that are present in a control aliquot of HuPEX-expressing HeLa cells before (D0) treatment with 6.7uM MNNG, and in a treatment aliquot of such HuPEX-expressing HeLa cells after 8 days (D8) of such MNNG treatment. Peptides showing a significantly increased relative abundance at D8 are marked by triangles.
**Figure 6****:** depicts the relative abundance of DNA sequences encoding for SEPs from the BugPEx library that are present in a control aliquot of BugPEx expressing HeLa cells before (D0) treatment with 6.7uM MNNG, and in a treatment aliquot of such BugPEx expressing HeLa cells after 8 days (D8) of such MNNG treatment. Axes are as for Figure 5, and peptides showing a significantly increased relative abundance at D8 are marked by triangles.
**Figure 7****:** depicts the relative abundance of DNA sequences encoding for SEPs from the OmePEx library that are present in a control aliquot of OmePEx-expressing HeLa cells before (D0) treatment with 6.7uM MNNG, and in a treatment aliquot of such OmePEx-expressing HeLa cells after 8 days (D8) of such MNNG treatment. Axes are as for Figure 5, and peptides showing a significantly increased relative abundance at D8 are marked by triangles.
**Figure 8** **and** **Figure 9****:** Phenotypic screen of PEx libraries for autophagy induction

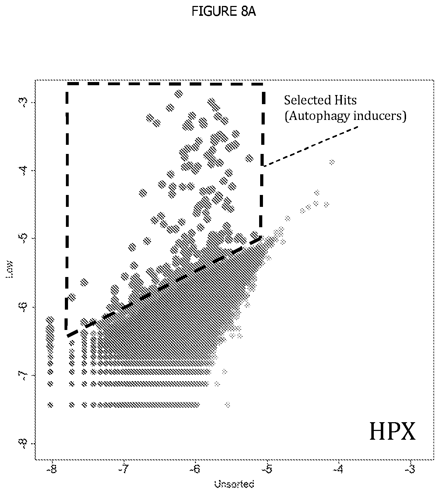

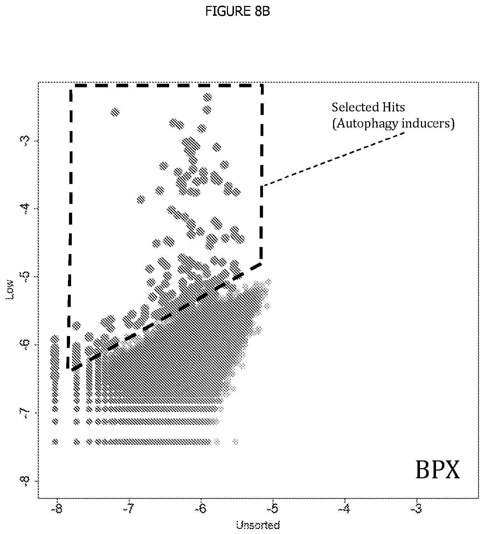

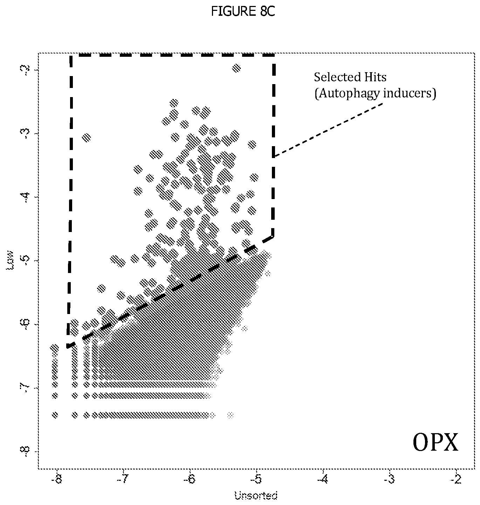

**Figure 8** **(A-C):** HEK293FT Cells were engineered to stably express an GFP-LC3/RFP-LC3DG autophagy reporter (Kaizuka et al Molecular Cell 2016). Subsequently, autophagy reporter cells were infected with the pooled HuPEx (HPX), BugPEx (BPX) and OmePEx (OPX) libraries and selected on puromycin. After selection, cells enriched in the low GFP-LC3 gate, compared to unsorted controls, were flow-sorted and peptide sequences were amplified and sent to NGS analysis as described previously. The graphs (Figure 8A (HPX), Figure 8B (BPX) and Figure 8C (OPX)) show the population of selected hits (autophagy inducers) in the marked region compared to control.

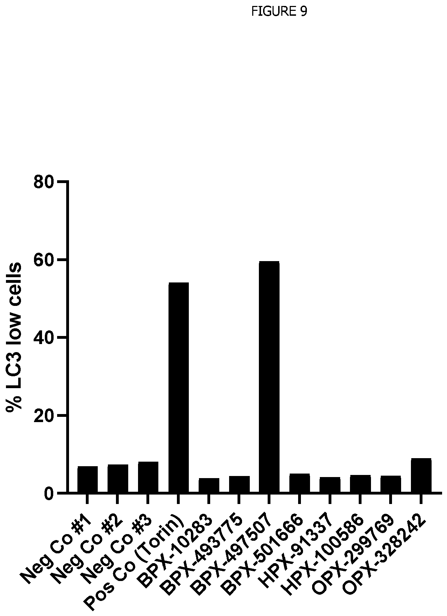

**Figure 9****:** Hits were re-run individually in a flow-cytometry experiment after infection with lentivirus carrying either control sequences or putative hits. A selection of candidates is shown with BPX-497507 representing a strong and robust hit able to induce autophagy as measured by GFP-LC3 reduction. Torin1 (250nM) is shown as a positive control.

The present invention, and particular non-limiting aspects and/or embodiments thereof, can be described in more detail as follows:
**In a first aspect, the present invention provides a library of nucleic acids,** each nucleic acid comprising a coding region of defined nucleic acid sequence encoding for a peptide having a length of between 25 and 110 amino acids, and having an amino acid sequence being a region of a sequence selected from the amino acid sequence of a naturally occurring protein of one or more organisms; wherein the library comprises nucleic acids that encode for a plurality of at least 10,000 different such peptides, and wherein the amino acid sequence of each of at least 50 of such peptides is a sequence region of the amino acid sequence of a different protein of a plurality of different such naturally occurring proteins, and wherein each peptide encoded by the library is predicted from its amino acid sequence to have an isoelectric point (pI) of greater than 8.0 or less than 6.0..

Suitably, a library in accordance with any aspect or embodiment of the invention comprises nucleic acids that encode a plurality of at least approximately 20,000, 50,000, 100,000, 200,000, 250,000, 300,000, 475,000 or 500,000 different such peptides. The library may also comprise nucleic acids that encode over 300,000 or 500,000 different such peptides. For example, in certain embodiments the library may comprise nucleic acids that encode for a plurality of at least 50,000 different such peptides, and wherein the amino acid sequence of each of at least 100 of such peptide is a sequence region of the amino acid sequence of at least 100 different naturally occurring proteins (or wherein, for clarity, in respect of each of the at least 100,000 different naturally occurring proteins, the library comprises one or more nucleic acids encoding for a peptide having an amino acid sequence being a sequence region of the amino acid sequence of such naturally occurring protein); in particular of such embodiments the library may comprise nucleic acids that encode for a plurality of at least 100,000 different such peptides, and wherein the amino acid sequence of each of at least 150 of such peptide is a sequence region of the amino acid sequence of at least 150 different naturally occurring proteins (or wherein, in respect of each of the at least 150 different naturally occurring proteins, the library comprises one or more nucleic acids encoding for a peptide having an amino acid sequence being a sequence region of the amino acid sequence of such naturally occurring protein). In another embodiment, the library may comprise nucleic acids that encode for a plurality of at least 10,000 different such peptides, and wherein the amino acid sequence of each of at least 1,000 of such peptides is a sequence region of the amino acid sequence of a different protein of such plurality of different naturally occurring proteins.

In one embodiment, the library may comprise nucleic acids that encode for a plurality of at least 200,000 different such peptides, and wherein the amino acid sequence of each of at least 20,000 of such peptide is a sequence region of the amino acid sequence of at least 20,000 different naturally occurring proteins; in particular of such embodiments the library may comprise nucleic acids that encode for a plurality of at least 300,000 different such peptides, and wherein the amino acid sequence of each of at least 25,000 of such peptide is a sequence region of the amino acid sequence of at least 25,000 different naturally occurring proteins. In one embodiment, the nucleic acids are present in the mixture in an amount that is proportional to the complexity and size of the genome or transcriptome of the organism of interest. In other embodiments, the number of different nucleic acids in a library in accordance with the invention may depend on the desired screening application and on the number of sequences that may be workable in a particular application. This may include a consideration of whether the library is for use as a primary or secondary screen, for example.

As used herein the term "different" in the context of the peptides encoded by the nucleic acids within the library of the invention means that any one peptide has at least one amino acid difference compared to any other peptide encoded within the library. In other words, each nucleic acid within the library encodes a unique peptide.

In one embodiment, a "naturally occurring protein" is one which has a sequence found in a reference proteome. Examples of reference proteomes and how to use information from reference proteomes are described herein. As used herein the term "different" in the context of the naturally occurring proteins means that any one such protein has at least one amino acid difference compared to any other such protein. In other words, each nucleic acid within the library encodes a unique peptide. Suitably, "different" naturally occurring proteins have an amino acid sequence identify that is less than about 98%, 95% or 92% sequence identity, such as less than about 95% or 90% sequence identity. In one suitable embodiment, "different" naturally occurring proteins have different entry numbers (or other identifier) of a database, such as having different UniProt identifiers. For example, the cyclin-dependent kinases with the UniProt (www.uniprot.org) identifiers P24941 and P11802 (human CDK2 and CDK4, respectively) are, in such an embodiment, "different" naturally occurring proteins.

Advantageously, each nucleic acid comprises a coding region of defined (or known) nucleic acid sequence encoding for a peptide. By "defined" (or "known") nucleic acid sequence is meant that the sequence of substantially all (e.g. each) nucleic acid sequence within the library is defined (or known). In particular, the library is non-random i.e. it does not represent a collection of random genomic sequences (which may or may not express peptide sequences), even if the genome sequence as a whole may have been determined (e.g. known), but the library has been designed, starting from protein sequences and, optionally filtered to generate a subset of nucleic acids encoding peptides with particular predicted features, in particular with specific and defined amino acid sequence. Thus, advantageously, the identity of substantially all (e.g. each) of the sequences in the library will be defined (or known) such that a library in accordance with the invention may have an inventory (that is, e.g., comprising or consisting of a pre-designated or pre-designed collection of individual members of defined (or known) sequences), even if may not be known which specific sequence is in which specific member of the library. This allows the sequences therein to be readily identified. Such libraries may be designed to have a desired complexity and/or to filter out undesired sequences, as described herein.

In some embodiments the nucleic acids of the library are synthetic (e.g., they have been - at least initially - generated by chemical rather than biological processes). Thus, suitably, the library provides synthetic or non-natural nucleic acids (and/or comprises non-natural sequences of nucleic acids). Such nucleic acids are, suitably, designed according to any one of the methods as described herein and synthesised according to methods available to those skilled in the art, particularly those high volume/high throughput methods included those described elsewhere herein. Importantly, such synthesised nucleic acids comprise design features which distinguish them from naturally occurring nucleic acids. Such design features include, for example, use of codon frequency tables to generate the nucleic acids such that the sequence of nucleotides making up the codons within the nucleic acids encoding the peptides do not represent those codons which would be found at that position in the amino acid sequence of the naturally occurring protein. In addition, the nucleic acids for use in the libraries in accordance with the invention may comprise restriction sites which would not be present in the naturally occurring nucleic acid sequences and would generate peptide sequences comprising additional amino acids which would not occur in the naturally occurring protein sequence. Suitably, the nucleic acids (e.g., the sequences thereof) for use in the libraries in accordance with the invention may be generated using the design principles set out in the methods as described herein.

In one embodiment of the library described herein, the library provides multiple nucleic acid sequences encoding multiple peptides derived from any one protein. That is, in respect of each of the different naturally occurring proteins the library comprises a plurality of (i.e., more than one) nucleic acids encoding for a peptide having an amino acid sequence being a sequence region of the amino acid sequence of such naturally occurring protein. Accordingly, with this meaning of such embodiment, the library described herein may be stated as relating to **a library of nucleic acids,** each nucleic acid comprising a coding region of defined (or known) nucleic acid sequence encoding for a peptide having a length of between 25 and 110 amino acids, and having an amino acid sequence being a region of a sequence selected from the amino acid sequence of a naturally occurring protein of one or more organisms; wherein the library comprises nucleic acids that encode for a plurality of at least 10,000 different such peptides, and wherein in respect of each of the different naturally occurring proteins, the library comprises one or more nucleic acids encoding for a peptide having an amino acid sequence being a sequence region of the amino acid sequence of such naturally occurring protein.

For example, in one such embodiment at least about 1% of the naturally occurring proteins a plurality of the nucleic acids encodes for different peptides from the amino acid sequences of such naturally occurring proteins. Suitably, in respect of at least about 5%, 10%, 25% or 50% of the naturally occurring proteins, a plurality of the nucleic acids encodes for different peptides from the amino acid sequences of such naturally occurring proteins. In other embodiments, a plurality of the nucleic acids encodes for different peptides from at least about 30, 35, 40, 45, 50, 55, 60, 65, 70, 75, 80, 85, 90, 95 or 100% of the naturally occurring proteins. In a particular embodiment, a plurality of the nucleic acids encodes for different peptides from the amino acid sequences of between about 90% and 100% of such naturally occurring proteins. Suitably a "different peptide" is one that has a different amino acid, such as differing by one or more (e.g. between about two and ten, five and 20, 15 and 40 or 30 and 50, or more than 50) amino acids.

In another embodiment, the invention provides a library of (e.g. synthetic) nucleic acids, wherein the plurality of the nucleic acids encodes for different peptides, the amino acid sequences of which are sequence regions spaced along the amino acid sequence of the naturally occurring protein. Suitably this spacing is chosen so as to generate a library which encodes a workable number of peptides and may be varied according to the desired number of peptides represented by the library. By "workable number" is meant a suitable number which can be generated economically whilst providing a suitable number for use in a screening application or method of choice. For example, for primary cell-based selection screens, a "workable number" may be larger (say, 300,000, 500,000 or even millions) that for secondary screening or array-based or pull-down screening (using say, tens of thousands up to about 250,000). Suitably, an expression library of nucleic acids described herein may, suitably, be used at a complexity of between about 250,000 and 500,000 different peptides (such as about 300,000); and a library of peptides expressed therefrom may be used for solid-phase screening at a complexity of greater than 500,000, such as greater than 750,000 1,000,000, 1,500,000 or 2,000,000 (or more). In other embodiments, a "workable number" may be smaller than such numbers of peptides (such as, in those embodiments directed to the "focused" section of naturally occurring proteins described below). Suitably, in such embodiments, an expression library of nucleic acids described herein may be used at a complexity of between about 5,000 and 250,000 different peptides (such as at least about 10,000), for example between about 10,000 and 25,000, 20,000 and 50,000, 40,000 and 100,000 or 100,000 and 200,000 different peptides.

Accordingly, in one embodiment, the sequence regions are spaced by a window of amino acids apart, or by multiples of such window, along the amino acid sequence of the naturally occurring protein wherein, the window is between 1, 5, 10, 15, 20, 25, 30, 35, 40 or 45 and about 55 amino acids; in particular wherein, the window is between about 2 and 40 amino acids, more particularly wherein, the window is between about 5 and about 20 amino acids; most particularly wherein the window of spacing is about 8, 10, 12 or 15 amino acids. In a suitable embodiment, the window used to space the sequence regions is the same number of amino acids (or multiples thereof) between each of the sequence regions along the amino acid sequence of the naturally occurring protein (and optionally, for each of the other naturally occurring proteins used to form the sequences of the peptides encoded by the nucleic acids of the library. In one further embodiment, the library may contain sequence regions spaced by multiples of such windows as one or more of the intervening peptides/amino acid sequences may get dropped from the selection process in view of not conforming to one or more of the various filtering criteria.

Suitably, the library is designed to be a complex library. By a "complex library" is meant one which has a multitude of different proteins and peptides represented therein, in particular one comprising sequence (or structurally) diverse peptides such as those defined from evolutionary diverse species. "Complexity" is therefore considered in terms of the numbers of proteins and peptides. Advantageously, the library of the present invention provides a much higher degree of complexity than those libraries available in the prior art. In one embodiment described herein the library comprises (synthetic) nucleic acids encoding at least 5,000 different peptides from at least 5,000 different proteins. In another embodiment described herein, the library of (synthetic) nucleic comprises nucleic acids encoding for at least 100,000 (or 20,000 or 50,000) different peptides from at least 10,000 (or 2,000 or 5,000, respectively) different naturally occurring proteins. It will be appreciated that the number of different peptides encoded by the library described herein may be upwards of at least 10,000, 20,000 50,000 or 100,000 different peptides encoded by at least 50, 75, 100 or 150 10,000 different naturally occurring proteins; the size of the library may be over at least 50,000, 100,000 or 250,000 peptides, for example. The number of different peptides and the number of different naturally occurring proteins from which they are derived may be varied according to the specific application. Accordingly, and as described above, the library may encode for at least approximately 5,000, 10,000, 50,000, 100,000, 200,000, 250,000, 300,000, 475,000 or 500,000 different such peptides, over 300,000 or 500,000 different such peptides (or, e.g. suitably for focused libraries, over 10,000 or 50,000 different such peptides); suitably wherein on average two or more (such as about 5, 8, 10 or 15) peptides are derived from the amino acid sequence of the same naturally occurring protein.

Given the complexity of certain embodiments of the library described herein - for example, those encoding for (or comprising) at least 10,000 (or 200,000 or 300,000) peptides from at least 1,000 or 10,000 (or 20,000 or 25,000, respectively) different naturally occurring proteins - the e.g. encoded peptides will in such embodiments, typically, represent those derived from a ***diverse*** selection of different naturally occurring proteins. Indeed, typically, the diverse selection of different naturally occurring proteins would (e.g. also or, optionally, only) comprise proteins that are non-secreted proteins and/or are not extracellular proteins, including those from a plurality of species. For example, the different naturally occurring proteins may comprise (e.g. also or, optionally, only) a set of proteins that include proteins other than (e.g. human and mouse) cytokines, chemokines, growth factors and their receptors (optionally, such other proteins as well as such cytokines, chemokines, growth factors and their receptors). In particular embodiments, the different naturally occurring proteins may comprise a set of proteins that include proteins that are cytoplasmic proteins (optionally, cytoplasmic protein as well as including non-cytoplasmic proteins such as also including secreted proteins and/or extracellular proteins).

In additional or alterative embodiments, a library described herein encodes for (or comprises) peptides that are not (previously) known to be - e.g., are not pre-selected to be - bioactive peptides, and/or could putatively modulate cellular responses by interacting with cell surface receptors.

In other embodiments of the library described herein - for example, those encoding for (or comprising) at least 50,000 (or 10,000 or 100,000) peptides from at least 100 (or 50 or 150, respectively) different naturally occurring proteins - the e.g. encoded peptides will in such embodiments, typically, represent those derived from a ***focused*** selection of different naturally occurring proteins. In such embodiment, the different naturally occurring proteins may be pre-selected from or a subset of a larger set of naturally occurring proteins (such as those from one or more reference proteomes) based on one more (e.g. pre-determined) criteria. In particular of such embodiments, all of the different naturally occurring proteins may fulfill such criteria, and each (e.g., all) of the (encoded) peptides of such a library is intended to have, or has, a sequence that is a sequence region of the amino acid sequence of a naturally occurring protein that fulfills such (e.g. pre-determined) criteria. One non-limiting example of such criteria can be that the naturally occurring proteins are those being secreted proteins and/or extracellular proteins, such as that (e.g. all of) the naturally occurring proteins may be cytokines, chemokines, growth factors and their receptors. Alternatively, such criteria may comprise that the naturally occurring proteins are not secreted proteins and/or are not extracellular proteins, such as that (e.g. all of) the naturally occurring proteins may not be cytokines, chemokines, growth factors and their receptors.

In alterative embodiments, criteria for (e.g., each and/or all of) the naturally occurring proteins from which the peptides encoded by (or comprised in) the library are derived, can include or consist of one or more of the following criteria; such protein/s is/are:
- From a subcellular compartment e.g. Cytoplasmic, nuclear, mitochondrial, cytoskeletal, or ribosomal
- One or more given enzymatic class. For example, a kinase, protease, esterase, or phosphatase;
- One or more given receptor type. For example, a G-coupled protein receptor or a nuclear hormone receptor;
- Membrane transport proteins and/or ion-channel proteins;
- Structural proteins;
- Transcription factors or DNA-binding proteins;
- DNA repair proteins. For example, a protein of the mismatch repair pathway;
- Involved in one or more (e.g. related or inter-related) cell-signaling/signal-transduction pathway. For example, the MAPK/ERK pathway, PI3K/Akt signaling, ErbB/HER signaling, mTOR signaling, NF-kappaB signaling or Jak/Stat (IL-6) receptor signaling;
- Interact (e.g., in-vivo, or as determined by laboratory procedures such as yeast 2-hybrid or affinity purification/mass spectrometry) with a given protein or at least one protein from a (e.g. functional) class of proteins. For example, interact with KRas or with a kinase (e.g., (ABL, BCL-ABL, SRC, KIT, PLK, CDKs, PLK, Aurora, MAPKs, JAKs, FLTs or EGFR);
- Associated with a given disease (e.g. hits from genome-wide association studies GWAS), such as cancer. For example, as BRCA1 and/or BRCA2 is associated with breast cancer, or as JAK2 is associated myeloproliferative neoplasms (Stadler et al 2010, J Clin Oncol 28:4255);
- Hits from functional screens e.g. CRISPR, RNAi, gene-trapping, mutagenesis, cDNA screens, PROTEINi screens.

In another embodiment, described herein is a library of (e.g. synthetic) nucleic acids, wherein each nucleic acid encodes a different peptide. Suitably, the mean number of nucleic acids that encode a (e.g. different) peptide from the naturally occurring proteins is greater than 1; in particular between about 1.01 and 1.5 such nucleic acids (peptides) per such protein (such as about 1.02, 1.05, 1.1, 1.2, 1.3 or 1.4), or is at least about 10 (or 2 or 5) nucleic acids (peptides) per such protein, in particular wherein the mean is between about 5 and about 2,000 such nucleic acids (peptides) per such protein or is between about 5 and about 1,000 such nucleic acids (peptides) per such protein (or between about 100 and about 1,500, or between about 250 and about 1,000; in particular between about 5 and about 100 or about 5 and about 50 ) such nucleic acids (peptides) per such protein. In some embodiments, the mean number of nucleic acids may be up to at least 500 nucleic acids, although it will be appreciated that the number of nucleic acids encoding different peptides for any particular protein will depend on the size of the protein as well as the size of the window by which the amino acid sequences are spaced. In a related embodiment, described herein is a library of (e.g. synthetic) nucleic acids, wherein 95% of the naturally occurring proteins represented therein are represented by between about 2 and about 20 (such as between about 3 and about 35 or 40) peptide sequences.

In another embodiment described herein, there is provided a library of (e.g. synthetic) nucleic acids, wherein the amino acid sequence of the naturally occurring protein is one selected from the group of amino acids sequences of proteins comprised in a reference proteome; in particular wherein the amino acid sequence of the naturally occurring protein is one selected from the group of amino acids sequences of (e.g., non-redundant) proteins comprised in a reference proteome.

In another embodiment, the reference proteome is one or more of the reference proteomes selected from the group of reference proteomes listed in any of Table A and/or Table B herein, or any updated versions of the proteomes listed therein.

In another embodiment, the amino acid sequences of the plurality of encoded peptides are sequence regions selected from amino acid sequences of naturally occurring proteins (or polypeptide chains or domains thereof) with a known three-dimensional structure; in particular wherein the naturally occurring protein (or polypeptide chain or domain thereof) is comprised in the Protein Data Bank (https://www.wwpdb.org), and optionally that has a Pfam annotation (http://pfam.xfam.org). In another embodiment, the sequence region selected from the amino acid sequence of the protein does not include an ambiguous amino acid of such amino acid sequence comprised in the reference proteome or the Protein Data Bank.

Suitably, the organism or species from which the amino acid sequence of a naturally occurring protein is selected to generate the (synthetic) nucleic acid library described herein is Homo sapiens.

In another embodiment described herein, the different naturally occurring proteins used to generate the library of (e.g. synthetic) nucleic acids are naturally occurring proteins of a plurality of different organisms or species. Suitably, a plurality of different species is selected from the group of (micro)organism species listed in Table A; in particular wherein the plurality of different species comprises at least 2, 3 or 5 (micro)organism species (such as at least about 10, 20, 25, or 50) across at least 2 (such as from at least about 3 or 5) of the phyla listed in Table A.

In another embodiment, a plurality of different species is selected from the group of species listed in Table B, in particular wherein the plurality of different species comprises at least 2, 3, 5 or 10 (such as at least about 20, 50, 100, 200, 300 or over 400) species listed in Table B, across at least about 3, 5 or 5 (in particular, across at least 5 or 6) of the sections of such table described therein as: archaea, bacteria, fungi, invertebrates, plants, protozoa, mammals and non-mammalian vertebrates.

Suitably, the plurality of different organisms or species is at least 10, 20, 50, 100 (in particular) or 250 different organisms or species. In some embodiments, the plurality may include up to about 20, 50, 100, 250 or 500 different organisms or species. Accordingly, a high diversity within the library may be achieved.

In another embodiment, the different naturally occurring proteins used to generate the library of (e.g. synthetic) nucleic acids described herein are naturally occurring proteins that are differentially expressed between two or more different cell populations (e.g. cell types) or tissue types. For example, the different naturally occurring proteins may be differentially expressed between a diseased and normal cell or tissue types; in particular wherein the different naturally occurring proteins are differentially expressed between human cancer cells and non-cancerous human cells, for example. In further embodiments, the different naturally occurring proteins are disease-specific; in particular wherein the different naturally occurring proteins are expressed by human cancer cells but not by non-cancerous human cells. In another embodiment, the different naturally occurring proteins may be differentially expressed between one cell population that has been infected by a pathogen or treated with a substance (such as a pathogenic substance or a drug) and a second cell population of the corresponding cell type that has not been so treated (or treated differently). For example, an inflammatory phenotype may be induced in a cell line by treatment with a pro-inflammatory substance such as phorbol myristate (PMA) and naturally occurring proteins that are differentially expressed in such treated population of cells compared to non-treated cells are considered. In another example differentially expressed naturally occurring proteins may be identified by comparison of protein expression between immune cells that have become exhausted (T cell exhaustion) compared to proficient immune cells (e.g. T cells).

In another embodiment, the library of (e.g. synthetic) nucleic acids described hereinis one in which the plurality of encoded peptides have diverse sequences; in particular wherein the plurality of encoded peptides differ in amino acid sequence from one another by at least 2 or 3 amino acids; in particular wherein the plurality of encoded peptides differ in amino acid sequence from one another by at least about 5, 8 or 10 amino acids. Suitably, the library of (synthetic) nucleic acids is one in which the diversity of the plurality of encoded peptides have a sequence similarity of less than about 90% or 80%; in particular have a sequence similarity of less than about 70%, 60% or 50%. Such sequence similarity may be determined using hierarchical clustering with CD-HIT (Fu et al 2012, Bioinformatics 28:3150; http://weizhongli-lab.org/cd-hit/). Suitable parameters for this are described in the Examples section herein.

In another embodiment the peptides encoded by the library described herein are predicted not to contain disordered regions; in particular wherein the prediction is as determined by SLIDER (Super-fast predictor of proteins with long intrinsically disordered regions; Peng et al, 2014; Proteins: Structure, Function and Bioinformatics 82:145; http://biomine.cs.vcu.edu/servers/SLIDER) and/or DISEMBL (Linding et al 2003, Structure 11:1453; http://dis.embl.de). Suitable parameters for this are described in the Examples section herein.

The peptides encoded by the library described herein are predicted to have an isoelectric point (pI) of less than about 6 and greater than about 8. Suitable examples of using the "pI" function of "R Peptide package" are described in the Examples section, herein.

In another embodiment, at least about 30%, 40% or 50% of the amino acid sequences of the peptides encoded by the library described herein are identical to a peptide in a different organism; in particular between about 30% and 70% of the amino acid sequences of the peptides encoded by the library described herein are identical to a peptide in a different organism.

In a further embodiment, each nucleic acid within the library described herein encodes for a peptide of the same length. It would be understood by the skilled person that this may include some limited variability. In other embodiments, the encoded peptide has a length of: between about 25 and about 100, 90, 85, 80, 75, 70, 65, 60, 55, 50 or 45; between about 30 and 110; between about 30 and about 100, 90, 85, 80, 75, 70, 65, 60, 55 or 50; between about 30 and about 100, 90, 85, 80, 75, 70, 65, 60 or 55; in particular wherein the encoded peptide has a length of between 30 and about 75; more particularly wherein the encoded peptide has a length of between about 35 and about 70 or between about 35 and about 50; and most particularly wherein the encoded peptide has a length of the encoded peptide that is between 35 and 60 amino acids, such as is 42, 43, 44, 45, 46, 47 or 48 amino acids in length. Suitably, the length of the peptides encoded by the library described herein will be determined by practical considerations such as the maximum limit of the oligonucleotide synthesis technique used (and taking into account the additional features added, as described herein, for example).

Suitably, in the library of (synthetic) nucleic acids described herein, the coding region encoding for the peptide uses the human codon frequency table set forth in Table 1.1. However, alterative human codon frequency tables may be used or, and depending on the species of the expression system in which the nucleic acid library is intended to be expressed, codon frequency tables of other species may be used. In one embodiment, the most frequent human codon is used to encode amino acids of the peptide. Accordingly, at least a portion of the nucleic acid sequences will be not the natural genomic sequence. Such sequences may also be modified further. In further embodiments, an alternative human codon is used to encode one or more amino acids of the peptide.

Suitably, "undesirable sequences or sub-sequences" are excluded from the library described herein. Such "undesirable sequences or sub-sequences" may include, for example, those sequences made from combinations of codons. Particular examples of such undesired sub-sequence include an internal Kozak sequence and/or a restriction enzyme site for a restriction enzyme intended to be used for the cloning of the resulting library, in each case generated from a combination of codons. Such undesirable sequences or sub-sequences can be avoided by using the next most commonly used (or another) codon in place of one or other of the codons in the combination. Thus, suitably, in the library of (synthetic) nucleic acids described herein, the coding region does not contain a combination of codons that forms an internal Kozak sequence. The person of ordinary skill will understand the term "Kozak sequence", and such meaning can include a sequence of nucleotide bases identified by the notation "(gcc)gccRccAUGG", where: (i) a lower-case letter denotes the most common base at a position where the base can nevertheless vary; (ii) upper-case letters indicate highly conserved bases, i.e. the "AUGG" sequence is constant or rarely, if ever, changes, with the exception being the IUPAC ambiguity code "R" which indicates that a purine (adenine or guanine) is always observed at this position (with adenine being believed to be more frequent); and (iii) the sequence in brackets "(gcc)" is of uncertain significance. In particular embodiments, a Kozak sequence is "CCATGG".

In some embodiments of the library of nucleic acids described herein, the following sequences are also undesirable sequences or subsequences: "GGATCC", "CTCGAG", "GGGGGG", "AAAAAA" ,"TTTTTT", "CCCCCC", sequences that cause issues with oligonucleotide synthesis or sequencing or PCR amplification, hairpin sequences, an in-frame STOP codon (except at the terminus).

In another embodiment, each nucleic acid in the library described herein further comprises one or more nucleic acid sequences 5' and/or 3' of the coding region that include at least one restriction enzyme recognition sequence; optionally with a linker nucleic acid sequence between the coding region and the restriction enzyme recognition sequence(s). Suitably, the coding region does not contain a combination of codons that forms the restriction enzyme recognition sequence(s) that is(are) included in the 5' and/or 3' nucleic acid sequence(s). As described above, this can be avoided by using the next most commonly used codon in place of one or other of the codons in the combination.

The nucleic acid library described herein (including also those embodiments where it is (e.g., initially) comprised by a synthetic nucleic acid library) may be amplified, propagated or otherwise maintained by non-chemical processes. For example, such library may be amplified by in-vitro enzymatic (e.g. biological) processes such as PCR or in-vitro transcription/translation; or may be replicated (and/or propagated/maintained) in-vivo processes such as being cloned into a vector which is replicated in a host cell (e.g., a bacterial or mammalian host cell).

In another embodiment, each nucleic acid in the library described herein further comprises additional sequences to enable amplification of the nucleic acid sequence (suitably, in each case not within the coding region that encodes for the peptide, such as 5' and/or 3' of the coding region and, optionally, the restriction enzyme recognition sequence(s)); in particular wherein such amplification is by PCR amplification.

In another embodiment, each nucleic acid in the described herein is cloned into a vector. The term "vector" will be art recognized, and includes the meaning of a nucleic acid that can be used to propagate, produce, maintain or introduce a nucleic acid comprised within it in/into a cell, such as for expression of a peptide or polypeptide encoded by a sequence comprising said nucleic acid. One type of vector is a plasmid, which refers to a linear or circular double-stranded DNA molecule into which additional nucleic acid segments can be ligated. Another type of vector is a viral vector (e.g., replication defective retroviruses, adenoviruses and adeno-associated viruses), wherein additional DNA segments can be introduced into the viral genome. Certain vectors are capable of autonomous replication in a cell, such as host cell, into which they are introduced (e.g., bacterial vectors comprising a bacterial origin of replication and episomal mammalian vectors). Other vectors (e.g., non-episomal mammalian vectors) integrate into the genome of a cell upon introduction into the cell and culturing under selective pressure, and thereby are replicated along with the genome. A vector can be used to direct the expression of a chosen peptide or polynucleotide in a cell; in particular a peptide encoded by a nucleic acid comprised in the library of the present invention. Suitably, when used to express a peptide of the present invention in a eukaryotic cell (such as a mammalian cell), the vector is a lentiviral vector, or is retroviral vector.

Suitably, each nucleic acid further comprises a start codon, a Kozak sequence, a stop codon and/or a nucleic acid sequence encoding a peptide tag, or wherein the vector comprises a start codon, a Kozak sequence, a stop codon and/or a nucleic acid sequence encoding a peptide tag operatively linked to the nucleic acid. Suitably, a peptide tag, if included, is one selected from the group consisting of: V4, FLAG, Strep/HA and GFP; in particular where the tag is a V4 tag or a FLAG tag.

In one embodiment, each nucleic acid is suitable for or capable of expressing a polypeptide comprising the encoded peptide.

In another embodiment, the polypeptide comprises the encoded peptide and further comprises an N-terminal methionine, one or more additional amino acids encoded by one or more restriction enzyme recognition sequence, and/or one or more peptide tags.

In another embodiment, the individual members (or subsets of members) of the library of (e.g. synthetic) nucleic acids described herein are in a pooled format (or form). Suitably, a library described herein in "pooled format" (or "pooled form") includes those where the individual members (or subset of members) thereof are in admixture with other members (or subsets); for example, a solution (or dried precipitate thereof) of such members contained in a single vessel, or a population of host cells containing recombinant vectors described herein.

Suitably, the individual members (or sub-sets of members) in the library described herein are spatially separated. A "spatially separated" library can be considered as a library in which a plurality of members (or sub-sets of members) of the library are physically separated, suitably in an ordered manner, from each other. Examples of a spatially separated library include those where individual members (or sub-sets of members) are comprised in individual wells of one or more mictrotitre plates, are arrayed on a solid surface or are bound (in an ordered manner) to a silicon wafer. In another embodiment, individual members (or sub-sets of members) in the library described herein are each individually addressable; that is they can be retrieved (e.g. without undue searching or screening) from the library. Suitable methods for addressing or interrogating the library described herein may include Next Generation Sequencing (NGS), PCR etc. Also, when the library is present in a spatially separated format (or form), the individual members (or sub-sets of members) may be "individually addressable" by knowing the spatial location of the applicable individual member (or sub-set). In either of these embodiments, use of a computer program, data file or database (such as those utilising a computer-readable medium or data-processing system) can facilitate the retrieval of individual members (or sub-sets of members) that are comprised in an individually addressable library described herein.

Suitably, a library described herein is not generated from cDNA.

**In another aspect, the invention provides a library of peptides** encoded by the library of (e.g. synthetic) nucleic acids in accordance with the invention. Suitably, the peptides are synthetic or recombinant. In one embodiment described herein, the individual members (or subsets of members) thereof are in a pooled format (or form). In another embodiment, the individual members (or subsets of members) thereof are spatially separate and or individually addressable.

In certain embodiments, any library of (synthetic) nucleic acids described herein may be comprised in an admixture with, may be an adjunct to (or otherwise combined with) or may be used together with, another library of nucleic acids that encode for peptides having a length of between about 25 and about 110 amino acids. For example, such other library may be one described in WO 2017/109499; in particular, such a library that encodes small Open Reading Frames (sORFs) such as that encodes at least 500, 1000, 1500 or about 2000 human sORFs.

Accordingly: (1) any library of peptides described herein may also be comprised in an admixture with, may be an adjunct to (or otherwise combined with) or may be used together with, another library of peptides that is encoded by such other library of nucleic acids; and (2) the uses, methods and processes involving a nucleic acids or peptide library described herein, can also involve its use in admixture with, as an adjunct to (or otherwise combined with) or use together with such other library of nucleic acids or peptides (respectively). For example, Example 4 herein, describes a screen using both a "HuPEx" library of the present disclosure optionally with a human sORF library described in WO2017/109499.

In some embodiments, the library of peptides is not a T7 display library, or is not a phage display library, or is not a display library. In some embodiments, the library is not a plurality of peptides derived from a plurality of (e.g. human) pathogens, such as from a plurality of viruses, bacteria or fungi that are, for example pathogenic to humans.

In another aspect described herein, is a container or carrier comprising the library of (e.g. synthetic) nucleic acids described herein and/or comprising the library of peptides described herein. Suitable containers include a vessel, (such as an Eppendorf tube), a microtiter plate or a silicon carrier.

In another aspect described herein, is a method of identifying at least one binding partner of a binding interaction between a peptide comprised in a library of the present invention to a target (in particular, to a protein target), the method comprising the steps of
- Exposing a peptide library of the invention to the target under conditions permitting binding between the target and at least one peptide of the library; and
- Identifying the binding peptide or the bound target.

In certain embodiments, the peptide library is exposed to the target by providing a library of nucleic acids described herein in a form (e.g. in host cells) and under conditions such that the library of peptides is expressed by the library of nucleic acids.

In another aspect (or embodiment of the above), disclosed herein is a method to identify the target and/or peptide; including, for example, elution of the peptide/nucleic acid, selection for cells expressing the peptide followed by e.g. PCR and sequencing identification. The target may be identified by, for example, pull-down mass-spectrometry. Such methods are described elsewhere herein, in WO2017/109499 and/or WO2013/116903.

**In a particular aspect, the invention also relates to a method of identifying a target protein that modulates a phenotype of a mammalian cell,** said method comprising exposing a population of in vitro cultured mammalian cells capable of displaying said phenotype to a library of nucleic acids of the present invention (or a library of peptides of the present invention), identifying in said cell population an alteration in said phenotype following said exposure, selection of said cells undergoing the phenotypic change and identifying a peptide encoded by (or a peptide of) such library that alters the phenotype of the cell, providing said peptide and identifying the cellular protein that binds to said peptide, said cellular protein being a target protein that modulates the phenotype of the mammalian cell. Suitable methods and phenotypic screens are described, for example, in WO2017/109499.

In certain embodiments of the invention, such method includes a further step of identifying a compound that binds to said target protein and displaces or blocks binding of said peptide. Such further step, thereby identifying a compound which binds to a target protein and displaces or blocks binding of said peptide wherein the compound modulates the phenotype of a mammalian cell.

Accordingly, described herein is a method of identifying a compound which binds to a target protein and displaces or blocks binding of a peptide wherein the compound modulates a phenotype of a mammalian cell, said method comprising the steps:
i. exposing a population of in vitro cultured mammalian cells capable of displaying said phenotype to a library of nucleic acids of the present invention or a library of peptides of the present invention;
ii. identifying a cell in the population that displays an alteration in said phenotype following said exposure;
iii. identifying a peptide encoded by (or of) such library that alters said phenotype of the cell;
iv. identifying a cellular protein that binds to said peptide, said cellular protein being a target protein that modulates said phenotype of the mammalian cell;
v. identifying a compound that binds to said target protein and displaces or blocks binding of said peptide.

**In one other aspect, the invention relates to a use** of: (a) a library of nucleic acids according to the present invention; and/or (b) a library of peptides according to the present invention, **to identify a peptide that binds to a target** (in particular, a protein target). In certain embodiments, the identified peptide modulates a phenotype of a mammalian cell.

Described herein is use of: (a) a library of nucleic acids according to the present invention; and/or (b) a library of peptides according to the present invention, to identify a target (in particular, a protein target) that modulates a phenotype of a mammalian cell.

**In yet another other aspect, the invention relates to a use** of: (a) a library of nucleic acids according to the present invention; and/or (b) a library of peptides according to the present invention, **to identify a compound** which binds to a target (in particular, a protein target) and, optionally, that displaces or blocks binding of a peptide to the target. In certain embodiments, the peptide and/or the compound modulates a phenotype of a mammalian cell.

Suitably in such aspects, methodologies for performing phenotypic screens using libraries (or peptides) described herein can range from: (1) pathway-specific readouts that use heterologous reporters (for example GFP or Luciferase) to register either total protein levels, protein localisation or ultimate pathway activity at the level of gene transcription in live cells; (2) registering endogenous protein levels, or their localisation, using antibodies or other affinity reagents, or pathway-specific transcriptional outputs using qPCR or RNA-sequencing in fixed 'non-living' cells; (3) high-content, or 'holistic' based readouts in live cells that are capable of registering specific 'destination' phenotypic readouts of therapeutic relevance, such as differentiation, senescence and cell-death, all of which are coordinated and can be specifically modulated by a complex interplay of multiple cellular pathways. In some embodiments described herein the assay readout method uses a GFP reporter, for instance as described in Kaizuka et al Molecular Cell 2016.

In a specific aspect described herein that covers 'holistic' phenotypic assays, Synthetic Lethality screening is of particular importance. Synthetic Lethality screening is an approach in which targets, for instance cancer targets, and candidate therapeutics are sought that can selectively impact tumour cells versus normal cells by exploiting unpredictable secondary points of weakness, which can occur in tumour cells as they heavily rewire their signalling pathways to support unrestrained cell proliferation. Such screens therefore must be performed in live cells and in an unbiased fashion by suppressing or modulating genes (using CRISPR), mRNA (using RNAi), or protein, or protein conformation (using Protein-i) in the cell and then determining whether a consistent negative impact on the overall growth or survival of a tumour cell type occurs; preferably one that harbours a specific genetic alteration(s) that occurs in a tumour situation versus a normal cell type. These direct 'holistic' cell-viability output based screens are performed using either large panels of genetically characterised tumour cells and normal cells to gain correlative information on tumour genotype- dependent responses, or more efficiently using specifically-engineered cell lines that are isogenic for a chosen mutant versus normal genotype that exists in cancer cells versus normal cells, respectively.

Suitably, in certain embodiments of uses and methods described herein - that involve the inventive libraries described herein - relate to phenotypes related to the modulation of cell-signalling pathways; in particular uses or method that involve the identification of peptides (e.g., from the libraries described herein) which modulate cell-signalling pathways and the identification of protein targets and surface sites on such proteins that participate in signal transduction and may be useful as drug targets to modulate cell-signalling pathways, in particular pathways which are active in cancer cells.

A cell signalling pathway is, suitably, a series of interacting factors in a cell that transmit an intracellular signal within the cell in response to an extracellular stimulus at the cell surface and leading to changes in cell phenotype. Transmission of signals along a cell signalling pathway typically results in the activation of one or more transcription factors which alter gene expression. Preferred cell signalling pathways for uses, methods or screens related to the present disclosure display aberrant activity in disease models, for example activation, up-regulation or mis-regulation in diseased cells, such a cancer cells. For example, a pathway may be constitutively activated (i.e. permanently switched on) in a cancer cell, or inappropriately activated by an extracellular ligand, for example in an inflammatory cell in rheumatoid arthritis.

A functional cell signalling pathway is typically considered as a pathway that is intact and capable of transmitting signals, if the pathway is switched on or activated, for example by an appropriate extracellular stimulus. An active cell-signalling pathway is typically considered a pathway that has been switched on, for example by an appropriate extracellular stimulus and is actively transmitting signals.

Suitable cell signalling pathways include any signalling pathway that results in a transcriptional event in response to a signal received by a cell.

Cell signalling pathways for investigation as described herein may include cell-signalling pathways that may be activated or altered in cancer cells, such as Ras/Raf, 20 Hedgehog, Fas, Wnt, Akt, ERK, TGFβ, EGF, PDGF, Met, PI3K and Notch signalling pathways.

Described herein is a computer-readable medium (for example, one for use in - e.g., one specifically adapted for use in - a screening method described herein) having information stored thereon comprising: (a) the nucleic acid sequences comprised in a library of nucleic acids of the present invention; and/or (b) the amino acid sequences of the peptides encoded by said nucleic acids.

Described herein is a data-processing system (for example, one for use in - e.g., one specifically adapted for use in - a screening method described herein) storing and/or processing information comprising: (a) the nucleic acid sequences comprised in a library of nucleic acids of the present invention; and/or (b) the amino acid sequences of the peptides encoded by said nucleic acids.

In view of the above, it will be appreciated that the present disclosure also relates to the following items:
Item 1: A library of nucleic acids as described in the first aspect of the invention, wherein the species of the organism is Homo sapiens.
Item 2: The library of nucleic acids according to item 1, wherein the different naturally occurring proteins are naturally occurring proteins of a plurality of organisms of different species.
Item 3: The library of nucleic acids according to item 2, wherein a plurality of different species is selected from the group of (micro) organism species listed in Table A; in particular wherein the plurality of different species comprises at least 20 (micro)organism species across at least 5 of the phyla listed in Table A.
Item 4: The library of nucleic acids according to item 2, wherein a plurality of different species is selected from the group of species listed in Table B, in particular wherein the plurality of different species comprises at least 50 of the species listed in Table B across at least 5 of the sections of Table B described therein as: archaea, bacteria, fungi, invertebrates, plants, protozoa, mammals and non-mammalian vertebrates.
Item 5: The library of nucleic acids according to any of items 2 to 4, wherein the plurality of different organisms is at least 100 different organisms.
Item 6: The library of nucleic acids according to any of items 1 to 4, wherein the different naturally occurring proteins are those that are differentially expressed between two or more different cell populations or tissue types.
Item 7: The library of nucleic acids according to item 6, wherein the different naturally occurring proteins are differentially expressed between a diseased and normal cell or tissue types; in particular wherein the different naturally occurring proteins are differentially expressed between human cancer cells and non-cancerous human cells.
Item 8: The library of nucleic acids according items 6 or 7, wherein the different naturally occurring proteins are disease-specific; in particular wherein the different naturally occurring proteins are expressed by human cancer cells but not by non-cancerous human cells.
Item 9: The library of nucleic acids according to any of items 1 to 8, wherein the plurality of encoded peptides have diverse sequences; in particular wherein the plurality of encoded peptides differ in amino acid sequence from one another by at least 2 or 3 amino acids; in particular wherein the plurality of encoded peptides differ in amino acid sequence from one another by at least about 5, 8 or 10 amino acids.
Item 10: The library of nucleic acids according to item 9, wherein the sequence similarity of the plurality of encoded peptides is less than about 80%.
Item 11: The library of nucleic acids according to any of items 1 to 10, wherein the peptides are predicted not to contain disordered regions.
Item 12: The library of nucleic acids according to any of items 1 to 11, wherein at least about 40% of the amino acid sequences of the peptide are identical to a peptide in a different organism.
Item 13: The library of nucleic acids according to any of items 1 to 12, wherein each nucleic acid encodes for a peptide of the same length.
Item 14: The library of nucleic acids according to any of items 1 to 13, wherein the encoded peptide has a length that is between 35 and 60 amino acids, such as is 42, 43, 44, 45, 46, 47 or 48 amino acids in length.
Item 15: The library of nucleic acids according to any of items 1 to 14, wherein the coding region encoding for the peptide uses the human codon frequency table set forth in Table 1.1.
Item 16: The library of nucleic acids of item 15, wherein the most frequent human codon is used to encode amino acids of the peptide.
Item 17: The library of nucleic acids of items 15 or 16, wherein an alternative human codon is used to encode one or more amino acids of the peptide.
Item 18: The library of nucleic acids according to any of items 1 to 17, wherein the coding region does not contain a combination of codons that forms an internal Kozak sequence; in particular wherein the Kozak sequence is ccatgg.
Item 19: The library of nucleic acids according to any of items 1 to 18, wherein each nucleic acid further comprises one or more nucleic acid sequences 5' and/or 3' of the coding region that include at least one restriction enzyme recognition sequence; optionally with a linker nucleic acid sequence between the coding region and the restriction enzyme recognition sequence(s).
Item 20: The library of nucleic acids according to item 19, wherein the coding region does not contain a combination of codons that forms the restriction enzyme recognition sequence(s) that is (are) included in the 5' and/or 3' nucleic acid sequence(s).
Item 21: The library of nucleic acids according to any of items 1 to 20, wherein each nucleic acid further comprises additional sequences to enable amplification of the nucleic acid sequence; in particular wherein such amplification is by PCR amplification.
Item 22: The library of nucleic acids according to any of items 1 to 21, wherein each nucleic acid is cloned into a vector.
Item 23: The library of nucleic acids according to any of items 1 to 22, wherein each nucleic acid further comprises a start codon, a Kozak sequence, a stop codon and/or a nucleic acid sequence encoding a peptide tag, or wherein the vector comprises a start codon, a Kozak sequence, a stop codon and/or a nucleic acid sequence encoding a peptide tag operatively linked to the nucleic acid.
Item 24: The library of nucleic acids of item 23, wherein each nucleic acid is suitable for or capable of expressing a polypeptide comprising the encoded peptide.
Item 25: The library of nucleic acids of item 24, wherein the polypeptide comprises the encoded peptide and further comprising an N-terminal methionine, one or more additional amino acids encoded by one or more restriction enzyme recognition sequence, and/or one or more peptide tags.
Item 26: The library of nucleic acids according to any of items 1 to 25, wherein the individual members thereof are in a pooled format.
Item 27: The library of nucleic acids according to any of items 1 to 26, wherein the individual members thereof are spatially separated.
Item 28: A library of peptides, encoded by the library of nucleic acids as described in another aspect of the invention, suitably according to any of items 1 to 27.
Item 29: The library of peptides of item 28, wherein the peptides are synthetic or recombinant.
Item 30: The library of peptides of item 28, wherein the individual members thereof are in a pooled format.
Item 31: The library of peptides of item 28, wherein the individual members thereof are spatially separated.
Item 32: A container or carrier comprising the library of nucleic acids as described in another aspect of the invention, suitably according to any of items 1 to 32.
Item 32a: A container or carrier comprising the library of peptides as described in another aspect of the invention, suitably according to any of items 29 to 31.
Item 33: The container or carrier of item 32 or 32a that is a microliter plate or a silicon carrier.
Item 34: A method of identifying at least one binding partner of a binding interaction between a peptide that is: (a) comprised in a library as described in another aspect of the invention, suitably according to item 28 to 31, or (b) is expressed by a library of nucleic acids as described in another aspect of the invention, suitably according to any of items 1 to 27, to a protein target; the method comprising the steps of:
   - exposing the invention to the protein target under conditions permitting binding between the target and at least one peptide of or expressed by the library; and
   - identifying the binding peptide or the bound target.
Item 35: A use of a library of nucleic acids as described in another aspect of the invention, suitably according to any of items 1 to 27, to identify a peptide that binds to a target protein; in particular wherein the peptide modulates a phenotype of a mammalian cell.
Item 36: A use of a library of nucleic acids as described in another aspect of the invention, suitably according to any of items 1 to 27, to identify a target protein that modulates a phenotype of a mammalian cell.
Item 37: A computer-readable medium having information stored thereon comprising: (a) the nucleic acid sequences comprised in a library of nucleic acids as described in another aspect of the invention, suitably according to any of items 1 to 27; and/or (b) the amino acid sequences of the peptides encoded by said nucleic acids.
Item 38: A data-processing system storing and/or processing information comprising: (a) the nucleic acid sequences comprised in a library of nucleic acids as described in another aspect of the invention, suitably according to any of items 1 to 27; and/or (b) the amino acid sequences of the peptides encoded by said nucleic acids.

In view of the above, it will be appreciated that the present disclosure also relates to the following clauses:
Clause 1: **A library of nucleic acids,** each nucleic acid comprising a coding region of defined nucleic acid sequence encoding for a peptide having a length of between 25 and 110 amino acids, and having an amino acid sequence being a region of a sequence selected from the amino acid sequence of a naturally occurring protein of one or more organisms; wherein the library comprises nucleic acids that encode for a plurality of at least 10,000 different such peptides, and wherein the amino acid sequence of each of at least 50 of such peptides is a sequence region of the amino acid sequence of a different protein of a plurality of different such naturally occurring proteins.
Clause 2: The library of nucleic acids of clause 1, wherein each of the plurality of different naturally occurring proteins fulfils one or more pre-determined criteria.
Clause 3: The library of nucleic acids of clause 2, wherein each of the plurality of naturally occurring proteins is associated with a given disease, such as cancer.
Clause 4: The library of nucleic acids of clause 3, wherein the disease is breast cancer.
Clause 5: The library of nucleic acids of clause 2, wherein each of the plurality of naturally occurring proteins is a cytoplasmic protein.
Clause 6: The library of nucleic acids of clause 5, wherein each of the plurality of naturally occurring proteins is a cytoplasmic kinase.
Clause 7: The library of nucleic acids of clause 2, wherein each of the plurality of naturally occurring proteins interacts with a given protein or at least one protein from a (functional) class of proteins.
Clause 8: The library of nucleic acids of clause 7, wherein each of the plurality of naturally occurring proteins interacts with KRas.
Clause 9: The library of nucleic acids of any one of clauses 1 to 8, wherein the library comprises nucleic acids that encode for a plurality of at least 50,000 different such peptides, and wherein the amino acid sequence of each of at least 100 of such peptide is a sequence region of the amino acid sequence of at least 100 different naturally occurring proteins; in particular wherein the library comprises nucleic acids that encode for a plurality of at least 100,000 different such peptides, and wherein the amino acid sequence of each of at least 150 of such peptide is a sequence region of the amino acid sequence of at least 150 different naturally occurring proteins.
Clause 10: The library of nucleic acids of any one of clauses 1 to 9, wherein the library comprises nucleic acids that encode for a plurality of at least 10,000 different such peptides, and wherein the amino acid sequence of each of at least 1,000 of such peptides is a sequence region of the amino acid sequence of a different protein of such plurality of different naturally occurring proteins.
Clause 11: The library of nucleic acids of any one of clauses 1 to 10, wherein the library comprises nucleic acids that encode for a plurality of at least 200,000 different such peptides, and wherein the amino acid sequence of each of at least 20,000 of such peptide is a sequence region of the amino acid sequence of at least 20,000 different naturally occurring proteins; in particular wherein the library comprises nucleic acids that encode for a plurality of at least 300,000 different such peptides, and wherein the amino acid sequence of each of at least 25,000 of such peptide is a sequence region of the amino acid sequence of at least 25,000 different naturally occurring proteins.
Clause 12: The library of nucleic acids of any one of clauses 1 or 11, wherein that in respect of at least about 1% of the naturally occurring proteins a plurality of the nucleic acids encodes for different peptides from the amino acid sequences of such naturally occurring proteins.
Clause 13: The library of nucleic acids of clause 12, wherein that in respect of at least about 50% of the naturally occurring proteins a plurality of the nucleic acids encodes for different peptides from the amino acid sequences of such naturally occurring proteins.
Clause 14: The library of nucleic acids of clause 13, wherein the plurality of the nucleic acids encodes for different peptides, and the amino acid sequences of which are sequence regions spaced along the amino acid sequence of the naturally occurring protein.
Clause 15: The library of nucleic acids of clause 14, wherein the sequence regions are spaced by a window of amino acids apart, or by multiples of such window, along the amino acid sequence of the naturally occurring protein wherein, the window is between 1 and about 55 amino acids; in particular wherein the window is between about 5 and about 20 amino acids; most particularly wherein the window of spacing is about 8, 10, 12 or 15 amino acids.
Clause 16: The library of nucleic acids of any one of clauses 1 to 14 comprising nucleic acids encoding for at least 100,000 different peptides from at least 10,000 different naturally occurring proteins.
Clause 17: The library of nucleic acids of any one of clauses 1 to 16, wherein each nucleic acid encodes a different peptide.
Clause 18: The library of nucleic acids of any one of clauses 1 to 17, wherein the mean number of nucleic acids that encode a different peptide from the naturally occurring proteins is greater than 1; in particular between about 1.01 and 1.5 such nucleic acids (peptides) per such protein.
Clause 19: The library of nucleic acids of clause 18, wherein the mean number of nucleic acids that encode a different peptide from the naturally occurring proteins is at least about 5 such nucleic acids (peptides) per such protein, in particular wherein the mean is between about 5 and about 2,000 such nucleic acids (peptides) per such protein or is between about 5 and about 1,000 nucleic acids (peptides) per such protein.
Clause 20: The library of nucleic acids of clause 19, wherein the mean number of nucleic acids that encode a different peptide from the naturally occurring proteins is between about 100 and about 1,500 such nucleic acids (peptides) per such protein or is between about 250 and about 1,000 such nucleic acids (peptides) per such protein.
Clause 21: The library of nucleic acids of clause 20, wherein the mean number of nucleic acids that encode a different peptide from the naturally occurring proteins is between about 5 and about 100 such nucleic acids (peptides) per such protein or is between about 5 and about 50 such nucleic acids (peptides) per such protein.
Clause 22: The library of nucleic acids of any one of clauses 1 to 21, wherein the amino acid sequence of the naturally occurring protein is one selected from the group of amino acids sequences of non-redundant proteins comprised in a reference proteome, suitably, the reference proteome is one or more of the reference proteomes selected from the group of reference proteomes listed in Table A and/or Table B, or an updated version of such reference proteome.
Clause 23: The library of nucleic acids of any one of clauses 1 to 22, wherein the amino acid sequences of the plurality of encoded peptides are sequence regions selected from amino acid sequences of naturally occurring proteins (or polypeptide chains or domains thereof) with a known three-dimensional structure; in particular wherein the naturally occurring protein (or polypeptide chain or domain thereof) is comprised in the Protein Data Bank, and optionally that has a Pfam annotation.
Clause 24: The library of nucleic acids of any one of clauses 22 to 23, wherein the sequence region selected from the amino acid sequence of the protein does not include an ambiguous amino acid of such amino acid sequence comprised in the reference proteome or the Protein Data Bank.
Clause 25: **A library of peptides** encoded by the library of nucleic acids of any one of clauses 1 to 24.

Described herein is a library of nucleic acids, each nucleic acid comprising a coding region of defined nucleic acid sequence encoding for a peptide having a length of between 25 and 110 amino acids, and having an amino acid sequence being a region of a sequence selected from the amino acid sequence of a naturally occurring protein of one or more organisms; wherein the library comprises nucleic acids that encode for a plurality of at least 10,000 different such peptides, and wherein the amino acid sequence of each of at least 50 of such peptides is a sequence region of the amino acid sequence of a different protein of a plurality of different such naturally occurring proteins, and wherein the isoelectric point (pI) of each encoded amino acid sequence is greater than 7.4 or less than 6.0, and/or wherein the nucleic acid sequence does not contain the sequences "GGATCC", and/or "CTCGAG", and/or wherein the nucleic acid sequence does not contain the sequences "GGGGGG" and/or "AAAAAA" and/or "TTTTTT" and/or "CCCCCC", and/or wherein the nucleic acid sequence does not contain sequences that cause issues with oligonucleotide synthesis or sequencing or PCR amplification , and/or wherein the nucleic acid sequence does not contain a hairpin sequence, and/or wherein the nucleic acid sequence does not contain an in-frame STOP codon (except at the terminus) and/or wherein the nucleic acid sequence does not contain a KOZAK sequence (except at the start), i.e. does not contain an internal KOZAK sequence.

The terms "of the [present] invention", "in accordance with the [present] invention", "according to the [present] invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments that are described.

**Table A: Database source of reference proteomes of an evolutionary diverse set of microbes**

| **NCBI ID** | **Species name** | **Domain** | **Phylum** | **Class** |
|---|---|---|---|---|
| NC_000854.2 | Aeropyrum pernix K1, Compl Gen | Archaea | Crenarchaeota | Thermoprotei |
| NC_002754.1 | Sulfolobus solfataricus P2, Compl Gen | Archaea | Crenarchaeota | Thermoprotei |
| NC_000917.1 | Archaeoglobus fulgidus DSM 4304, Compl Gen | Archaea | Euryarchaeota | Archaeoglobi |
| NC_006396.1 | Haloarcula marismortui ATCC 43049 Chrom I, complete sequence | Archaea | Eu rya rchaeota | Halobacteria |
| NC_006397.1 | Haloarcula marismortui ATCC 43049 Chrom II, complete sequence | Archaea | Eu rya rchaeota | Halobacteria |
| NC_006389.1 | Haloarcula marismortui ATCC 43049 plasmid pNG100, complete sequence | Archaea | Eu rya rchaeota | Halobacteria |
| NC_006390.1 | Haloarcula marismortui ATCC 43049 plasmid pNG200, complete sequence | Archaea | Eu rya rchaeota | Halobacteria |
| NC_006391.1 | Haloarcula marismortui ATCC 43049 plasmid pNG300, complete sequence | Archaea | Eu rya rchaeota | Halobacteria |
| NC_006392.1 | Haloarcula marismortui ATCC 43049 plasmid pNG400, complete sequence | Archaea | Eu rya rchaeota | Halobacteria |
| NC_006393.1 | Haloarcula marismortui ATCC 43049 plasmid pNG500, complete sequence | Archaea | Eu rya rchaeota | Halobacteria |
| NC_006394.1 | Haloarcula marismortui ATCC 43049 plasmid pNG600, complete sequence | Archaea | Eu rya rchaeota | Halobacteria |
| NC_006395.1 | Haloarcula marismortui ATCC 43049 plasmid pNG700, complete sequence | Archaea | Eu rya rchaeota | Halobacteria |
| NC_001869.1 | Halobacterium sp. NRC-1 plasmid pNRC100, complete sequence | Archaea | Eu rya rchaeota | Halobacteria |
| NC_002608.1 | Halobacterium sp. NRC-1 plasmid pNRC200 Compl Gen | Archaea | Eu rya rchaeota | Halobacteria |
| NC_002607.1 | Halobacterium sp. NRC-1, Compl Gen | Archaea | Euryarchaeota | Halobacteria |
| NC_001732.1 | Methanocaldococcus jannaschii DSM 2661 plasmid pDSM2661_1, complete sequence | Archaea | Euryarchaeota | Methanococci |
| NC_000961.1 | Pyrococcus horikoshii OT3 DNA, Compl Gen | Archaea | Euryarchaeota | Thermococci |
| NC_002689.2 | Thermoplasma volcanium GSS1 DNA, Compl Gen | Archaea | Euryarchaeota | Thermoplasmata |
| NC_009525.1 | Mycobacterium tuberculosis H37Ra, Compl Gen | Bacteria | Actinobacteria | Acti nobacteria |
| NC_003155.5 | Streptomyces avermitilis MA-4680 = NBRC 14893 DNA, Compl Gen | Bacteria | Acti nobacteria | Acti nobacteria |
| NC_004719.1 | Streptomyces avermitilis MA-4680 = NBRC 14893 plasmid SAP1 DNA, complete sequence | Bacteria | Acti nobacteria | Acti nobacteria |
| NC_002935.2 | Corynebacterium diphtheriae NCTC 13129, Compl Gen | Bacteria | Acti nobacteria | Actinomycetales |
| NC_004663.1 | Bacteroides thetaiotaomicron VPI-5482 Chrom, Compl Gen | Bacteria | Bacteroidetes | Bacteroidia |
| NC_004703.1 | Bacteroides thetaiotaomicron VPI-5482 plasmid p5482, complete sequence | Bacteria | Bacteroidetes | Bacteroidia |
| NC_002932.3 | Chlorobium tepidum TLS Chrom, Compl Gen | Bacteria | Chlorobi | Chlorobia |
| NC_001263.1 | Deinococcus radiodurans R1 Chrom 1, complete sequence | Bacteria | Deinococcus-Thermus | Deinococci |
| NC_001264.1 | Deinococcus radiodurans R1 Chrom 2, complete sequence | Bacteria | Deinococcus-Thermus | Deinococci |
| NC_000959.1 | Deinococcus radiodurans R1 plasmid CP1, complete sequence | Bacteria | Deinococcus-Thermus | Deinococci |
| NC_000958.1 | Deinococcus radiodurans R1 plasmid MP1, complete sequence | Bacteria | Deinococcus-Thermus | Deinococci |
| NC_001733.1 | Methanocaldococcus jannaschii DSM 2661 plasmid pDSM2661_2, complete sequence | Bacteria | Euryarchaeota | Methanococci |
| NC_000909.1 | Methanocaldococcus jannaschii DSM 2661, Compl Gen | Bacteria | Euryarchaeota | Methanococci |
| NC_005707.1 | Bacillus cereus ATCC 10987 plasmid pBc10987, complete sequence | Bacteria | Firmicutes | Bacilli |
| NC_003909.8 | Bacillus cereus ATCC 10987, Compl Gen | Bacteria | Firmicutes | Bacilli |
| NC_003210.1 | Listeria monocytogenes EGD-e Chrom, Compl Gen | Bacteria | Firmicutes | Bacilli |
| NC_008226.1 | Clostridioides difficile 630 plasmid pCD630, complete sequence | Bacteria | Firmicutes | Clostridia |
| NC_009089.1 | Clostridioides difficile 630, Compl Gen | Bacteria | Firmicutes | Clostridia |
| NC_002696.2 | Caulobacter crescentus CB15 Chrom, Compl Gen | Bacteria | Proteobacteria | Alpha Proteobacteria |
| NC_007493.1 | Rhodobacter sphaeroides 2.4.1 Chrom 1, complete sequence | Bacteria | Proteobacteria | Alphaproteobact eria |
| NC_007494.1 | Rhodobacter sphaeroides 2.4.1 Chrom 2, complete sequence | Bacteria | Proteobacteria | Alphaproteobact eria |
| NC_009007.1 | Rhodobacter sphaeroides 2.4.1 plasmid A, partial sequence | Bacteria | Proteobacteria | Alphaproteobact eria |
| NC_007488.1 | Rhodobacter sphaeroides 2.4.1 plasmid B, complete sequence | Bacteria | Proteobacteria | Alphaproteobact eria |
| NC_007489.1 | Rhodobacter sphaeroides 2.4.1 plasmid C, complete sequence | Bacteria | Proteobacteria | Alphaproteobact eria |
| NC_007490.1 | Rhodobacter sphaeroides 2.4.1 plasmid D, complete sequence | Bacteria | Proteobacteria | Alphaproteobact eria |
| NC_009008.1 | Rhodobacter sphaeroides 2.4.1 plasmid E, partial sequence | Bacteria | Proteobacteria | Alphaproteobact eria |
| NC_008767.1 | Neisseria meningitidis serogroup C FAM18 Compl Gen | Bacteria | Proteobacteria | Beta proteobacte ria |
| NC_002937.3 | Desulfovibrio vulgaris str. Hildenborough Chrom, Compl Gen | Bacteria | Proteobacteria | Delta proteobact eria |
| NC_005863.1 | Desulfovibrio vulgaris str. Hildenborough plasmid pDV, complete sequence | Bacteria | Proteobacteria | Delta proteobact eria |
| NC_002939.5 | Geobacter sulfurreducens PCA Chrom, Compl Gen | Bacteria | Proteobacteria | Delta proteobact eria |
| NC_002163.1 | Campylobacter jejuni subsp. jejuni NCTC 11168 = ATCC 700819 Chrom, Compl Gen | Bacteria | Proteobacteria | Epsilonproteoba cteria |
| NC_000915.1 | Helicobacter pylori 26695 Chrom, Compl Gen | Bacteria | Proteobacteria | Epsilonproteoba cteria |
| NC_009085.1 | Acinetobacter baumannii ATCC 17978 Chrom, Compl Gen | Bacteria | Proteobacteria | Gammaproteoba cteria |
| NC_009083.1 | Acinetobacter baumannii ATCC 17978 plasmid pAB1, complete sequence | Bacteria | Proteobacteria | Gammaproteoba cteria |
| NC_009084.1 | Acinetobacter baumannii ATCC 17978 plasmid pAB2, complete sequence | Bacteria | Proteobacteria | Gammaproteoba cteria |
| NC_000907.1 | Haemophilus influenzae Rd KW20 Chrom, Compl Gen | Bacteria | Proteobacteria | Gammaproteoba cteria |
| NC_002942.5 | Legionella pneumophila subsp. pneumophila str. Philadelphia 1 Chrom, Compl Gen | Bacteria | Proteobacteria | Gammaproteoba cteria |
| NC_002516.2 | Pseudomonas aeruginosa PAO1 Chrom, Compl Gen | Bacteria | Proteobacteria | Gammaproteoba cteria |
| NC_002950.2 | Porphyromonas gingivalis W83, Compl Gen | Bacteria | Proteobacteria | Porphyromonas gingivalis |
| NC_001318.1 | Borrelia burgdorferi B31 Chrom, Compl Gen | Bacteria | Spirochaetes | Spirochaetales |
| NC_001903.1 | Borrelia burgdorferi B31 plasmid cp26, complete sequence | Bacteria | Spirochaetes | Spirochaetales |
| NC_000948.1 | Borrelia burgdorferi B31 plasmid cp32-1, complete sequence | Bacteria | Spirochaetes | Spirochaetales |
| NC_000949.1 | Borrelia burgdorferi B31 plasmid cp32-3, complete sequence | Bacteria | Spirochaetes | Spirochaetales |
| NC_000950.1 | Borrelia burgdorferi B31 plasmid cp32-4, complete sequence | Bacteria | Spirochaetes | Spirochaetales |
| NC_000951.1 | Borrelia burgdorferi B31 plasmid cp32-6, complete sequence | Bacteria | Spirochaetes | Spirochaetales |
| NC_000952.1 | Borrelia burgdorferi B31 plasmid cp32-7, complete sequence | Bacteria | Spirochaetes | Spirochaetales |
| NC_000953.1 | Borrelia burgdorferi B31 plasmid cp32-8, complete sequence | Bacteria | Spirochaetes | Spirochaetales |
| NC_000954.1 | Borrelia burgdorferi B31 plasmid cp32-9, complete sequence | Bacteria | Spirochaetes | Spirochaetales |
| NC_001849.2 | Borrelia burgdorferi B31 plasmid lp17, complete sequence | Bacteria | Spirochaetes | Spirochaetales |
| NC_001851.2 | Borrelia burgdorferi B31 plasmid lp28-1, complete sequence | Bacteria | Spirochaetes | Spirochaetales |
| NC_001852.1 | Borrelia burgdorferi B31 plasmid lp28-2, complete sequence | Bacteria | Spirochaetes | Spirochaetales |
| NC_001853.1 | Borrelia burgdorferi B31 plasmid lp28-3, complete sequence | Bacteria | Spirochaetes | Spirochaetales |
| NC_001855.1 | Borrelia burgdorferi B31 plasmid Ip36, complete sequence | Bacteria | Spirochaetes | Spirochaetales |
| NC_001857.2 | Borrelia burgdorferi B31 plasmid Ip54, complete sequence | Bacteria | Spirochaetes | Spirochaetales |
| NC_000956.1 | Borrelia burgdorferi B31 plasmid Ip56, complete sequence | Bacteria | Spirochaetes | Spirochaetales |
| NC_000853.1 | Thermotoga maritima MSB8 Chrom, Compl Gen | Bacteria | Thermotogae | Thermotoga les |

**Table B: Database source of reference proteomes of an evolutionary diverse set of species**

| **RefSeq assembly accession ID** | **Species name** | **Assembly level** | **ftp path** (ftp://ftp.ncbi.nlm.nih.gov/genomes/all/GCF/...) |
|---|---|---|---|
| ***Archaea:*** | | | |
| GCF_000213215.1 | Acidianus hospitalis W1 | Compl Gen | 000/213/215/GCF_000213215.1_ASM21321v1 |
| GCF_000144915.1 | Acidilobus saccharovorans 345-15 | Compl Gen | 000/144/915/GCF_000144915.1_ASM14491v1 |
| GCF_000025665.1 | Aciduliprofundum boonei T469 | Compl Gen | 000/025/665/GCF_000025665.1_ASM2566v1 |
| GCF_000327505.1 | Aciduliprofundum sp. MAR08-339 | Compl Gen | 000/327/505/GCF_000327505.1_ASM32750v1 |
| GCF_000008665.1 | Archaeoglobus fulgidus DSM 4304 | Compl Gen | 000/008/665/GCF_000008665.1_ASM866v1 |
| GCF_000025285.1 | Archaeoglobus profundus DSM 5631 | Compl Gen | 000/025/285/GCF_000025285.1_ASM2528v1 |
| GCF_000385565.1 | Archaeoglobus sulfaticallidus PM70-1 | Compl Gen | 000/385/565/GCF_000385565.1_ASM38556v1 |
| GCF_000194625.1 | Archaeoglobus veneficus SNP6 | Compl Gen | 000/194/625/GCF_000194625.1_ASM19462v1 |
| GCF_000317795.1 | Caldisphaera lagunensis DSM 15908 | Compl Gen | 000/317/795/GCF_000317795.1_ASM31779v1 |
| GCF_000018305.1 | Caldivirga maquilingensis IC-167 | Compl Gen | 000/018/305/GCF_000018305.1_ASM1830v1 |
| GCF_000019605.1 | Candidatus Korarchaeum cryptofilum OPF8 | Compl Gen | 000/019/605/GCF_000019605.1_ASM1960v1 |
| GCF_000404225.1 | Candidatus Methanomassiliicoccus intestinalis Issoire-Mx1 | Compl Gen | 000/404/225/GCF_000404225.1_ASM40422v1 |
| GCF_000300255.2 | Candidatus Methanomethylophilus alvus Mx1201 | Compl Gen | 000/300/255/GCF_000300255.2_ASM30025v2 |
| GCF_000800805.1 | Candidatus Methanoplasma termitum | Compl Gen | 000/800/805/GCF_000800805.1_ASM80080v1 |
| GCF_000812185.1 | Candidatus Nitrosopelagicus brevis | Compl Gen | 000/812/185/GCF_000812185.1_ASM81218v1 |
| GCF_000299395.1 | Candidatus Nitrosopumilus sp. AR2 | Compl Gen | 000/299/395/GCF_000299395.1_ASM29939v1 |
| GCF_000955905.1 | Candidatus Nitrosotenuis cloacae | Compl Gen | 000/955/905/GCF_000955905.1_ASM95590v3 |
| GCF_000231015.2 | Desulfurococcus fermentans DSM 16532 | Compl Gen | 000/231/015/GCF_000231015.2_ASM23101v3 |
| GCF_000020905.1 | Desulfurococcus kamchatkensis 1221n | Compl Gen | 000/020/905/GCF_000020905.1_ASM2090v1 |
| GCF_000186365.1 | Desulfurococcus mucosus DSM 2162 | Compl Gen | 000/186/365/GCF_000186365.1_ASM18636v1 |
| GCF_000025505.1 | Ferroglobus placidus DSM 10642 | Compl Gen | 000/025/505/GCF_000025505.1_ASM2550v1 |
| GCF_000152265.2 | Ferroplasma acidarmanus fer1 | Compl Gen | 000/152/265/GCF_000152265.2_ASM15226v2 |
| GCF_000789255.1 | Geoglobus acetivorans | Compl Gen | 000/789/255/GCF_000789255.1_ASM78925v1 |
| GCF_001006045.1 | Geoglobus ahangari | Compl Gen | 001/006/045/GCF_001006045.1_ASM100604v1 |
| GCF_000196895.1 | Halalkalicoccus jeotgali B3 | Compl Gen | 000/196/895/GCF_000196895.1_ASM19689v1 |
| GCF_001305655.1 | Halanaeroarchaeum sulfurireducens | Compl Gen | 001/305/655/GCF_001305655.1_ASM130565v1 |
| GCF_000223905.1 | Haloarcula hispanica ATCC 33960 | Compl Gen | 000/223/905/GCF_000223905.1_ASM22390v1 |
| GCF_001488575.1 | Halobacterium hubeiense | Compl Gen | 001/488/575/GCF_001488575.1_Halobacterium _hubeiense_JI20-1 |
| GCF_000069025.1 | Halobacterium salinarum R1 | Compl Gen | 000/069/025/GCF_000069025.1_ASM6902v1 |
| GCF_000230955.2 | Halobacterium sp. DL1 | Compl Gen | 000/230/955/GCF_000230955.2_ASM23095v3 |
| GCF_000306765.2 | Haloferax mediterranei ATCC 33500 | Compl Gen | 000/306/765/GCF_000306765.2_ASM30676v2 |
| GCF_000025685.1 | Haloferax volcanii DS2 | Compl Gen | 000/025/685/GCF_000025685.1_ASM2568v1 |
| GCF_000172995.2 | Halogeometricum borinquense DSM 11551 | Compl Gen | 000/172/995/GCF_000172995.2_ASM17299v2 |
| GCF_000023965.1 | Halomicrobium mukohataei DSM 12286 | Compl Gen | 000/023/965/GCF_000023965.1_ASM2396v1 |
| GCF_000224475.1 | halophilic archaeon DL31 | Compl Gen | 000/224/475/GCF_000224475.1_ASM22447v1 |
| GCF_000217715.1 | Halopiger xanaduensis SH-6 | Compl Gen | 000/217/715/GCF_000217715.1_ASM21771v1 |
| GCF_000237865.1 | Haloquadratum walsbyi C23 | Compl Gen | 000/237/865/GCF_000237865.1_ASM23786v1 |
| GCF_000470655.1 | Halorhabdus tiamatea SARL4B | Compl Gen | 000/470/655/GCF_000470655.1_HATI1 |
| GCF_000023945.1 | Halorhabdus utahensis DSM 12940 | Compl Gen | 000/023/945/GCF_000023945.1_ASM2394v1 |
| GCF_000022205.1 | Halorubrum lacusprofundi ATCC 49239 | Compl Gen | 000/022/205/GCF_000022205.1_ASM2220v1 |
| GCF_000517625.1 | Halostagnicola larsenii XH-48 | Compl Gen | 000/517/625/GCF_000517625.1_ASM51762v1 |
| GCF_000025325.1 | Haloterrigena turkmenica DSM 5511 | Compl Gen | 000/025/325/GCF_000025325.1_ASM2532v1 |
| GCF_000015145.1 | Hyperthermus butylicus DSM 5456 | Compl Gen | 000/015/145/GCF_000015145.1_ASM1514v1 |
| GCF_000017945.1 | Ignicoccus hospitalis KIN4/I | Compl Gen | 000/017/945/GCF_000017945.1_ASM1794v1 |
| GCF_000204925.1 | Metallosphaera cuprina Ar-4 | Compl Gen | 000/204/925/GCF_000204925.1_ASM20492v1 |
| GCF_000016605.1 | Metallosphaera sedula DSM 5348 | Compl Gen | 000/016/605/GCF_000016605.1_ASM1660v1 |
| GCF_000762265.1 | Methanobacterium form icicum | Compl Gen | 000/762/265/GCF_000762265.1_ASM76226v1 |
| GCF_000191585.1 | Methanobacterium lacus | Compl Gen | 000/191/585/GCF_000191585.1_ASM19158v1 |
| GCF_000214725.1 | Methanobacterium paludis | Compl Gen | 000/214/725/GCF_000214725.1_ASM21472v1 |
| GCF_000499765.1 | Methanobacterium sp. MB1 | Compl Gen | 000/499/765/GCF_000499765.1_Methanobacter ium |
| GCF_001477655.1 | Methanobrevibacter millerae | Compl Gen | 001/477/655/GCF_001477655.1_ASM147765v1 |
| GCF_000024185.1 | Methanobrevibacter ruminantium M1 | Compl Gen | 000/024/185/GCF_000024185.1_ASM2418v1 |
| GCF_000016525.1 | Methanobrevibacter smithii ATCC 35061 | Compl Gen | 000/016/525/GCF_000016525.1_ASM1652v1 |
| GCF_000739065.1 | Methanocaldococcus bathoardescens | Compl Gen | 000/739/065/GCF_000739065.1ASM73906v1 |
| GCF_000023985.1 | Methanocaldococcus fervens AG86 | Compl Gen | 000/023/985/GCF_000023985.1_ASM2398v1 |
| GCF_000092305.1 | Methanocaldococcus infernus ME | Compl Gen | 000/092/305/GCF_000092305.1_ASM9230v1 |
| GCF_000025525.1 | Methanocaldococcus sp. FS406-22 | Compl Gen | 000/025/525/GCF_000025525.1_ASM2552v1 |
| GCF_000024625.1 | Methanocaldococcus vulcanius M7 | Compl Gen | 000/024/625/GCF_000024625.1_ASM2462v1 |
| GCF_000063445.1 | Methanocella arvoryzae MRE50 | Compl Gen | 000/063/445/GCF_000063445.1_ASM6344v1 |
| GCF_000251105.1 | Methanocella conradii HZ254 | Compl Gen | 000/251/105/GCF_000251105.1_ASM25110v1 |
| GCF_000013725.1 | Methanococcoides burtonii DSM 6242 | Compl Gen | 000/013/725/GCF_000013725.1_ASM1372v1 |
| GCF_000970325.1 | Methanococcoides methylutens MM1 | Compl Gen | 000/970/325/GCF_000970325.1_ASM97032v1 |
| GCF_000017185.1 | Methanococcus aeolicus Nankai-3 | Compl Gen | 000/017/185/GCF_000017185.1_ASM1718v1 |
| GCF_000017225.1 | Methanococcus maripaludis C7 | Compl Gen | 000/017/225/GCF_000017225.1_ASM1722v1 |
| GCF_000220645.1 | Methanococcus maripaludis X1 | Compl Gen | 000/220/645/GCF_000220645.1_ASM22064v1 |
| GCF_000017165.1 | Methanococcus vannielii SB | Compl Gen | 000/017/165/GCF_000017165.1_ASM1716v1 |
| GCF_000006175.1 | Methanococcus voltae A3 | Compl Gen | 000/006/175/GCF_000006175.1_ASM617v2 |
| GCF_000015765.1 | Methanocorpusculum labreanum Z | Compl Gen | 000/015/765/GCF_000015765.1_ASM1576v1 |
| GCF_000304355.2 | Methanoculleus bourgensis MS2 | Compl Gen | 000/304/355/GCF_000304355.2_Mb_MS2 |
| GCF_000015825.1 | Methanoculleus marisnigri JR1 | Compl Gen | 000/015/825/GCF_000015825.1_ASM1582v1 |
| GCF_000196655.1 | Methanohalobium evestigatum Z-7303 | Compl Gen | 000/196/655/GCF_000196655.1_ASM19665v1 |
| GCF_000025865.1 | Methanohalophilus mahii DSM 5219 | Compl Gen | 000/025/865/GCF_000025865.1_ASM2586v1 |
| GCF_000147875.1 | Methanolacinia petrolearia DSM 11571 | Compl Gen | 000/147/875/GCF_000147875.1_ASM14787v1 |
| GCF_000306725.1 | Methanolobus psychrophilus R15 | Compl Gen | 000/306/725/GCF_000306725.1_ASM30672v1 |
| GCF_000328665.1 | Methanomethylovorans hollandica DSM 15978 | Compl Gen | 000/328/665/GCF_000328665.1_ASM32866v1 |
| GCF_000017625.1 | Methanoregula boonei 6A8 | Compl Gen | 000/017/625/GCF_000017625.1_ASM1762v1 |
| GCF_000327485.1 | Methanoregula formicica SMSP | Compl Gen | 000/327/485/GCF_000327485.1_ASM32748v1 |
| GCF_000204415.1 | Methanosaeta concilii GP6 | Compl Gen | 000/204/415/GCF_000204415.1_ASM20441v1 |
| GCF_000235565.1 | Methanosaeta harundinacea 6Ac | Compl Gen | 000/235/565/GCF_000235565.1_ASM23556v1 |
| GCF_000217995.1 | Methanosalsum zhilinae DSM 4017 | Compl Gen | 000/217/995/GCF_000217995.1_ASM21799v1 |
| GCF_000007345.1 | Methanosarcina acetivorans C2A | Compl Gen | 000/007/345/GCF_000007345.1_ASM734v1 |
| GCF_000970305.1 | Methanosarcina barkeri 3 | Compl Gen | 000/970/305/GCF_000970305.1_ASM97030v1 |
| GCF_000970025.1 | Methanosarcina barkeri MS | Compl Gen | 000/970/025/GCF_000970025.1_ASM97002v1 |
| GCF_000970285.1 | Methanosarcina horonobensis HB-1 = JCM 15518 | Compl Gen | 000/970/285/GCF_000970285.1_ASM97028v1 |
| GCF_000970265.1 | Methanosarcina lacustris Z-7289 | Compl Gen | 000/970/265/GCF_000970265.1_ASM97026v1 |
| GCF_000970205.1 | Methanosarcina mazei S-6 | Compl Gen | 000/970/205/GCF_000970205.1_ASM97020v1 |
| GCF_000970085.1 | Methanosarcina siciliae T4/M | Compl Gen | 000/970/085/GCF_000970085.1_ASM97008v1 |
| GCF_000970045.1 | Methanosarcina sp. MTP4 | Compl Gen | 000/970/045/GCF_000970045.1_ASM97004v1 |
| GCF_000969965.1 | Methanosarcina sp. WWM596 | Compl Gen | 000/969/965/GCF_000969965.1_ASM96996v1 |
| GCF_000969885.1 | Methanosarcina thermophila TM-1 | Compl Gen | 000/969/885/GCF_000969885.1_ASM96988v1 |
| GCF_000969905.1 | Methanosarcina vacuolata Z-761 | Compl Gen | 000/969/905/GCF_000969905.1_ASM96990v1 |
| GCF_000012545.1 | Methanosphaera stadtmanae DSM 3091 | Compl Gen | 000/012/545/GCF_000012545.1_ASM1254v1 |
| GCF_000021965.1 | Methanosphaerula palustris E1-9c | Compl Gen | 000/021/965/GCF_000021965.1_ASM2196v1 |
| GCF_000013445.1 | Methanospirillum hungatei JF-1 | Compl Gen | 000/013/445/GCF_000013445.1_ASM1344v1 |
| GCF_000145295.1 | Methanothermobacter marburgensis str. Marburg Methanothermobacter | Compl Gen | 000/145/295/GCF_000145295.1_ASM14529v1 |
| GCF_000008645.1 | thermautotrophicus str. Delta H | Compl Gen | 000/008/645/GCF_000008645.1_ASM864v1 |
| GCF_000179575.2 | Methanothermococcus okinawensis IH1 | Compl Gen | 000/179/575/GCF_000179575.2_ASM17957v2 |
| GCF_000166095.1 | Methanothermus fervidus DSM 2088 | Compl Gen | 000/166/095/GCF_000166095.1_ASM16609v1 |
| GCF_000214415.1 | Methanotorris igneus Kol 5 | Compl Gen | 000/214/415/GCF_000214415.1_ASM21441v1 |
| GCF_000025625.1 | Natrialba magadii ATCC 43099 | Compl Gen | 000/025/625/GCF_000025625.1_ASM2562v1 |
| GCF_000230735.2 | Natrinema pellirubrum DSM 15624 | Compl Gen | 000/230/735/GCF_000230735.2_ASM23073v3 |
| GCF_000230715.2 | Natronobacterium gregoryi SP2 | Compl Gen | 000/230/715/GCF_000230715.2_ASM23071v3 |
| GCF_000328685.1 | Natronococcus occultus SP4 | Compl Gen | 000/328/685/GCF_000328685.1_ASM32868v1 |
| GCF_000591055.1 | Natronomonas moolapensis 8.8.11 | Compl Gen | 000/591/055/GCF_000591055.1_ASM59105v1 |
| GCF_000026045.1 | Natronomonas pharaonis DSM 2160 | Compl Gen | 000/026/045/GCF_000026045.1_ASM2604v1 |
| GCF_000725425.1 | Palaeococcus pacificus DY20341 | Compl Gen | 000/725/425/GCF_000725425.1_ASM72542v1 |
| GCF_000008265.1 | Picrophilus torridus DSM 9790 | Compl Gen | 000/008/265/GCF_000008265.1_ASM826v1 |
| GCF_000015205.1 | Pyrobaculum islandicum DSM 4184 | Compl Gen | 000/015/205/GCF_000015205.1_ASM1520v1 |
| GCF_000195935.2 | Pyrococcus abyssi GE5 | Compl Gen | 000/195/935/GCF_000195935.2_ASM19593v1 |
| GCF_000007305.1 | Pyrococcus furiosus DSM 3638 | Compl Gen | 000/007/305/GCF_000007305.1_ASM730v1 |
| GCF_000011105.1 | Pyrococcus horikoshii OT3 | Compl Gen | 000/011/105/GCF_000011105.1_ASM1110v1 |
| GCF_000211475.1 | Pyrococcus sp. NA2 | Compl Gen | 000/211/475/GCF_000211475.1_ASM21147v1 |
| GCF_000263735.1 | Pyrococcus sp. ST04 | Compl Gen | 000/263/735/GCF_000263735.1_ASM26373v1 |
| GCF_000215995.1 | Pyrococcus yayanosii CH1 | Compl Gen | 000/215/995/GCF_000215995.1_ASM21599v1 |
| GCF_001412615.1 | Pyrodictium delaneyi | Compl Gen | 001/412/615/GCF_001412615.1_ASM141261v1 |
| GCF_000223395.1 | Pyrolobus fumarii 1A | Compl Gen | 000/223/395/GCF_000223395.1_ASM22339v1 |
| GCF_000403645.1 | Salinarchaeum sp. Harcht-Bsk1 | Compl Gen | 000/403/645/GCF_000403645.1_ASM40364v1 |
| GCF_000092465.1 | Staphylothermus hellenicus DSM 12710 | Compl Gen | 000/092/465/GCF_000092465.1_ASM9246v1 |
| GCF_000015945.1 | Staphylothermus marinus F1 | Compl Gen | 000/015/945/GCF_000015945.1_ASM1594v1 |
| GCF_000012285.1 | Sulfolobus acidocaldarius DSM 639 | Compl Gen | 000/012/285/GCF_000012285.1_ASM1228v1 |
| GCF_000022385.1 | Sulfolobus islandicus L.S.2.15 | Compl Gen | 000/022/385/GCF_000022385.1_ASM2238v1 |
| GCF_000007005.1 | Sulfolobus solfataricus P2 | Compl Gen | 000/007/005/GCF_000007005.1_ASM700v1 |
| GCF_000011205.1 | Sulfolobus tokodaii str. 7 | Compl Gen | 000/011/205/GCF_000011205.1_ASM1120v1 |
| GCF_000151105.2 | Thermococcus barophilus MP | Compl Gen | 000/151/105/GCF_000151105.2_ASM15110v2 |
| GCF_000265525.1 | Thermococcus cleftensis | Compl Gen | 000/265/525/GCF_000265525.1_ASM26552v1 |
| GCF_000769655.1 | Thermococcus eurythermalis | Compl Gen | 000/769/655/GCF_000769655.1_ASM76965v1 |
| GCF_000022365.1 | Thermococcus gammatolerans EJ3 | Compl Gen | 000/022/365/GCF_000022365.1_ASM2236v1 |
| GCF_000009965.1 | Thermococcus kodakarensis KOD1 | Compl Gen | 000/009/965/GCF_000009965.1_ASM996v1 |
| GCF_000246985.2 | Thermococcus litoralis DSM 5473 | Compl Gen | 000/246/985/GCF_000246985.2_ASM24698v3 |
| GCF_000585495.1 | Thermococcus nautili | Compl Gen | 000/585/495/GCF_000585495.1_ASM58549v1 |
| GCF_000018365.1 | Thermococcus onnurineus NA1 | Compl Gen | 000/018/365/GCF_000018365.1_ASM1836v1 |
| GCF_000517445.1 | Thermococcus paralvinellae | Compl Gen | 000/517/445/GCF_000517445.1_ASM51744v1 |
| GCF_000022545.1 | Thermococcus sibiricus MM 739 | Compl Gen | 000/022/545/GCF_000022545.1_ASM2254v1 |
| GCF_000151205.2 | Thermococcus sp. AM4 | Compl Gen | 000/151/205/GCF_000151205.2_ASM15120v2 |
| GCF_000813245.1 | Thermofilum carboxyditrophus 1505 | Compl Gen | 000/813/245/GCF_000813245.1_ASM81324v1 |
| GCF_000015225.1 | Thermofilum pendens Hrk 5 | Compl Gen | 000/015/225/GCF_000015225.1_ASM1522v1 |
| GCF_000993805.1 | Thermofilum sp. 1807-2 | Compl Gen | 000/993/805/GCF_000993805.1_ASM99380v1 |
| GCF_000264495.1 | Thermogladius cellulolyticus 1633 | Compl Gen | 000/264/495/ GCF_000264495.1_ASM26449v1 |
| GCF_000195915.1 | Thermoplasma acidophilum DSM 1728 | Compl Gen | 000/195/915/GCF_000195915.1ASM19591v1 |
| GCF_000011185.1 | Thermoplasma volcanium GSS1 | Compl Gen | 000/011/185/GCF_000011185.1_ASM1118v1 |
| GCF_000350305.1 | Thermoplasmatales archaeon BRNA1 | Compl Gen | 000/350/305/GCF_000350305.1_ASM35030v1 |
| GCF_000253055.1 | Thermoproteus tenax Kra 1 | Compl Gen | 000/253/055/GCF_000253055.1_ASM25305v1 |
| GCF_000193375.1 | Thermoproteus uzoniensis 768-20 | Compl Gen | 000/193/375/GCF_000193375.1_ASM19337v1 |
| GCF_000092185.1 | Thermosphaera aggregans DSM 11486 | Compl Gen | 000/092/185/GCF_000092185.1_ASM9218v1 |
| GCF_000148385.1 | Vulcanisaeta distributa DSM 14429 | Compl Gen | 000/148/385/GCF_000148385.1_ASM14838v1 |
| GCF_000190315.1 | Vulcanisaeta moutnovskia 768-28 | Compl Gen | 000/190/315/GCF_000190315.1ASM19031v1 |

| ***Bacteria:*** | | | |
|---|---|---|---|
| GCF_000020965.1 | Dictyoglomus thermophilum H-6-12 | Compl Gen | 000/020/965/GCF_000020965.1_ASM2096v1 |
| GCF_001676785.2 | Brachyspira hyodysenteriae ATCC 27164 | Compl Gen | 001/676/785/GCF_001676785.2_ASM167678v2 |
| GCF_000017685.1 | Leptospira biflexa serovar Patoc strain 'Patoc 1 (Paris)' | Compl Gen | 000/017/685/GCF_000017685.1_ASM1768v1 |
| GCF_001399775.1 | Thermus aquaticus Y51MC23 | Compl Gen | 001/399/775/GCF_001399775.1_ASM139977v1 |
| GCF_000219605.1 | Pseudomonas stutzeri | Compl Gen | 000/219/605/GCF_000219605.1_ASM21960v1 |
| GCF_000021685.1 | Thermomicrobium roseum DSM 5159 | Compl Gen | 000/021/685/GCF_000021685.1_ASM2168v1 |
| GCF_000024405.1 | Sebaldella termitidis ATCC 33386 | Compl Gen | 000/024/405/GCF_000024405.1_ASM2440v1 |
| GCF_000146505.1 | Fibrobacter succinogenes subsp. succinogenes S85 | Compl Gen | 000/146/505/GCF_000146505.1_ASM14650v1 |
| GCF_000158275.2 | Fusobacterium nucleatum subsp. animalis 7_1 | Compl Gen | 000/158/275/GCF_000158275.2_ASM15827v2 |
| GCF_000025305.1 | Acidaminococcus fermentans DSM 20731 | Compl Gen | 000/025/305/GCF_000025305.1_ASM2530v1 |
| GCF_000010785.1 | Hydrogenobacter thermophilus TK-6 | Compl Gen | 000/010/785/GCF_000010785.1_ASM1078v1 |
| GCF_000284095.1 | Selenomonas ruminantium subsp. lactilytica TAM6421 | Compl Gen | 000/284/095/GCF_000284095.1_ASM28409v1 |
| GCF_000317695.1 | Anabaena cylindrica PCC 7122 | Compl Gen | 000/317/695/GCF_000317695.1_ASM31769v1 |
| GCF_000179635.2 | Ruminococcus albus 7 = DSM 20455 | Compl Gen | 000/179/635/GCF_000179635.2_ASM17963v2 |
| GCF_001543175.1 | Aerococcus urinae | Compl Gen | 001/543/175/GCF_001543175.1_ASM154317v1 |
| GCF_000953275.1 | Clostridioides difficile | Compl Gen | 000/953/275/GCF_000953275.1_CD630DERM |
| GCF_000145615.1 | Thermoanaerobacterium thermosaccharolyticum DSM 571 | Compl Gen | 000/145/615/GCF_000145615.1_ASM14561v1 |
| GCF_000013105.1 | Moorella thermoacetica ATCC 39073 | Compl Gen | 000/013/105/GCF_000013105.1_ASM1310v1 |
| GCF_000270085.1 | Erysipelothrix rhusiopathiae str. Fujisawa | Compl Gen | 000/270/085/GCF_000270085.1_ASM27008v1 |
| GCF_000612055.1 | Trueperella pyogenes | Compl Gen | 000/612/055/GCF_000612055.1_ASM61205v1 |
| GCF_001729525.1 | Brevibacterium linens | Compl Gen | 001/729/525/GCF_001729525.1_ASM172952v1 |
| GCF_000734015.1 | Thermodesulfobacterium commune DSM 2178 | Compl Gen | 000/734/015/GCF_000734015.1_ASM73401v1 |
| GCF_000058485.1 | Frankia alni ACN14a | Compl Gen | 000/058/485/GCF_000058485.1_ASM5848v1 |
| GCF_000092645.1 | Thermobispora bispora DSM 43833 | Compl Gen | 000/092/645/GCF_000092645.1_ASM9264v1 |
| GCF_000024385.1 | Thermomonospora curvata DSM 43183 | Compl Gen | 000/024/385/GCF_000024385.1_ASM2438v1 |
| GCF_000024985.1 | Sphaerobacter thermophilus DSM 20745 | Compl Gen | 000/024/985/GCF_000024985.1_ASM2498v1 |
| GCF_000269985.1 | Kitasatospora setae KM-6054 | Compl Gen | 000/269/985/GCF_000269985.1_ASM26998v1 |
| GCF_000008305.1 | Mesoplasma florum L1 | Compl Gen | 000/008/305/GCF_000008305.1_ASM830v1 |
| GCF_000021285.1 | Thermosipho africanus TCF52B | Compl Gen | 000/021/285/GCF_000021285.1_ASM2128v1 |
| GCF_000025205.1 | Gardnerella vaginalis 409-05 | Compl Gen | 000/025/205/GCF_000025205.1_ASM2520v1 |
| GCF_000009905.1 | Symbiobacterium thermophilum IAM 14863 | Compl Gen | 000/009/905/GCF_000009905.1_ASM990v1 |
| GCF_000015025.1 | Acidothermus cellulolyticus 11B | Compl Gen | 000/015/025/GCF_000015025.1_ASM1502v1 |
| GCF_000144695.1 | Acetohalobium arabaticum DSM 5501 | Compl Gen | 000/144/695/GCF_000144695.1_ASM14469v1 |
| GCF_000024945.1 | Veillonella parvula DSM 2008 | Compl Gen | 000/024/945/GCF_000024945.1_ASM2494v1 |
| GCF_000022325.1 | Caldicellulosiruptor bescii DSM 6725 | Compl Gen | 000/022/325/GCF_000022325.1_ASM2232v1 |
| GCF_000020485.1 | Halothermothrix orenii H 168 | Compl Gen | 000/020/485/GCF_000020485.1_ASM2048v1 |
| GCF_000175575.2 | Acidithiobacillus caldus ATCC 51756 | Compl Gen | 000/175/575/GCF_000175575.2_ASM17557v2 |
| GCF_000022565.1 | Acidobacterium capsulatum ATCC 51196 | Compl Gen | 000/022/565/GCF_000022565.1_ASM2256v1 |
| GCF_000247605.1 | Acetobacterium woodii DSM 1030 | Compl Gen | 000/247/605/GCF_000247605.1_ASM24760v1 |
| GCF_000019165.1 | Heliobacterium modesticaldum Ice1 | Compl Gen | 000/019/165/GCF_000019165.1_ASM1916v1 |
| GCF_000020945.1 | Coprothermobacter proteolyticus DSM 5265 | Compl Gen | 000/020/945/GCF_000020945.1_ASM2094v1 |
| GCF_000619905.2 | Nitrosospira briensis C-128 | Compl Gen | 000/619/905/GCF_000619905.2_ASM61990v2 |
| GCF_000012745.1 | Thiobacillus denitrificans ATCC 25259 | Compl Gen | 000/012/745/GCF_000012745.1_ASM1274v1 |
| GCF_000661895.1 | Rubrobacter radiotolerans | Compl Gen | 000/661/895/GCF_000661895.1_ASM66189v1 |
| GCF_000328625.1 | Halobacteroides halobius DSM 5150 | Compl Gen | 000/328/625/GCF_000328625.1_ASM32862v1 |
| GCF_000024605.1 | Ammonifex degensii KC4 | Compl Gen | 000/024/605/GCF_000024605.1_ASM2460v1 |
| GCF_000145035.1 | Butyrivibrio proteoclasticus B316 | Compl Gen | 000/145/035/GCF_000145035.1_ASM14503v1 |
| GCF_000092125.1 | Meiothermus silvanus DSM 9946 | Compl Gen | 000/092/125/GCF_000092125.1_ASM9212v1 |
| GCF_000024365.1 | Nakamurella multipartita DSM 44233 | Compl Gen | 000/024/365/GCF_000024365.1_ASM2436v1 |
| GCF_000023265.1 | Acidimicrobium ferrooxidans DSM 10331 | Compl Gen | 000/023/265/GCF_000023265.1_ASM2326v1 |
| GCF_000317125.1 | Chroococcidiopsis thermalis PCC 7203 | Compl Gen | 000/317/125/GCF_000317125.1_ASM31712v1 |
| GCF_000317025.1 | Pleurocapsa sp. PCC 7327 | Compl Gen | 000/317/025/GCF_000317025.1_ASM31702v1 |
| GCF_000816145.1 | Pseudothermotoga hypogea DSM 11164 = NBRC 106472 | Compl Gen | 000/816/145/GCF_000816145.1_ASM81614v1 |
| GCF_000024205.1 | Desulfotomaculum acetoxidans DSM 771 | Compl Gen | 000/024/205/GCF_000024205.1_ASM2420v1 |
| GCF_000011905.1 | Dehalococcoides mccartyi 195 | Compl Gen | 000/011/905/GCF_000011905.1_ASM1190v1 |
| GCF_000008625.1 | Aquifex aeolicus VF5 Desulfurobacterium | Compl Gen | 000/008/625/GCF_000008625.1_ASM862v1 |
| GCF_000191045.1 | thermolithotrophum DSM 11699 | Compl Gen | 000/191/045/GCF_000191045.1_ASM19104v1 |
| GCF_000222305.1 | Borreliella bissettii DN127 | Compl Gen | 000/222/305/GCF_000222305.1_ASM22230v1 |
| GCF_000018605.1 | Petrotoga mobilis SJ95 | Compl Gen | 000/018/605/GCF_000018605.1_ASM1860v1 |
| GCF_000184705.1 | Thermaerobacter marianensis DSM 12885 | Compl Gen | 000/184/705/GCF_000184705.1_ASM18470v1 |
| GCF_000025885.1 | Aminobacterium colombiense DSM 12261 | Compl Gen | 000/025/885/GCF_000025885.1_ASM2588v1 |
| GCF_000317065.1 | Pseudanabaena sp. PCC 7367 | Compl Gen | 000/317/065/GCF_000317065.1_ASM31706v1 |
| GCF_000237205.1 | Simkania negevensis Z | Compl Gen | 000/237/205/GCF_000237205.1_ASM23720v1 |
| GCF_000023885.1 | Slackia heliotrinireducens DSM 20476 | Compl Gen | 000/023/885/GCF_000023885.1_ASM2388v1 |
| GCF_000305935.1 | Thermacetogenium phaeum DSM 12270 | Compl Gen | 000/305/935/GCF_000305935.1_ASM30593v1 |
| GCF_000092405.1 | Syntrophothermus lipocalidus DSM 12680 | Compl Gen | 000/092/405/GCF_000092405.1_ASM9240v1 |
| GCF_000317575.1 | Stanieria cyanosphaera PCC 7437 | Compl Gen | 000/317/575/GCF_000317575.1_ASM31757v1 |
| GCF_000328705.1 | Saccharothrix espanaensis DSM 44229 | Compl Gen | 000/328/705/GCF_000328705.1_ASM32870v1 |
| GCF_000025645.1 | Thermoanaerobacter italicus Ab9 | Compl Gen | 000/025/645/GCF_000025645.1_ASM2564v1 |
| GCF_000014965.1 | Syntrophobacter fumaroxidans MPOB | Compl Gen | 000/014/965/GCF_000014965.1_ASM1496v1 |
| GCF_000017805.1 | Roseiflexus castenholzii DSM 13941 | Compl Gen | 000/017/805/GCF_000017805.1_ASM1780v1 |
| GCF_000012865.1 | Carboxydothermus hydrogenoformans Z-2901 | Compl Gen | 000/012/865/GCF_000012865.1_ASM1286v1 |
| GCF_000017305.1 | Kineococcus radiotolerans SRS30216 = ATCC BAA-149 | Compl Gen | 000/017/305/GCF_000017305.1_ASM1730v1 |
| GCF_000281175.1 | Caldilinea aerophila DSM 14535 = NBRC 104270 | Compl Gen | 000/281/175/GCF_000281175.1_ASM28117v1 |
| GCF_000025605.1 | Thermocrinis albus DSM 14484 | Compl Gen | 000/025/605/GCF_000025605.1_ASM2560v1 |
| GCF_000021945.1 | Chloroflexus aggregans DSM 9485 | Compl Gen | 000/021/945/GCF_000021945.1_ASM2194v1 |
| GCF_000025005.1 | Thermobaculum terrenum ATCC BAA-798 | Compl Gen | 000/025/005/GCF_000025005.1_ASM2500v1 |
| GCF_000199675.1 | Anaerolinea thermophila UNI-1 | Compl Gen | 000/199/675/GCF_000199675.1_ASM19967v1 |
| GCF_000021025.1 | Laribacter hongkongensis HLHK9 | Compl Gen | 000/021/025/GCF_000021025.1_ASM2102v1 |
| GCF_000217795.1 | Thermodesulfatator indicus DSM 15286 | Compl Gen | 000/217/795/GCF_000217795.1_ASM21779v1 |
| GCF_000010305.1 | Gemmatimonas aurantiaca T-27 | Compl Gen | 000/010/305/GCF_000010305.1_ASM1030v1 |
| GCF_000299235.1 | Leptospirillum ferriphilum ML-04 | Compl Gen | 000/299/235/GCF_000299235.1_ASM29923v1 |
| GCF_000024345.1 | Kribbella flavida DSM 17836 | Compl Gen | 000/024/345/GCF_000024345.1_ASM2434v1 |
| GCF_000212395.1 | Thermodesulfobium narugense DSM 14796 | Compl Gen | 000/212/395/GCF_000212395.1_ASM21239v1 |
| GCF_000195335.1 | Marinithermus hydrothermalis DSM 14884 | Compl Gen | 000/195/335/GCF_000195335.1_ASM19533v1 |
| GCF_000754265.1 | Candidatus Baumannia cicadellinicola | Compl Gen | 000/754/265/GCF_000754265.1_ASM75426v1 |
| GCF_000183745.1 | Oceanithermus profundus DSM 14977 | Compl Gen | 000/183/745/GCF_000183745.1_ASM18374v1 |
| GCF_000025265.1 | Conexibacter woesei DSM 14684 | Compl Gen | 000/025/265/GCF_000025265.1_ASM2526v1 |
| GCF_000194605.1 | Fluviicola taffensis DSM 16823 | Compl Gen | 000/194/605/GCF_000194605.1_ASM19460v1 |
| GCF_000494755.1 | Actinoplanes friuliensis DSM 7358 | Compl Gen | 000/494/755/GCF_000494755.1_ASM49475v1 |
| GCF_000016985.1 | Alkaliphilus metalliredigens QYMF | Compl Gen | 000/016/985/GCF_000016985.1_ASM1698v1 |
| GCF_000185805.1 | Thermovibrio ammonificans HB-1 | Compl Gen | 000/185/805/GCF_000185805.1_ASM18580v1 |
| GCF_000020225.1 | Akkermansia muciniphila ATCC BAA-835 | Compl Gen | 000/020/225/GCF_000020225.1_ASM2022v1 |
| GCF_000317495.1 | Crinalium epipsammum PCC 9333 | Compl Gen | 000/317/495/GCF_000317495.1_ASM31749v1 |
| GCF_000021725.1 | Nautilia profundicola AmH | Compl Gen | 000/021/725/GCF_000021725.1_ASM2172v1 |
| GCF_000213255.1 | Mahella australiensis 50-1 BON | Compl Gen | 000/213/255/GCF_000213255.1_ASM21325v1 |
| GCF_000020505.1 | Chlorobaculum parvum NCIB 8327 | Compl Gen | 000/020/505/GCF_000020505.1_ASM2050v1 |
| GCF_000024545.1 | Stackebrandtia nassauensis DSM 44728 | Compl Gen | 000/024/545/GCF_000024545.1_ASM2454v1 |
| GCF_000144645.1 | Thermosediminibacter oceani DSM 16646 | Compl Gen | 000/144/645/GCF_000144645.1_ASM14464v1 |
| GCF_001688625.1 | Flavonifractor plautii | Compl Gen | 001/688/625/GCF_001688625.1_ASM168862v1 |
| GCF_000024025.1 | Catenulispora acidiphila DSM 44928 | Compl Gen | 000/024/025/GCF_000024025.1_ASM2402v1 |
| GCF_000021565.1 | Persephonella marina EX-HI | Compl Gen | 000/021/565/GCF_000021565.1_ASM2156v1 |
| GCF_000021545.1 | Sulfurihydrogenibium azorense Az-Fu1 | Compl Gen | 000/021/545/GCF_000021545.1_ASM2154v1 |
| GCF_000024165.1 | Candidatus Accumulibacter phosphatis clade IIA str. UW-1 | Compl Gen | 000/024/165/GCF_000024165.1_ASM2416v1 |
| GCF_000092425.1 | Truepera radiovictrix DSM 17093 | Compl Gen | 000/092/425/GCF_000092425.1_ASM9242v1 |
| GCF_000019905.1 | Exiguobacterium sibiricum 255-15 | Compl Gen | 000/019/905/GCF_000019905.1_ASM1990v1 |
| GCF_000283575.1 | Oscillibacter valericigenes Sjm18-20 | Compl Gen | 000/283/575/GCF_000283575.1_ASM28357v1 |
| GCF_000023705.1 | Methylotenera mobilis JLW8 | Compl Gen | 000/023/705/GCF_000023705.1_ASM2370v1 |
| GCF_000271665.2 | Pelosinus fermentans JBW45 | Compl Gen | 000/271/665/GCF_000271665.2_ASM27166v2 |
| GCF_000145255.1 | Calhonella capsiferriformans ES-2 | Compl Gen | 000/145/255/GCF_000145255.1_ASM14525v1 |
| GCF_000020005.1 | Natranaerobius thermophilus JW/NM-WN-LF | Compl Gen | 000/020/005/GCF_000020005.1_ASM2000v1 |
| GCF_000020785.1 | Hydrogenobaculum sp. Y04AAS1 | Compl Gen | 000/020/785/GCF_000020785.1_ASM2078v1 |
| GCF_000265425.1 | Terriglobus roseus DSM 18391 | Compl Gen | 000/265/425/GCF_000265425.1_ASM26542v1 |
| GCF_000020145.1 | Elusimicrobium minutum Pei191 | Compl Gen | 000/020/145/GCF_000020145.1_ASM2014v1 |
| GCF_000226295.1 | Chloracidobacterium thermophilum B | Compl Gen | 000/226/295/GCF_000226295.1ASM22629v1 |
| GCF_000348785.1 | Ilumatobacter coccineus YM16-304 | Compl Gen | 000/348/785/GCF_000348785.1_ASM34878v1 |
| GCF_000306785.1 | Modestobacter marinus | Compl Gen | 000/306/785/GCF_000306785.1_ASM30678v1 |
| GCF_000183405.1 | Calditerrivibrio nitroreducens DSM 19672 | Compl Gen | 000/183/405/GCF_000183405.1_ASM18340v1 |
| GCF_000328765.2 | Tepidanaerobacter acetatoxydans Re1 | Compl Gen | 000/328/765/GCF_000328765.2_ASM32876v2 |
| GCF_000284115.1 | Phycisphaera mikurensis NBRC 102666 | Compl Gen | 000/284/115/GCF_000284115.1_ASM28411v1 |
| GCF_000025965.1 | Aromatoleum aromaticum EbN1 | Compl Gen | 000/025/965/GCF_000025965.1_ASM2596v1 |
| GCF_000143165.1 | Dehalogenimonas lykanthroporepellens BL-DC-9 | Compl Gen | 000/143/165/GCF_000143165.1_ASM14316v1 |
| GCF_000297055.2 | Sulfuricella denitrificans skB26 | Compl Gen | 000/297/055/GCF_000297055.2_ASM29705v2 |
| GCF_000166415.1 | Halanaerobium hydrogeniformans | Compl Gen | 000/166/415/GCF_000166415.1_ASM16641v1 |
| GCF_000284335.1 | Caldisericum exile AZM16c01 Caldanaerobacter | Compl Gen | 000/284/335/GCF_000284335.1_ASM28433v1 |
| GCF_000007085.1 | subterraneus subsp. tengcongensis MB4 | Compl Gen | 000/007/085/GCF_000007085.1_ASM708v1 |
| GCF_000177635.2 | Desulfurispirillum indicum S5 | Compl Gen | 000/177/635/GCF_000177635.2_ASM17763v2 |
| GCF_000178955.2 | Granulicella mallensis MP5ACTX8 | Compl Gen | 000/178/955/GCF_000178955.2_ASM17895v2 |
| GCF_000192745.1 | Polymorphum gilvum SL003B-26A1 | Compl Gen | 000/192/745/GCF_000192745.1_ASM19274v1 |
| GCF_000724625.1 | Fimbriimonas ginsengisoli Gsoil 348 | Compl Gen | 000/724/625/GCF_000724625.1_ASM72462v1 |
| GCF_000279145.1 | Melioribacter roseus P3M-2 | Compl Gen | 000/279/145/GCF_000279145.1_ASM27914v1 |
| GCF_000147715.2 | Mesotoga prima MesG1.Ag.4.2 | Compl Gen | 000/147/715/GCF_000147715.2_ASM14771v3 |
| GCF_001443605.1 | bacterium L21-Spi-D4 | Compl Gen | 001/443/605/GCF_001443605.1_ASM144360v1 |
| GCF_001007995.1 | 'Deinococcus soli' Cha et al. 2014 | Compl Gen | 001/007/995/GCF_001007995.1_ASM100799v1 |
| GCF_000484535.1 | Gloeobacter kilaueensis JS1 | Compl Gen | 000/484/535/GCF_000484535.1_ASM48453v1 |
| GCF_000940805.1 | Gynuella sunshinyii YC6258 | Compl Gen | 000/940/805/GCF_000940805.1_ASM94080v1 |
| GCF_000828975.1 | Burkholderiales bacterium GJ-E10 | Compl Gen | 000/828/975/GCF_000828975.1_ASM82897v1 |
| GCF_000828655.1 | Thermotoga caldifontis AZM44c09 | Compl Gen | 000/828/655/GCF_000828655.1_ASM82865v1 |
| GCF_001281505.1 | Lawsonella clevelandensis | Compl Gen | 001/281/505/GCF_001281505.1_ASM128150v1 |
| GCF_001023575.1 | Actinobacteria bacterium IMCC26256 | Compl Gen | 001/023/575/GCF_001023575.1_ASM102357v1 |
| GCF_001507665.1 | Deinococcus actinosclerus | Compl Gen | 001/507/665/GCF_001507665.1_ASM150766v1 |
| GCF_000952975.1 | Peptoniphilus sp. ING2-D1G | Compl Gen | 000/952/975/GCF_000952975.1_D1G |

| ***Fungi:*** | | | |
|---|---|---|---|
| GCF_000002945.1 | Schizosaccharomyces pombe | Chrom | 000/002/945/GCF_000002945.1_ASM294v2 |
| GCF_000315895.1 | Millerozyma farinosa CBS 7064 | Chrom | 000/315/895/GCF_000315895.1_ASM31589v1 |
| GCF_000209165.1 | Scheffersomyces stipitis CBS 6054 | Chrom | 000/209/165/GCF_000209165.1_ASM20916v1 |
| GCF_000146045.2 | Saccharomyces cerevisiae S288c | Compl Gen | 000/146/045/GCF_000146045.2_R64 |
| GCF_000243375.1 | Torulaspora delbrueckii | Chrom | 000/243/375/GCF_000243375.1_ASM24337v1 |
| GCF_000002525.2 | Yarrowia lipolytica CLIB122 | Chrom | 000/002/525/GCF_000002525.2_ASM252v1 |
| GCF_000026365.1 | Zygosaccharomyces rouxii | Chrom | 000/026/365/GCF_000026365.1_ASM2636v1 |
| GCF_000006445.2 | Debaryomyces hansenii CBS767 | Chrom | 000/006/445/GCF_000006445.2_ASM644v2 |
| GCF_000182925.2 | Neurospora crassa OR74A | Chrom | 000/182/925/GCF_000182925.2_NC12 |
| GCF_000091045.1 | Cryptococcus neoformans var. neoformans JEC21 | Chrom | 000/091/045/GCF_000091045.1_ASM9104v1 |
| GCF_000328475.2 | Ustilago maydis 521 | Chrom | 000/328/475/GCF_000328475.2_Umaydis521_2.0 |
| GCF_000182965.3 | Candida albicans SC5314 | Chrom | 000/182/965/GCF_000182965.3_ASM18296v3 |
| GCF_000002545.3 | [Candida] glabrata | Chrom | 000/002/545/GCF_000002545.3_ASM254v2 |
| GCF_000149955.1 | Fusarium oxysporum f. sp. lycopersici 4287 | Chrom | 000/149/955/GCF_000149955.1_ASM14995v2 |
| GCF_000240135.3 | Fusarium graminearum PH-1 | Chrom | 000/240/135/GCF_000240135.3_ASM24013v3 |
| GCF_000091225.1 | Encephalitozoon cuniculi GB-M1 | Chrom | 000/091/225/GCF_000091225.1_ASM9122v1 |
| GCF_000237345.1 | Naumovozyma castellii CBS 4309 | Chrom | 000/237/345/GCF_000237345.1_ASM23734v1 |
| GCF_000227115.2 | Naumovozyma dairenensis CBS 421 | Chrom | 000/227/115/GCF_000227115.2_ASM22711v2 |
| GCF_000277815.2 | Encephalitozoon hellem ATCC 50504 | Chrom | 000/277/815/GCF_000277815.2_ASM27781v3 |
| GCF_000002515.2 | Kluyveromyces lactis | Compl Gen | 000/002/515/GCF_000002515.2_ASM251v1 |
| GCF_000091025.4 | Eremothecium gossypii ATCC 10895 | Compl Gen | 000/091/025/GCF_000091025.4_ASM9102v4 |
| GCF_000226115.1 | Thielavia terrestris NRRL 8126 | Compl Gen | 000/226/115/GCF_000226115.1_ASM22611v1 |
| GCF_000185945.1 | Cryptococcus gattii WM276 | Chrom | 000/185/945/GCF_000185945.1_ASM18594v1 |
| GCF_000026945.1 | Candida dubliniensis CD36 | Compl Gen | 000/026/945/GCF_000026945.1_ASM2694v1 |
| GCF_000235365.1 | Eremothecium cymbalariae DBVPG#7215 | Chrom | 000/235/365/GCF_000235365.1_ASM23536v1 |
| GCF_000146465.1 | Encephalitozoon intestinalis ATCC 50506 | Chrom | 000/146/465/GCF_000146465.1_ASM14646v1 |
| GCF_000226095.1 | Thermothelomyces thermophila ATCC 42464 | Compl Gen | 000/226/095/GCF_000226095.1_ASM22609v1 |
| GCF_001672515.1 | Colletotrichum higginsianum IMI 349063 | Chrom | 001/672/515/GCF_001672515.1_ASM167251v1 |
| GCF_000303195.2 | Fusarium pseudograminearum CS3096 | Chrom | 000/303/195/GCF_000303195.2_FP7 |
| GCF_000236905.1 | Tetrapisispora phaffii CBS 4417 | Chrom | 000/236/905/GCF_000236905.1_ASM23690v1 |
| GCF_000149555.1 | Fusarium verticillioides 7600 | Chrom | 000/149/555/GCF_000149555.1_ASM14955v1 |
| GCF_000315875.1 | Candida orthopsilosis | Chrom | 000/315/875/GCF_000315875.1_ASM31587v1 |
| GCF_000002495.2 | Magnaporthe oryzae 70-15 | Chrom | 000/002/495/GCF_000002495.2_MG8 |
| GCF_000142805.1 | Lachancea thermotolerans CBS 6340 | Chrom | 000/142/805/GCF_000142805.1_ASM14280v1 |
| GCF_000304475.1 | Kazachstania africana CBS 2517 | Chrom | 000/304/475/GCF_000304475.1_Ka_CBS2517 |
| GCF_000027005.1 | Komagataella phaffii GS115 | Chrom | 000/027/005/GCF_000027005.1_ASM2700v1 |
| GCF_000280035.1 | Encephalitozoon romaleae SJ-2008 | Chrom | 000/280/035/GCF_000280035.1_ASM28003v2 |
| GCF_000002655.1 | Aspergillus fumigatus Af293 | Chrom | 000/002/655/GCF_000002655.1_ASM265v1 |
| GCF_001640025.1 | Sugiyamaella lignohabitans | Compl Gen | 001/640/025/GCF_001640025.1_ASM164002v2 |
| GCF_000187245.1 | Ogataea parapolymorpha DL-1 | Chrom | 000/187/245/GCF_000187245.1_Hansenula_2 |
| GCF_000219625.1 | Zymoseptoria tritici IPO323 | Chrom | 000/219/625/GCF_000219625.1_MYCGR_v2.0 |
| GCF_000315915.1 | Tetrapisispora blattae CBS 6284 | Chrom | 000/315/915/GCF_000315915.1_ASM31591v1 |
| GCF_001298625.1 | Saccharomyces eubayanus | Chrom | 001/298/625/GCF_001298625.1_SEUB3.0 |

| ***Invertebrates:*** | | | |
|---|---|---|---|
| GCF_000237925.1 | Schistosoma mansoni | Chrom | 000/237/925/GCF_000237925.1_ASM23792v2 |
| GCF_000004555.1 | Caenorhabditis briggsae | Chrom | 000/004/555/GCF_000004555.1_ASM455v1 |
| GCF_000002985.6 | Caenorhabditis elegans | Compl Gen | 000/002/985/GCF_000002985.6_WBcel235 |
| GCF_000002335.3 | Tribolium castaneum | Chrom | 000/002/335/GCF_000002335.3_Tcas5.2 |
| GCF_000005575.2 | Anopheles gambiae str. PEST | Chrom | 000/005/575/GCF_000005575.2_AgamP3 |
| GCF_000001215.4 | Drosophila melanogaster | Chrom | 000/001/215/GCF_000001215.4_Release_6_plu s_ISO1_MT |
| GCF_000269505.1 | Drosophila miranda | Chrom | 000/269/505/GCF_000269505.1_DroMir_2.2 |
| GCF_000001765.3 | Drosophila pseudoobscura pseudoobscura | Chrom | 000/001/765/GCF_000001765.3_Dpse_3.0 |
| GCF_000754195.2 | Drosophila simulans | Chrom | 000/754/195/GCF_000754195.2_ASM75419v2 |
| GCF_000005975.2 | Drosophila yakuba | Chrom | 000/005/975/GCF_000005975.2_dyak_caf1 |
| GCF_000002325.3 | Nasonia vitripennis | Chrom | 000/002/325/GCF_000002325.3_Nvit_2.1 |
| GCF_000002195.4 | Apis mellifera | Chrom | 000/002/195/GCF_000002195.4_Amel_4.5 |
| GCF_000224145.2 | Ciona intestinalis | Chrom | 000/224/145/GCF_000224145.2_KH |
| GCF_001277935.1 | Drosophila busckii | Chrom | 001/277/935/GCF_001277935.1_ASM127793v1 |
| GCF_000214255.1 | Bombus terrestris | Chrom | 000/214/255/GCF_000214255.1_Bter_1.0 |

| ***Plants:*** | | | |
|---|---|---|---|
| GCF_000317415.1 | Citrus sinensis | Chrom | 000/317/415/GCF 000317415.1_Csi_vaiencia_1 .0 |
| GCF_000987745.1 | Gossypium hirsutum | Chrom | 000/987/745/GCF_000987745.1_ASM98774v1 000208745.1 |
| GCF_000208745.1 | Theobroma cacao | Chrom | 000/208/745/GCF__Criollo_cocoa_ genome_V2 |
| GCF_000004075.2 | Cucumis sativus | Chrom | 000/004/075/GCF_000004075.2_ASM407v2 |
| GCF_000002775.3 | Populus trichocarpa | Chrom | 000/002/775/GCF_000002775.3_Poptr2_0 |
| GCF_000001735.3 | Arabidopsis thaliana | Chrom | 000/001/735/GCF_000001735.3_TAIR10 |
| GCF_000686985.1 | Brassica napus | Chrom | 000/686/985/GCF_000686985.1_Brassica_napu bly_y1.0 |
| GCF_000309985.1 | Brassica rapa | Chrom | 000/309/985/GCF_000309985.1_Brapa_1.0 |
| GCF_000695525.1 | Brassica oleracea var. oleracea | Chrom | 000/695/525/GCF_000695525.1_BOL |
| GCF_000148765.1 | Malus domestica | Chrom | 000/148/765/GCF _000148765.1_ MalDomGD1.0 |
| GCF_000340665.1 | Cajanus cajan | Chrom | 000/340/665/GCF _000340665.1_C.cajan_V1.0 |
| GCF_000331145.1 | Cicer arietinum | Chrom | 000/331/145/GCF_000331145.1_ASM33114v1 |
| GCF_000004515.4 | Glycine max | Chrom | 000/004/515/GCF_000004515.4_Glycine_max_v |
| GCF_001865875.1 | Lupinus angustifolius | Chrom | 001/865/875/GCF_001865875.1_LupAngTanjil_ |
| GCF_000219495.3 | Medicago truncatula | Chrom | 000/219/495/GCF_000219495.3_MedtrA17_4.0 |
| GCF_000499845.1 | Phaseolus vulgaris | Chrom | 000/499/845/GCF_000499845.1_PhaVulg1_0 |
| GCF_001190045.1 | Vigna angularis | Chrom | 001/190/045/GCF_001190045.1_Vigan1.1 |
| GCF_001625215.1 | Daucus carota subsp. sativus | Chrom | 001/625/215/GCF_001625215.1_ASM162521v1 |
| GCF_000710875.1 | Capsicum annuum | Chrom | 000/710/875/GCF_000710875.1_Pepper_Zunla_ 1_Ref_v1.0 |
| GCF_000188115.3 | Solanum lycopersicum | Chrom | 000/188/115/GCF_000188115.3_SL2.50 000/512/975/GCF_000512975.1_S_indicum_v1. |
| GCF_000512975.1 | Sesamum indicum | Chrom | 0 |
| GCF_001433935.1 | Oryza sativa Japonica Group | Chrom | 001/433/935/GCF_001433935.1_IRGSP-1.0 |
| GCF_000231095.1 | Oryza brachyantha | Chrom | 000/231/095/GCF_000231095.1_Oryza_brachya |
| GCF_000263155.2 | Setaria italica | Chrom | 000/263/155/GCF_000263155.2_Setaria_italica _v2.0 |
| GCF_000003195.2 | Sorghum bicolor | Chrom | 000/003/195/GCF_000003195.2_Sorbil |
| GCF_000005005.1 | Zea mays | Chrom | 000/005/005/GCF_000005005.1_B73_RefGen_v |
| GCF_001540865.1 | Ananas comosus | Chrom | 001/540/865/GCF_001540865.1_ASM154086v1 |
| GCF_000313855.2 | Musa acuminata subsp. malaccensis | Chrom | 000/313/855/GCF_000313855.2_ASM31385v2 |
| GCF_001876935.1 | Asparagus officinalis | Chrom | 001/876/935/GCF_001876935.1_Aspof.V1 |
| GCF_000005505.2 | Brachypodium distachyon | Chrom | 000/005/505/GCF_000005505.2_Brachypodium _distachyon_v2.0 |
| GCF_001406875.1 | Solanum pennellii | Chrom | 001/406/875/GCF_001406875.1_SPENNV200 |
| GCF_000612285.1 | Gossypium arboreum | Chrom | 000/612/285/GCF_000612285.1_Gossypium_ar boreum_v1.0 |
| GCF_000327365.1 | Gossypium raimondii | Chrom | 000/327/365/GCF_000327365.1_Graimondii2_0 |
| GCF_000003745.3 | Vitis vinifera | Chrom | 000/003/745/GCF_000003745.3_12X |
| GCF_000091205.1 | Cyanidioschyzon merolae strain 10D | Compl Gen | 000/091/205/GCF_000091205.1_ASM9120v1 |
| GCF_001879085.1 | Nicotiana attenuata | Chrom | 001/879/085/GCF_001879085.1_NIATTr2 |
| GCF_000442705.1 | Elaeis guineensis | Chrom | 000/442/705/GCF_000442705.1_EG5 |
| GCF_000184155.1 | Fragaria vesca subsp. vesca | Chrom | 000/184/155/GCF_000184155.1_FraVesHawai_1.0 |
| GCF_000214015.2 | Ostreococcus tauri | Chrom | 000/214/015/GCF_000214015.2_version_05060 6 |
| GCF_000633955.1 | Camelina sativa | Chrom | 000/633/955/GCF_ 000633955.1_Cs |
| GCF_000346735.1 | Prunus mume | Chrom | 000/346/735/GCF_000346735.1_P.mume_V1.0 |
| GCF_000741045.1 | Vigna radiata var. radiata | Chrom | 000/741/045/GCF_000741045.1_Vradiata_ver6 |
| GCF_000511025.2 | Beta vulgaris subsp. vulgaris | Chrom | 000/511/025/GCF_000511025.2_RefBeet-1.2.2 |
| GCF_000092065.1 | Ostreococcus lucimarinus CCE9901 | Compl Gen | 000/092/065/GCF_000092065.1_ASM9206v1 |
| GCF_000090985.2 | Micromonas commoda | Compl Gen | 000/090/985/GCF_000090985.2_ASM9098v2 |
| GCF_000826755.1 | Ziziphus jujuba | Chrom | 000/826/755/GCF_000826755.1_ZizJuj_1.1 |

| ***Protoza:*** | | | |
|---|---|---|---|
| GCF_000150955.2 | Phaeodactylum tricornutum CCAP 1055/1 | Chrom | 000/150/955/GCF_000150955.2_ASM15095v2 |
| GCF_000002845.2 | Leishmania braziliensis MHOM/BR/75/M2904 | Chrom | 000/002/845/GCF_000002845.2_ASM284v2 |
| GCF_000227135.1 | Leishmania donovani | Chrom | 000/227/135/GCF_000227135.1_ASM22713v2 |
| GCF_000002725.2 | Leishmania major strain Friedlin | Compl Gen | 000/002/725/GCF_000002725.2_ASM272v2 |
| GCF_000234665.1 | Leishmania mexicana MHOM/GT/2001/U1103 | Chrom | 000/234/665/GCF_000234665.1_ASM23466v4 |
| GCF_000002875.2 | Leishmania infantum JPCM5 | Chrom | 000/002/875/GCF_000002875.2_ASM287v2 |
| GCF_000755165.1 | Leishmania panamensis | Chrom | 000/755/165/GCF_000755165.1_ASM75516v1 |
| GCF_000210295.1 | Trypanosoma brucei gambiense DAL972 | Chrom | 000/210/295/GCF_000210295.1_ASM21029v1 |
| GCF_000165345.1 | Cryptosporidium parvum Iowa II | Chrom | 000/165/345/GCF_000165345.1_ASM16534v1 |
| GCF_000006565.2 | Toxoplasma gondii ME49 | Chrom | 000/006/565/GCF_000006565.2_TGA4 |
| GCF_900002335.2 | Plasmodium chabaudi chabaudi | Chrom | 900/002/335/GCF_900002335.2_PCHAS01 |
| GCF_000321355.1 | Plasmodium cynomolgi strain B | Chrom | 000/321/355/GCF_000321355.1_PcynB_1.0 |
| GCF_000002765.3 | Plasmodium falciparum 3D7 | Chrom | 000/002/765/GCF_000002765.3_ASM276v1 |
| GCF_000006355.1 | Plasmodium knowlesi strain H | Chrom | 000/006/355/GCF_000006355.1_ASM635v1 |
| GCF_001601855.1 | Plasmodium reichenowi | Chrom | 001/601/855/GCF_001601855.1_ASM160185v1 |
| GCF_000002415.2 | Plasmodium vivax | Chrom | 000/002/415/GCF_000002415.2_ASM241v2 |
| GCF_000165395.1 | Babesia bovis | Chrom | 000/165/395/GCF_000165395.1_ASM16539v1 |
| GCF_000981445.1 | Babesia bigemina | Chrom | 000/981/445/GCF_000981445.1_Bbig001 |
| GCF_000691945.1 | Babesia microti strain RI | Chrom | 000/691/945/GCF_000691945.1_ASM69194v1 |
| GCF_000342415.1 | Theileria equi strain WA | Chrom | 000/342/415/GCF_000342415.1_JCVI-bewag-v1.1 |
| GCF_000003225.2 | Theileria annulata strain Ankara | Chrom | 000/003/225/GCF_000003225.2_ASM322v1 |
| GCF_000165365.1 | Theileria parva | Chrom | 000/165/365/GCF_000165365.1_ASM16536v1 |
| GCF_000208865.1 | Neospora caninum Liverpool | Chrom | 000/208/865/GCF_000208865.1_ASM20886v2 |
| GCF_000149405.2 | Thalassiosira pseudonana CCMP1335 | Chrom | 000/149/405/GCF_000149405.2_ASM14940v2 |
| GCF_000004695.1 | Dictyostelium discoideum | Chrom | 000/004/695/GCF_000004695.1_dicty_2.7 |
| GCF_000740895.1 | Theileria orientalis strain Shintoku | Compl Gen | 000/740/895/GCF_000740895.1_ASM74089v1 |
| GCF_001680005.1 | Plasmodium coatneyi | Chrom | 001/680/005/GCF_001680005.1_ASM168000v1 |
| GCF_001602025.1 | Plasmodium gaboni | Chrom | 001/602/025/GCF_001602025.1_ASM160202v1 |

| ***Mammals:*** | | | |
|---|---|---|---|
| GCF_000002275.2 | Ornithorhynchus anatinus | Chrom | 000/002/275/GCF_000002275.2_Ornithorhynch us_anatinus_5.0.1 |
| GCF_000004665.1 | Callithrix jacchus | Chrom | 000/004/665/GCF_000004665.1_Callithrix_jacchus-3.2 |
| GCF_000364345.1 | Macaca fascicularis | Chrom | 000/364/345/GCF_000364345.1_Macaca_fascic ularis_5.0 |
| GCF_000772875.2 | Macaca mulatta | Chrom | 000/772/875/GCF_000772875.2_Mmul_8.0.1 |
| GCF_000264685.2 | Papio anubis | Chrom | 000/264/685/GCF_000264685.2 _Panu_2.0 |
| GCF_000151905.2 | Gorilla gorilla gorilla | Chrom | 000/151/905/GCF_000151905.2_gorGor4 |
| GCF_000258655.2 | Pan paniscus | Chrom | 000/258/655/GCF_000258655.2_panpan1.1 |
| GCF_000001515.7 | Pan troglodytes | Chrom | 000/001/515/GCF_000001515.7_Pan_tro_3.0 |
| GCF_000001545.4 | Pongo abelii | Chrom | 000/001/545/GCF_000001545.4_P_pygmaeus_ |
| GCF_000001405.36 | Homo sapiens | Chrom | 000/001/405/GCF_000001405.36_GRCh38.p10 |
| GCF_000002285.3 | Canis lupus familiaris | Chrom | 000/002/285/GCF_000002285.3_CanFam3.1 |
| GCF_000181335.2 | Felis catus | Chrom | 000/181/335/GCF_000181335.2_Felis_catus_8. 0 |
| GCF_000002305.2 | Equus caballus | Chrom | 000/002/305/GCF_000002305.2_EquCab2.0 |
| GCF_000003025.5 | Sus scrofa | Chrom | 000/003/025/GCF_000003025.5_Sscrofa10.2 |
| GCF_000003055.6 | Bos taurus | Chrom | 000/003/055/GCF_000003055.6_Bos_taurus_U MD3.1.1 |
| GCF_000247795.1 | Bos indicus | Chrom | _ 000/247/795/GCF_000247795.1_Bos-_indicus_1. 0 |
| GCF_001704415.1 | Capra hircus | Chrom | 001/704/415/GCF_001704415.1_ARS1 |
| GCF_000298735.2 | Ovis aries | Chrom | 000/298/735/GCF_000298735.2_Oar_v4.0 |
| GCF_000003625.3 | Oryctolagus cuniculus | Chrom | 000/003/625/GCF_000003625.3_OryCun2.0 |
| GCF_000001635.25 | Mus musculus | Chrom | 000/001/635/GCF_000001635.25_GRCm38.p5 |
| GCF_000001895.5 | Rattus norvegicus | Chrom | 000/001/895/GCF_000001895.5_Rnor_6.0 |
| GCF_000002295.2 | Monodelphis domestica | Chrom | 000/002/295/GCF_000002295.2_MonDom5 |
| GCF_000409795.2 | Chlorocebus sabaeus | Chrom | 00/409/795/GCF_000409795.2_Chlorocebus_s abeus_1.1 |
| GCF_000146795.2 | Nomascus leucogenys | Chrom | 000/146/795/GCF_000146795.2_Nleu_3.0 |
| GCF_000317375.1 | Microtus ochrogaster | Chrom | 000/317/375/GCF_000317375.1_MicOch1.0 |

| ***Non-mammalian ve rtebrates:*** | | | |
|---|---|---|---|
| GCF_000242695.1 | Lepisosteus oculatus | Chrom | 000/242/695/GCF_000242695.1_LepOcul |
| GCF_000002035.5 | Danio rerio | Chrom | 000/002/035/GCF_000002035.5_GRCz10 |
| GCF_000951615.1 | Cyprinus carpio | Chrom | 000/951/615/GCF_000951615.1_common_carp _genome |
| GCF_001660625.1 | Ictalurus punctatus | Chrom | 001/660/625/GCF _001660625.1_IpCoco_1.2 |
| GCF_000721915.3 | Esox lucius | Chrom | 000/721/915/GCF_000721915.3_Eluc_V3 |
| GCF_000233375.1 | Salmo salar | Chrom | 000/233/375/GCF_000233375.1_ICSASG_v2 |
| GCF_000633615.1 | Poecilia reticulata | Chrom | 000/633/615/GCF_000633615.1_Guppy_female _1.0_MT |
| GCF_000313675.1 | Oryzias latipes | Chrom | 000/313/675/GCF_000313675.1_ASM31367v1 |
| GCF_001858045.1 | Oreochromis niloticus | Chrom | 001/858/045/GCF_001858045.1_ASM185804v2 |
| GCF_001663975.1 | Xenopus laevis | Chrom | 001/663/975/GCF_001663975.1_Xenopus_laevi s_v2 |
| GCF_000004195.3 | Xenopus tropicalis | Chrom | 000/004/195/GCF_000004195.3_Xenopus_tropi calis_v9.1 |
| GCF_000241765.3 | Chrysemys picta bellii | Chrom | 000/241/765/GCF_000241765.3_Chrysemys_pic tabellii-3.0.3 |
| GCF_000002315.4 | Gallus gallus | Chrom | _ 000/002/315/GCF_000002315.4_Gallus_gallus-5.0 |
| GCF_000146605.2 | Meleagris gallopavo | Chrom | 000/146/605/GCF_000146605.2_Turkey_5.0 |
| GCF_001522545.2 | Parus major | Chrom | 001/522/545/GCF_001522545.2_Parus_major1.1 |
| GCF_000090745.1 | Anolis carolinensis | Chrom | 000/090/745/GCF_000090745.1_AnoCar2.0 |
| GCF_000180615.1 | Takifugu rubripes | Chrom | 000/180/615/GCF_000180615.1_FUGU5 |
| GCF_000151805.1 | Taeniopygia guttata | Chrom | 000/151/805/GCF_000151805.1_Taeniopygia_guttata-3.2.4 |
| GCF_000247815.1 | Ficedula albicollis | Chrom | 000/247/815/GCF_000247815.1_FicAlb1.5 |
| GCF_001577835.1 | Coturnix japonica | Chrom | 001/577/835/GCF-001577835.1-Coturnix-japo nica_2.0 |
| GCF_001465895.1 | Nothobranchius furzeri | Chrom | 001/465/895/GCF_001465895.1_Nfu_20140520 |
| GCF_000523025.1 | Cynoglossus semilaevis | Chrom | 000/523/025/GCF_000523025.1_Cse_v1.0 |

The examples show:
**Example 1:** Design of a nucleic acid library of the invention that encodes short peptides based on amino acid sequences of proteins comprised in the human proteome ("HuPEx").

First, a single "mega protein" amino acid sequence was generated by concatenating the amino acid sequences of a plurality of individual proteins (in this case, all 21,018 such proteins) comprised in the reference proteome of *Homo sapiens.* Hence, such amino acid sequences can be considered to be of naturally occurring proteins; that is they occur naturally in humans. The reference proteome used (UP000005640_9606.fasta) was obtained from the EMBL-EBI web-based resource: "Reference proteomes - Primary proteome sets for the Quest For Orthologs" (http://www.ebi.ac.uk/reference_proteomes, accessed on 05-Feb-2017), Release 2017_01 based on UniProt Release 2017_01.

Each join between the amino acid sequences of two concatenated proteins was marked by using a spacer symbol "_", such that (for example) two example proteins listed, in FASTA-format, in a human reference proteome:
>tr|A0A024R161|A0A024R161_HUMAN Guanine nucleotide-binding protein subunit gamma OS=Homo sapiens GN=DNAJC25-GNG10 PE=3 SV=1
>tr|A0A075B6F4|A0A075B6F4_HUMAN T-cell receptor beta variable 21/OR9-2 (pseudogene) (Fragment) OS=Homo sapiens GN=TRBV21OR9-2 PE=4 SV=1

would be concatenated to form a single amino acid sequence, with the region around the join shown below:
[...] LVGVPAGSNPFREPRSCALL_XRFLSEPTRCLRLLCCVALS [...]

Second, a computer program selected all 46 amino acid-long regions that were spaced apart by a window of 10 amino acids from the "mega protein" amino acid sequence representing the plurality of proteins (in this case, all 21018. However in other embodiments the computer program could be instructed to select amino acid regions having an alterative (predefined) length, for example any such length that is between 25 amino acids and 110 amino acids; and/or the computer program could be instructed to space such regions apart by an alterative (predefined) window, for example such a window of between about 2 and 40 amino acids. For example, a filing tiling of such a selection of 46 amino acid regions spaced by a 10 amino acid window from a concatenation of the first proteins above would generate the following amino acid sequences representing short peptides (only the first three such peptides sequences are shown):

Third, the resulting set of 46 amino acid-long peptide sequences where filtered by removing from the set any sequences with any one (or more) of the following features:
(a) Any sequence having a length NOT equal to 46 amino acids (both, as a quality-control step for the computer program, and to remove any - short - sequences arising from the end of a protein sequence);
(b) Any sequence comprising the spacer symbol (in this case, "_") representing a join between the amino acid sequences of two proteins;
(c) Any sequence comprising a symbol for an ambiguous amino acid (for example, "B", "J", "X" or "Z"; where in certain databases, such ambiguity codes can have the following meanings: B = D or N, J = I or L, X = unknown, Z = E or Q; as an example, the first 46 amino acid-long peptide sequence of the second protein shown above - as it starts with an "X" - would hence be removed from the set); and/or
(d) Any sequence that is NOT unique (that is, any sequence that is 100% identical to any other sequence).

Fourth, the isoelectric point (pI) of each resulting peptide sequence remaining in the set was predicted using generally available pI-prediction software (in this case, the "pI" function of the "R peptide Package"; Osorio et al 2015, The R Journal. 7:4; https://cran.r-project.org/web/packages/Peptides/index.html; using the argument pKscale = "EMBOSS"). Any peptide having an amino acid sequence predicted to have a pI of between 6 and 8 was also removed from the set. The isoelectric point of a peptide is the pH at which the peptide would have no net charge, and can often precipitate out of a solution of such pH. Accordingly, peptides with a predicted pI of around physiological pH (e.g. 6 to 8) are, according to the invention excluded from the set, as they may be those more likely to have unfavourable properties under such conditions (e.g., by precipitating upon expression).

Fifth, the resulting set of amino acid sequences (after the filtering described in the third and second step) was subjected to a final filtering to positively select only those 46 amino acid sequences that showed 100% identity to an amino acid sequence present in the reference proteome of Mouse *(Mus musculus,* UP000000589_10090.fasta, obtained as described above).

As will now be understood by the person of ordinary skill, the final set of (46-long) amino acid sequences will be spaced apart along the naturally occurring protein(s) by a window of (in this case) 10 amino acids or multiples of 10 amino acids. This is because one or more intervening amino acid sequences may have been omitted from the final set because it did not conform to one or other of the various filtering criteria.

The final set of (46-long) amino acid sequences consisted, in this example, of about 300,000 individual sequences, with at least one such sequence representative of over 21,000 different proteins of the human proteome, and a mean of about 14.2 such sequences per protein and a standard deviation of 3.8 sequences (with 95% of the proteins will be represented by between 7.6 and 20.7 peptide sequences).

As will now be apparent to the person of ordinary skill, the final set of amino acid sequences of peptides that represent the proteome(s) used for the selection may be more or less than this number, and the distribution of the number of peptides for each of the proteins may also differ. Not only will the number and nature of the final set of amino acid sequences depend on the sequences of the plurality of proteins first concatenated (for example, the proteome of a single species used, or those of multiple species), but other factors can affect the final set. For example, the sequence regions initially selected may be shorter (or longer) than the 46 amino acids used herein, and/or spacing window may be shorter (or longer) than the 10 amino acids used herein. Furthermore, one or more of the filtering criteria described above may be omitted, or different filtering criteria may be applied instead or as well (examples of other filtering steps are described in other examples herein). Indeed, depending on the particular properties (such as size, diversity, coverage and/or solubility etc.) desired for a library of the present invention, and/or the method of its physical preparation (such as described below), and in particular the limitations in size, complexity or cost of the method used for its physical preparation, the person of ordinary skill will now have the ability to select only those amino acid sequences for peptides to form any particular library suitable for their needs.

Six, each 46 amino acid sequence was reverse translated using for each amino acid the most frequently used codon for such amino acid of the human codon frequency table shown in Table 1.1 below (Codon Usage Database; Nakamura et al, 2000, NAR 28:292; http://www.kazusa.or.jp/codon/cgi-bin/showcodon.cgi?species=9606&aa=1&style=N, accessed 04-Jun-2017).

As will now be apparent to the person of ordinary skill, however, alterative human codon frequency tables may be used or, and depending on the species of the expression system in which the nucleic acid library is intended to be expressed, codon frequency tables of other species may be used.

**Table 1.1: Human codon frequency table (codon | aa | fraction per codon per aa).**

| | | | | | | | | | | | |
|---|---|---|---|---|---|---|---|---|---|---|---|
| UUU | F | 0.46 | UCU | S | 0.19 | UAU | Y | 0.44 | UGU | C | 0.46 |
| UUC | F | 0.54 | UCC | S | 0.22 | UAC | Y | 0.56 | UGC | C | 0.54 |
| UUA | L | 0.08 | UCA | S | 0.15 | UAA | * | 0.30 | UGA | * | 0.47 |
| UUG | L | 0.13 | UCG | S | 0.05 | UAG | * | 0.24 | UGG | W | 1.00 |
| CUU | L | 0.13 | CCU | P | 0.29 | CAU | H | 0.42 | CGU | R | 0.08 |
| CUC | L | 0.20 | CCC | P | 0.32 | CAC | H | 0.58 | CGC | R | 0.18 |
| CUA | L | 0.07 | CCA | P | 0.28 | CAA | Q | 0.27 | CGA | R | 0.11 |
| CUG | L | 0.40 | CCG | P | 0.11 | CAG | Q | 0.73 | CGG | R | 0.20 |
| AUU | I | 0.36 | ACU | T | 0.25 | AAU | N | 0.47 | AGU | S | 0.15 |
| AUC | I | 0.47 | ACC | T | 0.36 | AAC | N | 0.53 | AGC | S | 0.24 |
| AUA | I | 0.17 | ACA | T | 0.28 | AAA | K | 0.43 | AGA | R | 0.21 |
| AUG | M | 1.00 | ACG | T | 0.11 | AAG | K | 0.57 | AGG | R | 0.21 |
| GUU | V | 0.18 | GCU | A | 0.27 | GAU | D | 0.46 | GGU | G | 0.16 |
| GUC | V | 0.24 | GCC | A | 0.40 | GAC | D | 0.54 | GGC | G | 0.34 |
| GUA | V | 0.12 | GCA | A | 0.23 | GAA | E | 0.42 | GGA | G | 0.25 |
| GUG | V | 0.46 | GCG | A | 0.11 | GAG | E | 0.58 | GGG | G | 0.25 |

Seven, the resulting nucleic acid sequences encoding for the peptides were then analysed for undesired sub-sequences resulting from combinations of codons. Particular examples of such undesired sub-sequence include an internal Kozak sequence and/or a restriction enzyme site for a restriction enzyme intended to be used for the cloning of the resulting library, in each case generated from a combination of codons. If an undesired sub-sequence was present in a nucleic acid sequence, then the next most commonly used codon was used in place of one or other of the codons in the combination such that undesired subsequence was no longer present in the nucleic acid sequence.

In this example, a Kozak sequence of "CCATGG" was deemed undesired, and any codon combinations in the nucleic acid sequences that formed such a sub-sequence were adapted to use a less frequent codon so that such sub-sequence was no longer present.

As the library of this example was to be cloned into expression vectors using certain restriction enzymes, any sub-sequences in nucleic acid sequences that were recognition sites for such restriction enzymes were also deemed undesirable. The restriction enzymes BamHI and XhoI were envisioned as restriction enzymes that may be sued in such cloning procedures, and hence any of the following sub-sequences (the respective recognition site for such restriction enzymes) were deemed undesirable: 5'GGATCC and 5' CTCGAG. Any codon combinations in the nucleic acid sequences that formed such any sub-sequence that was such a (predefined) restriction enzymes recognition site were adapted to use a less frequent codon so that such sub-sequence was no longer present. As will be now apparent to the person of ordinary skill, depending on any restriction enzyme(s) planned to be used, the applicable sub-sequence consisting of a recognition site of such restriction enzyme(s) can be so removed by appropriate use of an alternative codon.

As will also be apparent to the person of ordinary skill, as the nucleic acid sequences were generated by reverse translation of the (naturally occurring) amino acid sequences, the resulting library of nucleic acid sequences will comprise nucleic acids sequences that themselves are non-natural, as codons will be used (and/or combinations or codons) that are not those used by the naturally occurring genomic sequence to code one or more particular amino acids at that position in the protein. Accordingly, the nucleic acid libraries described herein will comprise a plurality of non-natural nucleic acid sequences.

**Example 2:** Synthesis of the HuPEx nucleic acid library of the invention.

First, each nucleic acid sequence in the library designed in Example 1 to encode for unique 46 amino acid long peptide "fragments" of naturally occurring human proteins was adapted with 5' and 3' sequences adapted to provide the sequence of an oligonucleotide enable cloning of the nucleic acid library and/or to enable expression of the peptide encoded therein.

In this example, the general structure of each resulting nucleic acid sequence of such oligonucleotide (with the 5' and 3' regions) was:
Forward-amp_BamHI_Kozac_Gly_VARIABLE-REGION_Stop_XhoI_Reverse-amp,

where "Forward-amp" and "Reverse-amp" represent nucleic acid sequences chosen so that the resulting oligo could be amplified by PCR using appropriate primers; "BamHI" and "XhoI" represent nucleic acid sequences of the respective restriction enzyme's recognition site; "Kozak" represents a Kozak sequence (including a start codon); "Gly" represents a codon for a single glycine acid-linker; "Stop" represents a stop codon; and "VARIABLE-REGION" represents an individual 138bp nucleic acid that encodes a given 46 amino acid long peptide (present in the set), from the library of nucleic acid designed in Example 1.

In this example, the forward and reverse amplification sequences, Kozak sequence and stop codon used is set forth in Table 2.1 below.

**Table 2.1: Common sequences used in the HuPEx library.**

| **Feature** | **Sequence (5'to 3')** | **SEQ ID NO.:** |
|---|---|---|
| Forward amplification | TGCCACCTGACGTCTAAGAA | 1 |
| Reverse amplification | GCTCACTCAAAGGCGGTAAT | 2 |
| Kozak sequence | CCATGG | N/A |
| Stop codon | TAA | N/A |

Accordingly, a nucleic acid sequence of an indicative oligonucleotide resulting from such design and encoding for the following indicative 46 amino acid sequence **(SEQ ID NO. 13)** present in the set:

### LAQTACVVGR PGPHPTQFLA AKERTKSHVP SLLDADVEGQ SRDYTV,

has a complete nucleic acid sequence shown below (SEQ ID NO. 3), where the following features are marked as follows: 138bp region coding for the above 46 amino acid sequence in bold; forward and reverse amplification sequences in lower case; restriction enzyme sites boxed, Kozak sequence double underlined; and "^{∗}" is indicated above the first base of the start and stop codons.
**SEQ ID NO. 3:** Nucleic acid sequence of an indicative oligonucleotide

As will now be apparent to the person or ordinary art, the nucleic acid sequence of the final oligonucleotides may be, for example, alternatively designed not to comprise a Kozak/start or stop codon(s). In this case, the Kozak/start and stop codon(s) can be provided by the host vector. Such a design (as demonstrated in other examples below) can allow the libraries to be produced in tagged format, by cloning them into a vector with, for example, a vector-encoded FLAG tag, N- or C-terminally to the encoded peptide.

In alternative embodiments, two versions of a library of the invention may be constructed: one with and one without a start codon. Such an embodiment can be useful as an internal reference for data analysis of screens using the library, as it would allow effects of the peptide from effects of the vector/construct itself (which should be identified as false positives) to be distinguished. For example, to distinguish those cases where particular sequences are amplified better by PCR, DNA sequences that attract some cellular machinery and cause indirect effects etc.

Second, the set of nucleic acid sequences of all approximately 300,000 oligonucleotides was used to chemically synthesis each of the oligonucleotides by conventional approaches.

In this case, the semi-pooled 10,000-well silicon chip-based oligonucleotide synthesis approach of Twist Bioscience (San Francisco, California) was used to synthesise all approximately 300,000 oligonucleotides, and the oligonucleotides were therefore available in sub-pools each having a complexity of approximately 2,000 oligonucleotides. It should be noted here, that if each sub-pool-synthesised oligonucleotide where synthesised with a different combination of pairs of primer-pairs (for example, 45 different primer-pairs used in all combinations would provide 45^{∗}45 = 2025 different combinations), then it would then be possible to recover an ^{∗}individual^{∗} sequence by conducting PCR with only using the applicable specific primer-pair combination. However, other synthetic oligonucleotide approaches could be used, such as array-based synthesis (e.g., Affymetrix) or in-situ synthesis printing (Agilent).

**Example 3:** Cloning of the HuPEx nucleic acid library of the invention into expression vectors.

The set of approximately 300,000 oligonucleotides were (in pooled or sub-polled format) cloned into a lentiviral expression system briefly described as follows.

First, a pool of oligonucleotides was subjected to PCR amplification (by standard procedures and using the primers having the sequences 5'-TGCCACCTGACGTCTAAGAA-3' **(SEQ ID NO. 4)** and 5'-ATTACCGCCTTTGAGTGAGC-3¹ **(SEQ ID NO. 5),** corresponding to the forward and reverse amplification sequences, respectively, in the oligonucleotides. Second, the resulting product was digested with the applicable restriction enzymes (in this case BamHI and XhoI) to provide sticky-ended constructs for cloning.

Third, the sticky-ended constructs were ligated into a sample of a BamHI/XhoI-digested lentiviral vector (for example, pMOST25). Sixty (60) base pairs of the cloning site of pMOST25 is shown below by SEQ ID NO. 6, and the resulting recombinant construct with a BamHI/XhoI-digested amplification product of the indicative oligonucleotide shown in Example 2, would have the sequence shown by SEQ ID NO. 7. BamHI and XhoI restriction sites are boxed; the Kozak sequence is double underlined; the first base of the start/stop codons marked above with a "^{∗}"; and the 138bp region coding for the 46 amino acid sequence in bold.
**SEQ ID NO. 6:** 60bp of the cloning site of pMOST25 lentiviral vector.
**SEQ ID NO. 7:** Indicative recombinant construct.

Transformation/transfection of the recombinant vector into a host cell will enable propagation, expression and/or screening of the nucleic acid library of the invention. As will be apparent to the person of ordinary skill, the a nucleic acid library - after being amplified and/or propagated/maintained in host cells - may be considered to no longer be "synthetic", as the nucleic acid molecules will, by then, have been enzymatically (either in-vitro or in-vivo) produced. However, such a nucleic acid library is still considered to be one of the present invention.

Expression of the construct shown in SEQ ID NO. 7 will result in the 48 amino acid peptide having a sequence as shown in SEQ ID NO. 8, where the sequence of the originally encoded 46 amino acid indicative peptide is shown in bold after the initial methionine and the linking glycine.
**SEQ ID NO. 8:** Indicative expressed peptide.
MG**LAQTACVV GRPGPHPTQF LAAKERTKSH VPSLLDADVE GQSRDYTV**

As the person or ordinary skill we note, such expressed comprises N- and C-terminal amino acids corresponding to the 5' and 3' restriction enzyme recognition sites (and, in this case, a single linking Val). Accordingly, even though the original 46 amino acid sequence is a fragment of a naturally occurring protein, the resulting 52 amino acid expressed peptide is a non-natural. Accordingly, the libraries of described herein will encode for a plurality of non-natural peptides.

The cloning of the library can be conducted in a pooled manner, or in a semi-polled manner. Furthermore, the resulting pooled/sub-pooled clones can be individually picked/arrayed using automated cell-sorting technologies, such as the QPix (Molecular Devices), FACS or single-cell dispensing (e.g. VIPS^{™} Cell Dispenser or Solentim Ltd, UK, or the Single Cell Printer - SCP - or Cytena GmbH, Germany).

**Example 4:** Phenotypic screening with the lentiviral-cloned HuPEx library.

A HuPEx library, such as designed and constructed above, was used to screen for phenotypic alterations using the assay formats described above, either as pooled or arrayed libraries. Prior to such screen, the HuPEx library can, alternatively, be pooled with one or more other analogous libraries that expressed short peptides. For example, the HuPEx library can be screened in a pool together with a library that expresses human small Open Reading Frames (sORFs), such as the sORF library described in WO 2017/109499 (in particular, in Example A) of WO 2017/109499).

### (1) Pooled 6-thioguanine resistance screen

Resistance to the chemotherapeutic drug 6-thioguanine has been previously demonstrated to be a fairly strict selection system; with a narrow group of proteins being able to mediate the phenotype (see Wang et al 2014, Science 343:80). The inventors sought to use this system to demonstrate how a library of the present invention can be utilised in identifying phenotype-modulating proteins even under such stringent conditions.

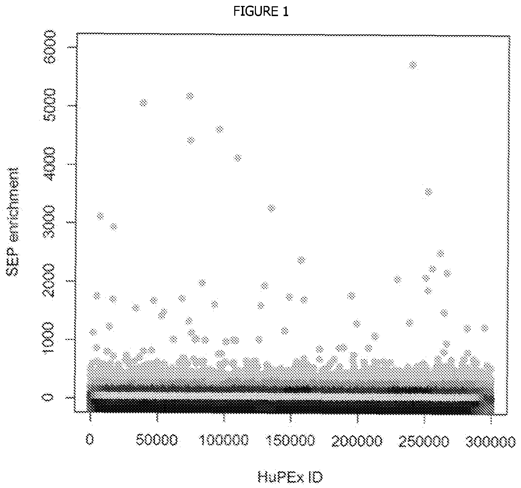

HEK293 cells were transfected with the pooled HuPEx nucleic acid library, cloned into the lentiviral vector as above, which is designed to express a plurality of short-expressed-peptides (SEPs). Virus was harvested, titered, and a batch of KBM7 cells was infected with the SEP-expressing viruses. The library of virus-transduced KBM7 cells was subsequently exposed to a concentration of 6-thioguanine that was experimentally determined to kill 99.999% of KBM7 cells. Survivors, carrying inserts that express resistance-inducing SEPs, were isolated from the pool, expanded and genomic DNA was harvested. The inserts that express resistance-inducing SEPs were amplified using PCR and submitted to Next Generation Sequencing. After bioinformatics analysis of the data, SEPs that mediate the resistance to 6-thioguanine, and likely acting upon mismatch repair processes, were identified **(****Figure 1****).**

### (2) Pooled PTEN synthetic lethality screen

To find SEPs able to selectively inhibit proliferation or kill cells lacking the PTEN tumour suppressor, SEPs were screened in an isogenic cell model pair (MCF10A WT and MCF10A PTEN knockout).

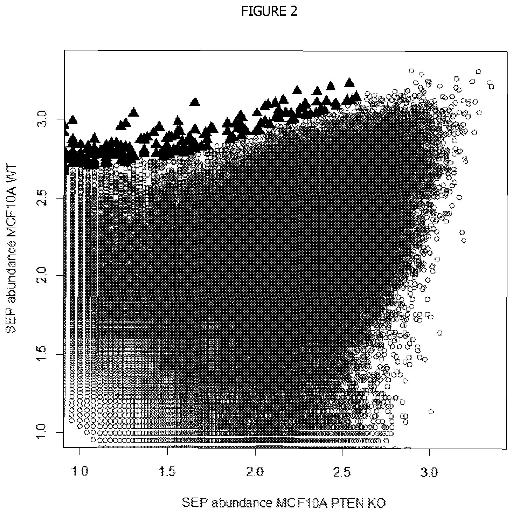

As in (1) above, a pool of cells was infected with a library of HuPEx expressing SEPs. Cells were also infected with BugPEx and OmePEx libraries in a similar fashion. Under all conditions, the target cell lines, MCF10A and MCF10A PTEN KO, were infected in parallel. The cells were plated at low density in order to allow for cell growth over a period of five days. Samples from either cell population were then submitted to NGS as in (1). The relative abundances of SEP sequences in the wildtype-control control set (MCF10A) and the PTEN knockout set (MCF10A PTEN KO) were compared, and SEPs depleted in the knockout cells were identified **(****Figure 2****).**

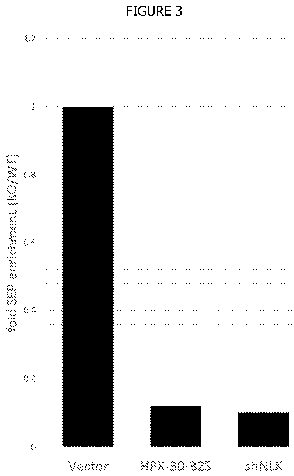

Identified hits were then validated in the same models by repeating the primary assay with additional controls. Hits were considered validated if they showed a significant reduction of cell growth in MCF10A PTEN KO cells compared to MCF10A cells in three biological replicates. Figure 3 shows an example validated hit being HuPEx sequence #30-325, a 46 amino acid sequence from human Tetraspanin-3 (the sequence of which, together with the two leading two amino acids - "MG" - as it is expressed by pMOST25 is shown as **SEQ ID NO. 15),** which inhibited growth > 60% in PTEN knockout cells compared to the control cell line, to an amount comparable with a positive control (shRNA against NLK, previously described to be synthetic lethal with PTEN knockout. Mendes-Pereira et. Al, PlosONE 2012). The nucleic acid sequence of the oligonucleotide that was synthesised to express such peptide is shown in **SEQ ID NO. 16).**

**Example 5:** Design and construction of a nucleic acid library of the invention that encodes short peptides based on amino acid sequences of proteins comprised in the proteomes of evolutionary diverse microbiota ("BugPEx").

A library of the invention was designed and constructed wherein the amino acid sequences of the source-proteins were those of naturally occurring proteins from a plurality of different species; in this example, the protein sequences comprised in the reference proteomes of an evolutionary diverse set of micro-organisms.

First, a mega protein amino acid sequence was generated as described in Example 1, but by using all the protein sequences comprised in the set of reference proteomes set out Table A. The second, third and fourth steps of Example 1 were conducted by analogy, to generate a filtered set of several hundred thousand unique 46 amino acid long sequences.

In this case however, the fifth step of Example 1 was replaced by an alternative filtering step to select, from such list of several hundred thousand amino acid sequences, 500,000 of such sequences that were predicted to be least likely to have a disordered segment. For example, the program DisEMBL (Linding et al 2003, Structure 11:1453; http://dis.embl.de) can be applied to consider and sort on the three intrinsically disordered protein parameters of loops/coils, hot-loops and Remark-465. The resulting number of amino acids present in a disordered stretch in a given peptide is counted, and such counts are ranked for each of the three parameters. The peptide sequences are then ranked by the mean of all three ranked-parameters so that peptides predicted to be highly disordered are ranked at the bottom of the list. Alternatively, peptides having a long disordered segment can be predicted using SLIDER (Super-fast predictor of proteins with long intrinsically disordered regions; Peng et al, 2014; Proteins: structure, Function and Bioinformatics 82:145; http://biomine.cs.vcu.edu/servers/SLIDER) using default parameters.

The resulting 600,000 amino acid sequences in the filtered set were reverse-translated as described to nucleic acid sequences in the sixth step of Example 1, with alterative codons used to avoid undesired codon-combinations as described by the seventh step in Example 1. With an estimated 80,000 naturally occurring proteins represented by such 600,000 peptides, this suggests a mean coverage of about 7.5 nucleic acids (peptides) per naturally occurring protein

For synthesis of oligonucleotides comprising these 600,000 nucleic acid sequences, the procedure described in Example 2 was followed except that in this case the general structure of the resulting oligonucleotide (with the 5' and 3' regions) was:
Forward-amp_BamHI_Val_VARIABLE-REGION_XhoI_Reverse-amp,
where "Val" represents a codon for a single valine amino acid-linker, and the other features are as described in Example 2, such that or an indicative 46 amino acid sequence **(SEQ ID NO. 14)** of:

### PRYLKGWLKD WQLSLRRPS FRASRQRPII SLNERILEFN KRNITA,

the resulting oligonucleotide sequence has a complete nucleic acid sequence shown below (SEQ ID NO. 9), where the following features are marked as follows: 138bp region coding for the above 46 amino acid sequence in bold; forward and reverse amplification sequences in lower case; restriction enzyme sites boxed.
**SEQ ID NO. 9:** Nucleic acid sequence of an indicative oligonucleotide

Note that in this case, the oligonucleotide does not comprise a Kozak/start or a stop codon, which in this example is provided by the expression vector into which the oligonucleotide is cloned, as described next.

The oligonucleotides were amplified and digested as described in first and second steps of Example 3. In this case, however, the resulting digest products were cloned into a sample of a BamHI/XhoI-digested lentiviral vector comprising a Kozak/start and stop codon (eg pMOST25A). Sixty (60) base pairs of the cloning site of pMOST25A is shown below by SEQ ID NO. 10, and the resulting recombinant construct with a BamHI/ Xhol-digested amplification product of the indicative oligonucleotide shown in this Example 5, would have the sequence shown by SEQ ID NO. 11. BamHI and XhoI restriction sites are boxed; the first base of the start/stop codons marked above with a "^{∗}"; and the 138bp region coding for the 46 amino acid sequence in bold.
**SEQ ID NO. 10:** 60bp of the cloning site of pMOST25A lentiviral vector.
**SEQ ID NO. 11:** Indicative recombinant construct.

Expression of the construct shown in SEQ ID NO. 11 will result in the 52 amino acid peptide having a sequence as shown in SEQ ID NO. 12, where the sequence of the originally encoded 46 amino acid indicative peptide is shown in bold after the initial methionine, the two amino acids encoded by the BamHI site and the linking valine, followed by the two amino acids encoded by the XhoI site.
**SEQ ID NO. 12:** Indicative expressed peptide.
MGSV**PRYLKG WLKDVVQLSL RRPSFRASRQ RPIISLNERI LEFNKRNITA** LE

**Example 6:** Design and construction of a nucleic acid library of the invention that encodes short peptides based on amino acid sequences of proteins comprised in the proteomes of evolutionarily diverse organisms ("OmePEx").

Another library of the invention was designed and constructed wherein the amino acid sequences of the source-proteins were those of naturally occurring proteins comprised in the reference proteomes of a yet more evolutionary diverse set of species, including a number of species from each of those being: archaea; bacteria, fungi, invertebrates, plants, protozoa, mammals and non-mammalian vertebrates.

First, a mega protein amino acid sequence was generated as described in Example 1, but by using all the protein sequences comprised in 467 reference proteomes set out Table B. The second and third steps of Example 1 were conducted by analogy, to generate a pre-filtered set of over 1 million unique 46 amino acid long sequences.

This set of pre-filtered sequences were subjected to hierarchical clustering using iterative runs of CD-HIT (Fu et al 2012, Bioinformatics 28:3150; http://weizhongli-lab.org/cd-hit/) with more stringent thresholds to obtain maximum diversity in the resulting peptides, described briefly as follows: Three rounds of clustering were performed; a first round used an 80% sequence similarity threshold parameter ("-c 0.8"; example command: cdhit -i peptides. faa -o peptides_0.8 . faa -c 0.8 -T 0 -M 32000 -n 5); a second round, using the output of the first round, used a 60% sequence similarity threshold ("-c 0.6"); and a third and final run, using the output of the second round, used a sequence similarity threshold of 50% ("-c 0.5").

The isoelectric point (pI) of each resulting peptide sequence remaining was predicted, and any having a predicted pI of between 6 and 8 was also removed from the set, as described in step four of Example 1.

Analogously to as described in Example 2, the resulting set of amino acid sequences were further filtered to remove those predicted to have intrinsically disordered regions. In this case however, the top 475,000 sequences (ie, those least predicted to comprise intrinsically disordered regions) were selected to form a first set of unique 46 amino acid sequences.

Separately, a second set of 25,000 unique 46 amino acid sequences were generated using the procedure described above in this Example 6, except that the amino acid sequences of the naturally occurring proteins first concatenated were those proteins having a known three-dimensional structure. In this case, about 10,150 polypeptide chains present in Protein Data Bank (https://www.wwpdb.org, on 5-Feb-2017) and that had a Pfam annotation (http://pfam.xfam.org, version 30.0).

The first and second sets of amino acid sequences were combined, and 500,000 oligonucleotides, each encoding a separate of the amino acid sequences, was designed (having the same general structure) and synthesises as described in Example 5. The resulting synthesised oligonucleotides were PCR amplified, BamHI/XhoI digested and cloned into pMOST25A as described in Example 5 to form an expression library of the invention.

**Example 7** [prophetic]: Design and construction of a nucleic acid library that encodes short peptides based on differentially expressed proteins ("DiffPEx").

In this example, a query of a gene-expression database (e.g. the EMBL-EBI Expression Atlas; Geenhttp://www.ebi.ac.uk/gxa/home/) is conducted to identify a sub-set of genes in the proteome that are differentially expressed between two tissue types. For example, the 5,000 most differentially expressed proteins between a human melanoma cell line (or patient sample) and a comparable but non-cancerous human cell line are identified by such a query.

The reference amino acid sequences of this set of 5,000 proteins are used to generate a filtered set of over 20,000 unique 46 amino acid sequences and over 20,000 oligonucleotides, each encoding a separate of the amino acid sequences, are designed (having the same general structure), synthesises and cloned into pMOST25A as described in Example 5; *except* that in this case: (1) the window spacing along each protein's amino acid sequence is less than 10 amino acids so as to increase the density of tiling of the amino acid sequences across the sequence of these naturally occurring proteins; and (2) the SLIDER program (for prediction and filtering out of unstructured regions) is not used.

**Example 8** [prophetic]: Generation of peptide libraries encoded by nucleic acid libraries of the invention.

A library of peptides of the invention (eg, one encoded by a nucleic acid library of the invention) is generated as follows.

First, the amplified and BamHI/XhoI-digested oligonucleotides of Example 7 are, instead, cloned into a CIS display construct (Odegrip et al 2003; PNAS 101:2806), having the general design:
Promoter_NUCLEIC_ACID LIBRARY_repA_CIS_ori

CIS display exploits the ability of a DNA replication initiator protein (RepA) to bind exclusively to the template DNA from which it has been expressed, a property called cis-activity. The peptide library is created by ligation of a nucleic acid of the invention to a DNA fragment that encodes RepA. After in vitro transcription and translation, a pool of protein-DNA complexes is formed where each protein is stably associated with the DNA that encodes it. These complexes are amenable to the affinity selection of ligands to targets of interest.

CIS display, exploits the high-fidelity cis-activity that is exhibited by a group of bacterial plasmid DNA-replication initiation proteins typified by RepA of the R1 plasmid (Nikoletti et al 1988, J. Bacteriol. 170:1311). In this context, cis-activity refers to the property of the RepA family of proteins to bind exclusively to the template DNA from which they have been expressed. R1 plasmid replication is initiated through the binding of RepA to the plasmid origin of replication (*ori*)*. Ori* is separated from the RepA-coding sequence by a DNA element termed *CIS.* This element is thought to be critical in controlling the cis- activity of RepA (Masai & Arai 1988, Nucleic Acids Res. 16:6493). The consensus model for cis-activity is that the *CIS* element, which contains a rho- dependent transcriptional terminator, causes the host RNA polymerase to stall. This delay allows nascent RepA polypeptide emerging from translating ribosomes to bind transiently to *CIS,* which in turn directs the protein to bind to the adjacent ori site (Praszkier & Pittard 1999, J. Bacteriol. 181:2765).

By genetically fusing peptide libraries to the N-terminus of the RepA protein, we can achieve a direct linkage of peptides to the DNA molecules that encode them; thus, the link between genotype to phenotype that is the common feature of display technologies is established.

The peptide library is generated by in-vitro transcription and translation using an *E.coli* lysate system as described by Odegrip et al (2003), and solid phase selection for peptides (and hence the DNA sequence encoding them) that bind to immobilised target can be conducted (e.g., by one or more rounds of selection) also as described by Odegrip et al (2003).

**Example 9:** Phenotypic screening with the lentiviral-cloned HuPEx library, and identification of SEP-binding targets.

Using a phenotypic screen, the inventors were able to identify short-expressed-peptides (SEPs) from the HuPEx library described above that increased the survival of HeLa cells treated with methylnitronitrosoguanidine (MNNG), an inducer of cell death via parthanatos. Parthanatos, is a PARP-1 dependent form of programmed cell death (Yu et al, 2006; PNAS 103: 2653) that plays a role in neuronal cell death and is associated with diseases including Parkinsons disease, stroke, heart attack and diabetes.

HEK293 cells were transfected with the pooled HuPEx nucleic acid library, cloned into the lentiviral vector as above, which is designed to express a plurality of SEPs. Virus was harvested, titered, and a batch of HeLa cells was infected with the SEP-expressing viruses and selected for 8 days. The library of virus-transduced HeLa cells was then exposed ("D0" time point) to a near-lethal dose (6.7uM) of MNNG. This dose was established as the maximum dose where co-incubation with the PARP-1 inhibitor olarparib was still able to rescue HeLa cells from parthanatos/cell-death.

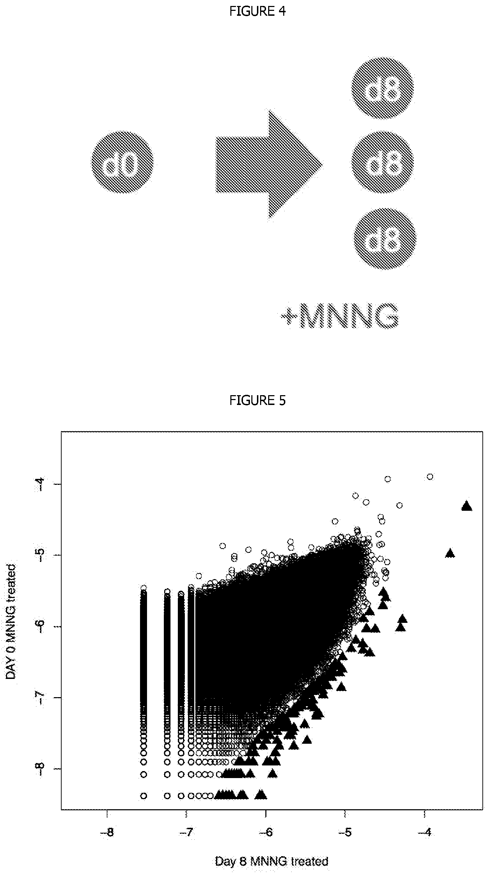

Genomic DNA was extracted from a control aliquot of such HeLa cells without MNNG treatment at D0, and large-scale amplicon DNA sequencing was performed to determine the relative abundance of HuPEx SEP-coding nucleic acids, and again from a second aliquot of such HeLa cells after culture for 8 days ("D8") in the presence of 6.7uM MNNG (Figure 4), so as to identify those expressed SEPS from the HuPEx library that showed an increased relative abundance at D8 compared to D0, and thus increased the survival of HeLa cells in the presence of MNNG.

Of a predicted 300,000 different SEP-coding inserts represented in the HuPEx library, almost 288,000 were represented at least one member (as found by amplicon DNA sequencing) at D0; and by showing a significant increase in relative abundance at D8, at least 72 SEPs were identified from the HuPEx library as increasing the survival of HeLa cells in the presence of MNNG (Figure 5).

Of these SEPs, a number were determined to be fragments of naturally occurring proteins that were associated with detoxification mechanisms. For example, a KEGG-pathway analysis (http://www.kegg.jp/kegg/pathway.html) determined that certain of such SEPs were fragments of naturally occurring proteins involved in "chemical carcinogenesis" and "metabolism of xenobiotics by cytochrome p450).

Certain of the identified SEPs were used as a "bait" in yeast 2-hybrid screening technology (against a human cDNA library as prey) to identify the protein target(s) that the HuPEX SEP would bind to in HeLa cells.

**Example 10:** Phenotypic screening with the lentiviral-cloned BugPEx and OmePEx libraries.

The BugPEx and the OmePEx libraries were screened in a phenotypic screen of parthanatos, analogously to as described in Example 9, to identify SEPs expressed from each of these libraries that showed increased relative abundance at D8 compared to D0, and hence were able to increase the survival of HeLa cells in the presence of the parthanatos-inducing MNNG.

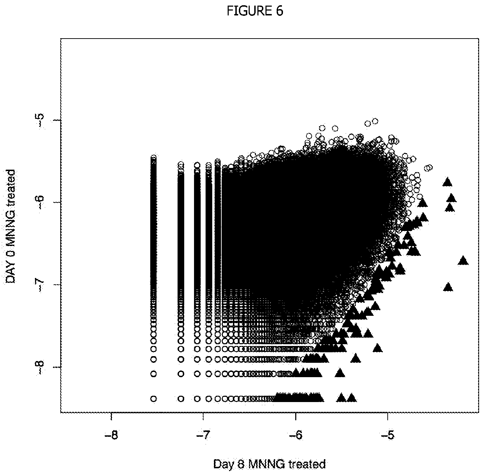

Of a predicted 600,000 different SEP-coding inserts represented in the BugPEx library, almost 510,000 were represented at least one member (as found by amplicon DNA sequencing) at D0; and by showing a significant increase in relative abundance at D8, at least 58 SEPs were identified from the BugPEx library as increasing the survival of HeLa cells in the presence of MNNG (Figure 6).

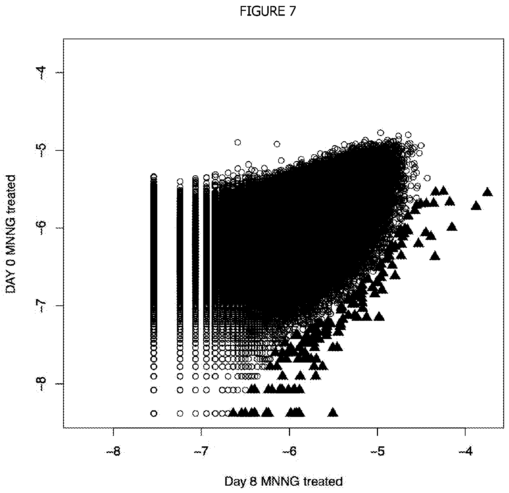

Of a predicted 500,000 different SEP-coding inserts represented in the OmePEx library, almost 490,000 were represented at least one member (as found by amplicon DNA sequencing) at D0; and by showing a significant increase in their relative abundance at D8, at least 64 SEPs were identified from the OmePEx library as increasing the survival of HeLa cells in the presence of MNNG (Figure 7).

**Example 11:** Phenotypic screening with the lentiviral-cloned -PEx libraries

Using a phenotypic screen, the inventors were able to identify a short-expressed-peptide (SEP) from the combined HuPEx (HPX), BugPEx (BPX) & OmePEx (OPX) libraries described above that decreased GFP-LC3 in HEK293FT cells engineered to express GFP-LC3/RFP-LC3DG Autophagic Flux Reporter (AFR cells, Kaizuka et al. Molecular Cell 2016).

HEK293FT cells were transfected with the pooled HuPEx, BugPEx & OmePEx nucleic libraries, cloned into the lentiviral vector as described herein, for example pMOST25a as shown in SEQ ID No. 10 , which is designed to express a plurality of SEPs. Virus was harvested, titered, and a batch of HEK293FT-AFR cells was infected with the SEP-expressing viruses and selected for 4 days and SEP expressing cells were then expanded for a further 2 days without selection. The library of virus-transduced HEK293FT-AFR cells were then assessed by flow cytometry, SEP transduced HEK293FT-AFR cells enriched in the low GFP-LC3 gate, compared to unsorted controls, were flow-sorted and peptide sequences were amplified and sent to NGS analysis i.e. amplicon DNA sequencing as described in the previous example . Figures 8A-C show the population of selected hits (marked region) compared to control.

SEPs sequences identified as being enriched in low GFP-LC3 gate were cloned into suitable lenti-viral expression and SEP-expressing virus generated in HEK293FT cells. Each SEP-expression virus population was individually assessed by flow cytometry in conditions described above. Each SEP expressing population was assessed in comparison to control-SEP expressing populations or uninfected HEK293FT-AFR cells treated with Torin1, an inducer of Autophagic Flux (Figure 9). A selection of candidates is shown with BPX-497507 representing a strong and robust hit able to induce Autophagy as measured by GFP-LC3 reduction. Torin1 (250nM) is shown as a positive control.

## Claims

1. **A library of nucleic acids,** each nucleic acid comprising a coding region of defined nucleic acid sequence encoding for a peptide having a length of between 25 and 110 amino acids, and having an amino acid sequence being a region of a sequence selected from the amino acid sequence of a naturally occurring protein of one or more organisms; wherein the library comprises nucleic acids that encode for a plurality of at least 10,000 different such peptides, and wherein the amino acid sequence of each of at least 50 of such peptides is a sequence region of the amino acid sequence of a different protein of a plurality of different such naturally occurring proteins, and wherein each peptide encoded by the library is predicted from its amino acid sequence to have an isoelectric point (pI) of greater than 8.0 or less than 6.0.

2. The library of nucleic acids of claim 1, wherein each of the plurality of different naturally occurring proteins fulfils one or more pre-determined criteria.

3. The library of nucleic acids of claim 2, wherein either:
i) each of the plurality of naturally occurring proteins is associated with a given disease, such as cancer; optionally wherein the disease is breast cancer; or
ii) each of the plurality of naturally occurring proteins is a cytoplasmic protein; optionally wherein each of the plurality of naturally occurring proteins is a cytoplasmic kinase; or
iii) each of the plurality of naturally occurring proteins interacts with a given protein or at least one protein from a (functional) class of proteins; optionally wherein each of the plurality of naturally occurring proteins interacts with KRas.

4. The library of nucleic acids of any one of claims 1 to 3 wherein the library comprises nucleic acids that encode for a plurality of at least 50,000 different such peptides, and wherein the amino acid sequence of each of at least 100 of such peptide is a sequence region of the amino acid sequence of at least 100 different naturally occurring proteins; in particular wherein the library comprises nucleic acids that encode for a plurality of at least 100,000 different such peptides, and wherein the amino acid sequence of each of at least 150 of such peptide is a sequence region of the amino acid sequence of at least 150 different naturally occurring proteins.

5. The library of nucleic acids of any one of claims 1 to 4, wherein the library comprises nucleic acids that encode for a plurality of at least 10,000 different such peptides, and wherein the amino acid sequence of each of at least 1,000 of such peptides is a sequence region of the amino acid sequence of a different protein of such plurality of different naturally occurring proteins.

6. The library of nucleic acids of any one of claims 1 to 5, wherein the library comprises nucleic acids that encode for a plurality of at least 200,000 different such peptides, and wherein the amino acid sequence of each of at least 20,000 of such peptide is a sequence region of the amino acid sequence of at least 20,000 different naturally occurring proteins; in particular wherein the library comprises nucleic acids that encode for a plurality of at least 300,000 different such peptides, and wherein the amino acid sequence of each of at least 25,000 of such peptide is a sequence region of the amino acid sequence of at least 25,000 different naturally occurring proteins.

7. The library of nucleic acids of any one of claims 1 to 6, wherein that in respect of at least about 1% of the naturally occurring proteins a plurality of the nucleic acids encodes for different peptides from the amino acid sequences of such naturally occurring proteins; optionally
wherein that in respect of at least about 50% of the naturally occurring proteins a plurality of the nucleic acids encodes for different peptides from the amino acid sequences of such naturally occurring proteins;
and optionally wherein the plurality of the nucleic acids encodes for different peptides, and the amino acid sequences of which are sequence regions spaced along the amino acid sequence of the naturally occurring protein;
and optionally wherein the sequence regions are spaced by a window of amino acids apart, or by multiples of such window, along the amino acid sequence of the naturally occurring protein wherein, the window is between 1 and about 55 amino acids; in particular wherein the window is between about 5 and about 20 amino acids; most particularly wherein the window of spacing is about 8, 10, 12 or 15 amino acids.

8. The library of nucleic acids of any one of claims 1 to 7 comprising nucleic acids encoding for at least 100,000 different peptides from at least 10,000 different naturally occurring proteins.

9. The library of nucleic acids of any one of claims 1 to 8, wherein each nucleic acid encodes a different peptide.

10. The library of nucleic acids of any one of claims 1 to 9, wherein the mean number of nucleic acids that encode a different peptide from the naturally occurring proteins is greater than 1; in particular between about 1.01 and 1.5 such nucleic acids (peptides) per such protein; optionally
wherein the mean number of nucleic acids that encode a different peptide from the naturally occurring proteins is at least about 5 such nucleic acids (peptides) per such protein, in particular wherein the mean is between about 5 and about 2,000 such nucleic acids (peptides) per such protein or is between about 5 and about 1,000 nucleic acids (peptides) per such protein; and optionally
wherein the mean number of nucleic acids that encode a different peptide from the naturally occurring proteins is between about 100 and about 1,500 such nucleic acids (peptides) per such protein or is between about 250 and about 1,000 such nucleic acids (peptides) per such protein; or
wherein the mean number of nucleic acids that encode a different peptide from the naturally occurring proteins is between about 5 and about 100 such nucleic acids (peptides) per such protein or is between about 5 and about 50 such nucleic acids (peptides) per such protein.

11. The library of nucleic acids of any one of claims 1 to 10, wherein the amino acid sequence of the naturally occurring protein is one selected from the group of amino acids sequences of non-redundant proteins comprised in a reference proteome, suitably, the reference proteome is one or more of the reference proteomes selected from the group of reference proteomes listed in Table A and/or Table B, or an updated version of such reference proteome.

12. The library of nucleic acids of any one of claims 1 to 11, wherein the amino acid sequences of the plurality of encoded peptides are sequence regions selected from amino acid sequences of naturally occurring proteins (or polypeptide chains or domains thereof) with a known three-dimensional structure; in particular wherein the naturally occurring protein (or polypeptide chain or domain thereof) is comprised in the Protein Data Bank, and optionally that has a Pfam annotation; optionally
wherein the sequence region selected from the amino acid sequence of the protein does not include an ambiguous amino acid of such amino acid sequence comprised in the reference proteome or the Protein Data Bank.

13. The library of nucleic acids according to any preceding claim, wherein the library is for expression in a mammalian cell, preferably a human cell; and/or optionally
wherein the library is cloned into a lentiviral vector or a retroviral vector.

14. **A library of peptides** encoded by the library of nucleic acids of any one of claims 1 to 13.

15. **A method of identifying a target protein that modulates a phenotype of a mammalian cell,** said method comprising:
a. exposing a population of in vitro cultured mammalian cells capable of displaying said phenotype to a library of nucleic acids according to any of claims 1-13 or a library of peptides according to claim 14,
b. identifying in said cell population an alteration in said phenotype following said exposure,
c. selection of said cells undergoing the phenotypic change and identifying a peptide encoded by (or a peptide of) such library that alters the phenotype of the cell,
d. providing said peptide and identifying the cellular protein that binds to said peptide, said cellular protein being a target protein that modulates the phenotype of the mammalian cell;
optionally wherein the method includes a further step of identifying a compound that binds to said target protein and displaces or blocks binding of said peptide, wherein the compound modulates the phenotype of a mammalian cell.

16. **Use** of: (a) a library of nucleic acids according to any of claims 1-13; and/or (b) a library of peptides according to claim 14, **to identify a peptide that binds to a target;** optionally
wherein the target is a protein target; and/or optionally
wherein the identified peptide modulates a phenotype of a mammalian cell.

17. **Use** of: (a) a library of nucleic acids according to any of claims 1-13; and/or (b) a library of peptides according to claim 14, **to identify a compound** which binds to a target; optionally
wherein the target is a protein target; and/or optionally
wherein said compound displaces or blocks binding of a peptide to the target; and/or optionally
wherein, the peptide and/or the compound modulates a phenotype of a mammalian cell.

18. A method according to claim 15, or Use according to claim 16 or claim 17, wherein the phenotype is a phenotype related to the modulation of a cell-signalling pathway; optionally
wherein the method or use comprises the identification of peptides which modulate cell-signalling pathways and the identification of protein targets and surface sites on such proteins that participate in signal transduction; and/or optionally
wherein the cell-signalling pathway is active or altered in cancer cells.

## Patentansprüche

1. Bibliothek von Nukleinsäuren, wobei jede Nukleinsäure eine kodierende Region von definierter Nukleinsäuresequenz umfasst, die für ein Peptid kodiert, das eine Länge zwischen 25 und 110 Aminosäuren aufweist, und eine Aminosäuresequenz aufweist, die eine Region einer Sequenz ist, ausgewählt aus der Aminosäuresequenz eines natürlich vorkommenden Proteins von einem oder mehreren Organismen; wobei die Bibliothek Nukleinsäuren umfasst, die für eine Vielzahl von zumindest 10.000 verschiedener solcher Peptide kodieren, und wobei die Aminosäuresequenz von jedem von zumindest 50 solcher Peptide eine Sequenzregion der Aminosäuresequenz eines anderen Proteins aus einer Vielzahl von verschiedenen solchen natürlich vorkommenden Proteinen ist, und wobei für jedes Peptid, das durch die Bibliothek kodiert wird, von seiner Aminosäuresequenz vorhergesagt wird, dass es einen isoelektrischen Punkt (pI) von mehr als 8,0 oder weniger als 6,0 aufweist.

2. Bibliothek von Nukleinsäuren nach Anspruch 1, wobei jedes aus der Vielzahl von verschiedenen natürlich vorkommenden Proteinen ein oder mehrere vorbestimmte Kriterien erfüllt.

3. Bibliothek von Nukleinsäuren nach Anspruch 2, wobei entweder:
i) jedes aus der Vielzahl von natürlich vorkommenden Proteinen mit einer gegebenen Krankheit verbunden ist, wie Krebs; wobei optional die Krankheit Brustkrebs ist; oder
ii) jedes aus der Vielzahl von natürlich vorkommenden Proteinen ein zytoplasmatisches Protein ist; wobei optional jedes aus der Vielzahl von natürlich vorkommenden Proteinen eine zytoplasmatische Kinase ist; oder
iii) jedes aus der Vielzahl von natürlich vorkommenden Proteinen mit einem gegebenen Protein oder zumindest einem Protein aus einer (funktionellen) Klasse von Proteinen interagiert; wobei optional jedes aus der Vielzahl von natürlich vorkommenden Proteinen mit KRas interagiert.

4. Bibliothek von Nukleinsäuren nach einem der Ansprüche 1 bis 3, wobei die Bibliothek Nukleinsäuren umfasst, die für eine Vielzahl von zumindest 50.000 verschiedener solcher Peptide kodieren, und wobei die Aminosäuresequenz von jedem von zumindest 100 solcher Peptide eine Sequenzregion der Aminosäuresequenz von zumindest 100 verschiedenen natürlich vorkommenden Proteinen ist; wobei die Bibliothek insbesondere Nukleinsäuren umfasst, die für eine Vielzahl von zumindest 100.000 verschiedener solcher Peptide kodieren, und wobei die Aminosäuresequenz von jedem von zumindest 150 solcher Peptide eine Sequenzregion der Aminosäuresequenz von zumindest 150 verschiedenen natürlich vorkommenden Proteinen ist.

5. Bibliothek von Nukleinsäuren nach einem der Ansprüche 1 bis 4, wobei die Bibliothek Nukleinsäuren umfasst, die für eine Vielzahl von zumindest 10.000 verschiedenen solcher Peptide kodieren, und wobei die Aminosäuresequenz von jedem von zumindest 1.000 solcher Peptide eine Sequenzregion der Aminosäuresequenz eines anderen Proteins einer solchen Vielzahl von verschiedenen natürlich vorkommenden Proteinen ist.

6. Bibliothek von Nukleinsäuren nach einem der Ansprüche 1 bis 5, wobei die Bibliothek Nukleinsäuren umfasst, die für eine Vielzahl von zumindest 200.000 verschiedener solcher Peptide kodieren, und wobei die Aminosäuresequenz von jedem von zumindest 20.000 solcher Peptide eine Sequenzregion der Aminosäuresequenz von zumindest 20.000 verschiedener natürlich vorkommender Proteine ist; wobei die Bibliothek insbesondere Nukleinsäuren umfasst, die für eine Vielzahl von zumindest 300.000 verschiedener solcher Peptide kodieren, und wobei die Aminosäuresequenz von jedem von zumindest 25.000 solcher Peptide eine Sequenzregion der Aminosäuresequenz von zumindest 25.000 verschiedener natürlich vorkommender Proteine ist.

7. Bibliothek von Nukleinsäuren nach einem der Ansprüche 1 bis 6, wobei in Bezug auf zumindest etwa 1 % der natürlich vorkommenden Proteine eine Vielzahl der Nukleinsäuren für verschiedene Peptide aus den Aminosäuresequenzen solcher natürlich vorkommender Proteine kodiert; wobei optional
in Bezug auf zumindest etwa 50 % der natürlich vorkommenden Proteine eine Vielzahl der Nukleinsäuren für verschiedene Peptide aus den Aminosäuresequenzen solcher natürlich vorkommenden Proteine kodiert;
und wobei optional die Vielzahl der Nukleinsäuren für verschiedene Peptide kodiert und deren Aminosäuresequenzen Sequenzregionen sind, die entlang der Aminosäuresequenz des natürlich vorkommenden Proteins beabstandet sind;
und wobei optional die Sequenzregionen um ein Fenster von Aminosäuren oder um Vielfache eines solchen Fensters entlang der Aminosäuresequenz des natürlich vorkommenden Proteins beabstandet sind, wobei das Fenster zwischen 1 und etwa 55 Aminosäuren ist; wobei insbesondere das Fenster zwischen etwa 5 und etwa 20 Aminosäuren ist; wobei vor allem das Fenster der Beabstandung etwa 8, 10, 12 oder 15 Aminosäuren ist.

8. Bibliothek von Nukleinsäuren nach einem der Ansprüche 1 bis 7, umfassend Nukleinsäuren, die für zumindest 100.000 verschiedene Peptide von zumindest 10.000 verschiedenen natürlich vorkommenden Proteinen kodieren.

9. Bibliothek von Nukleinsäuren nach einem der Ansprüche 1 bis 8, wobei jede Nukleinsäure ein verschiedenes Peptid kodiert.

10. Bibliothek von Nukleinsäuren nach einem der Ansprüche 1 bis 9, wobei die mittlere Anzahl an Nukleinsäuren, die ein verschiedenes Peptid von den natürlich vorkommenden Proteinen kodieren, größer als 1 ist; insbesondere zwischen etwa 1,01 und 1,5 solcher Nukleinsäuren (Peptide) pro solchem Protein; wobei optional
die mittlere Anzahl an Nukleinsäuren, die ein verschiedenes Peptid von den natürlich vorkommenden Proteinen kodieren, zumindest etwa 5 solcher Nukleinsäuren (Peptide) pro solchem Protein ist, wobei insbesondere das Mittel zwischen etwa 5 und etwa 2.000 solcher Nukleinsäuren (Peptide) pro solchem Protein ist oder zwischen etwa 5 und etwa 1.000 Nukleinsäuren (Peptide) pro solchem Protein ist; und wobei optional
die mittlere Anzahl an Nukleinsäuren, die ein verschiedenes Peptid von den natürlich vorkommenden Proteinen kodieren, zwischen etwa 100 und etwa 1.500 solcher Nukleinsäuren (Peptide) pro solchem Protein ist oder zwischen etwa 250 und etwa 1.000 solcher Nukleinsäuren (Peptide) pro solchem Protein ist; oder
wobei die mittlere Anzahl an Nukleinsäuren, die ein verschiedenes Peptid von den natürlich vorkommenden Proteinen kodieren, zwischen etwa 5 und etwa 100 solcher Nukleinsäuren (Peptide) pro solchem Protein ist oder zwischen etwa 5 und etwa 50 solcher Nukleinsäuren (Peptide) pro solchem Protein ist.

11. Bibliothek von Nukleinsäuren nach einem der Ansprüche 1 bis 10, wobei die Aminosäuresequenz des natürlich vorkommenden Proteins eine ausgewählt aus der Gruppe von Aminosäuresequenzen von nicht-redundanten Proteinen ist, die in einem Referenzproteom enthalten sind, geeigneterweise das Referenzproteom eines oder mehrere der Referenzproteome ausgewählt aus der Gruppe von Referenzproteomen ist, die in Tabelle A und/oder Tabelle B aufgelistet sind, oder eine aktualisierte Version eines solchen Referenzproteoms.

12. Bibliothek von Nukleinsäuren nach einem der Ansprüche 1 bis 11, wobei die Aminosäuresequenzen der Vielzahl von kodierten Peptiden Sequenzregionen ausgewählt aus Aminosäuresequenzen von natürlich vorkommenden Proteinen (oder Polypeptidketten oder Domänen davon) mit einer bekannten dreidimensionalen Struktur sind; wobei insbesondere das natürlich vorkommende Protein (oder die Polypeptidkette oder Domäne davon) in der Proteindatenbank enthalten ist, und das optional eine Pfam-Annotation aufweist; wobei optional die Sequenzregion ausgewählt aus der Aminosäuresequenz des Proteins keine mehrdeutige Aminosäure einer solchen Aminosäuresequenz beinhaltet, die in dem Referenzproteom oder der Proteindatenbank enthalten ist.

13. Bibliothek von Nukleinsäuren nach einem vorhergehenden Anspruch, wobei die Bibliothek zur Expression in einer Säugerzelle, bevorzugt einer menschlichen Zelle ist; und/oder wobei optional
die Bibliothek in einen lentiviralen Vektor oder einen retroviralen Vektor kloniert ist.

14. Bibliothek von Peptiden, die durch die Bibliothek von Nukleinsäuren nach einem der Ansprüche 1 bis 13 kodiert werden.

15. Verfahren zum Identifizieren eines Zielproteins, das einen Phänotyp einer Säugerzelle moduliert, wobei das Verfahren Folgendes umfasst:
a. Aussetzen einer Population von in vitro kultivierten Säugerzellen, die dazu in der Lage sind, den Phänotyp anzuzeigen, einer Bibliothek von Nukleinsäuren nach einem der Ansprüche 1-13 oder einer Bibliothek von Peptiden nach Anspruch 14,
b. Identifizieren einer Veränderung des Phänotyps in der Zellpopulation nach der Aussetzung,
c. Auswählen der Zellen, die die phänotypische Veränderung durchmachen, und Identifizieren eines Peptids, das durch eine (oder ein Peptid davon) solche Bibliothek kodiert wird, die den Phänotyp der Zelle verändert,
d. Bereitstellen des Peptids und Identifizieren des zellulären Proteins, das an das Peptid bindet, wobei das zelluläre Protein ein Zielprotein ist, das den Phänotyp der Säugerzelle moduliert;
wobei optional das Verfahren einen weiteren Schritt des Identifizierens einer Verbindung beinhaltet, die an das Zielprotein bindet und die Bindung des Peptids verdrängt oder blockiert, wobei die Verbindung den Phänotyp einer Säugerzelle moduliert.

16. Verwendung von: (a) einer Bibliothek von Nukleinsäuren nach einem der Ansprüche 1-13; und/oder (b) einer Bibliothek von Peptiden nach Anspruch 14, um ein Peptid zu identifizieren, das an ein Ziel bindet; wobei optional
das Ziel ein Proteinziel ist; und/oder wobei optional
das identifizierte Peptid einen Phänotyp einer Säugerzelle moduliert.

17. Verwendung von: (a) einer Bibliothek von Nukleinsäuren nach einem der Ansprüche 1-13; und/oder (b) einer Bibliothek von Peptiden nach Anspruch 14, um eine Verbindung zu identifizieren, die an ein Ziel bindet; wobei optional
das Ziel ein Proteinziel ist; und/oder wobei optional
die Verbindung die Bindung eines Peptids an das Ziel verdrängt oder blockiert; und/oder wobei optional das Peptid und/oder die Verbindung einen Phänotyp einer Säugerzelle moduliert.

18. Verfahren nach Anspruch 15 oder Verwendung nach Anspruch 16 oder Anspruch 17, wobei der Phänotyp ein Phänotyp ist, der sich auf die Modulation eines Zellsignalweges bezieht; wobei optional
das Verfahren oder die Verwendung die Identifizierung von Peptiden, die Zellsignalwege modulieren, und die Identifizierung von Proteinzielen und Oberflächenstellen auf solchen Proteinen umfasst, die an Signalübertragung teilnehmen; und/oder wobei optional
der Zellsignalweg in Krebszellen aktiv oder verändert ist.

## Revendications

1. Banque d'acides nucléiques, chaque acide nucléique comprenant une région codante d'une séquence d'acide nucléique définie qui code un peptide présentant une longueur comprise entre 25 et 110 acides aminés, et présentant une séquence d'acides aminés qui est une région d'une séquence choisie parmi la séquence d'acides aminés d'une protéine naturelle d'un ou plusieurs organismes ; ladite banque comprenant des acides nucléiques qui codent une pluralité d'au moins 10 000 dits peptides différents, et ladite séquence d'acides aminés de chacun d'au moins 50 desdits peptides étant une région de séquence de la séquence d'acides aminés d'une protéine différente parmi une pluralité desdites protéines naturelles différentes, et dans laquelle, pour chaque peptide codé par la banque, on prévoit à partir de sa séquence d'acides aminés qu'il possède un point isoélectrique (pI) supérieur à 8,0 ou inférieur à 6,0.

2. Banque d'acides nucléiques selon la revendication 1, chacune de la pluralité de protéines naturelles différentes remplissant un ou plusieurs critères prédéfinis.

3. Banque d'acides nucléiques selon la revendication 2, soit :
i) chacune de la pluralité de protéines naturelles étant associée à une maladie donnée, tel qu'un cancer ; éventuellement ladite maladie étant le cancer du sein ; soit
ii) chacune de la pluralité de protéines naturelles étant une protéine cytoplasmique ; éventuellement chacune de la pluralité de protéines naturelles étant une kinase cytoplasmique ; soit
iii) chacune de la pluralité de protéines naturelles interagissant avec une protéine donnée ou au moins une protéine parmi une catégorie (fonctionnelle) de protéines ; éventuellement chacune de la pluralité de protéines naturelles interagissant avec KRas.

4. Banque d'acides nucléiques selon l'une quelconque des revendications 1 à 3, ladite banque comprenant des acides nucléiques qui codent une pluralité d'au moins 50 000 dits peptides différents, et ladite séquence d'acides aminés de chacun d'au moins 100 desdits peptides étant une région de séquence de la séquence d'acides aminés d'au moins 100 protéines naturelles différentes ; en particulier ladite banque comprenant des acides nucléiques qui codent une pluralité d'au moins 100 000 dits peptides différents, et ladite séquence d'acides aminés de chacun d'au moins 150 dudit peptide étant une région de séquence de la séquence d'acides aminés d'au moins 150 protéines naturelles différentes.

5. Banque d'acides nucléiques selon l'une quelconque des revendications 1 à 4, ladite banque comprenant des acides nucléiques qui codent une pluralité d'au moins 10 000 dits peptides différents, et ladite séquence d'acides aminés de chacun d'au moins 1 000 desdits peptides étant une région de séquence de la séquence d'acides aminés d'une protéine différente de ladite pluralité de protéines naturelles différentes.

6. Banque d'acides nucléiques selon l'une quelconque des revendications 1 à 5, ladite banque comprenant des acides nucléiques qui codent une pluralité d'au moins 200 000 dits peptides différents, et ladite séquence d'acides aminés de chacun d'au moins 20 000 desdits peptides étant une région de séquence de la séquence d'acides aminés d'au moins 20 000 protéines naturelles différentes ; en particulier ladite banque comprenant des acides nucléiques qui codent une pluralité d'au moins 300 000 dits peptides différents, et ladite séquence d'acides aminés de chacun d'au moins 25 000 dudit peptide étant une région de séquence de la séquence d'acides aminés d'au moins 25 000 protéines naturelles différentes.

7. Banque d'acides nucléiques selon l'une quelconque des revendications 1 à 6, en ce qui concerne au moins environ 1 % des protéines naturelles une pluralité des acides nucléiques codant différents peptides des séquences d'acides aminés desdites protéines naturelles ; éventuellement
en ce qui concerne au moins environ 50 % des protéines naturelles une pluralité des acides nucléiques codant différents peptides des séquences d'acides aminés desdites protéines naturelles ;
et éventuellement ladite pluralité des acides nucléiques codant différents peptides, et les séquences d'acides aminés desquels étant des régions de séquence espacées le long de la séquence d'acides aminés de la protéine naturelle ;
et éventuellement lesdites régions de séquence étant espacées par une fenêtre d'acides aminés, ou par de multiples dites fenêtres, le long de la séquence d'acides aminés de la protéine naturelle, ladite fenêtre faisant entre 1 et environ 55 acides aminés ; en particulier ladite fenêtre faisant entre environ 5 et environ 20 acides aminés ; plus particulièrement ladite fenêtre d'espacement faisant environ 8, 10, 12 ou 15 acides aminés.

8. Banque d'acides nucléiques selon l'une quelconque des revendications 1 à 7 comprenant des acides nucléiques qui codent au moins 100 000 peptides différents d'au moins 10 000 protéines naturelles différentes.

9. Banque d'acides nucléiques selon l'une quelconque des revendications 1 à 8, chaque acide nucléique codant un peptide différent.

10. Banque d'acides nucléiques selon l'une quelconque des revendications 1 à 9, le nombre moyen d'acides nucléiques qui codent un peptide différent des protéines naturelles étant supérieur à 1 ; en particulier compris entre environ 1,01 et 1,5 dits acides nucléiques (peptides) par dite protéine ; éventuellement
ledit nombre moyen d'acides nucléiques qui codent un peptide différent des protéines naturelles étant au moins environ 5 dits acides nucléiques (peptides) par dite protéine, en particulier la moyenne étant comprise entre environ 5 et environ 2 000 dits acides nucléiques (peptides) par dite protéine ou étant compris entre environ 5 et environ 1 000 dits acides nucléiques (peptides) par dite protéine ; et éventuellement
ledit nombre moyen d'acides nucléiques qui codent un peptide différent des protéines naturelles étant compris entre environ 100 et environ 1 500 dits acides nucléiques (peptides) par dite protéine ou étant compris entre environ 250 et environ 1 000 dits acides nucléiques (peptides) par dite protéine ; ou
ledit nombre moyen d'acides nucléiques qui codent un peptide différent des protéines naturelles étant compris entre environ 5 et environ 100 dits acides nucléiques (peptides) par dite protéine ou étant compris entre environ 5 et environ 50 dits acides nucléiques (peptides) par dite protéine.

11. Banque d'acides nucléiques selon l'une quelconque des revendications 1 à 10, ladite séquence d'acides aminés de la protéine naturelle étant l'une choisie dans le groupe de séquences d'acides aminés de protéines non redondantes comprises dans un protéome de référence, de manière appropriée le protéome de référence étant un ou plusieurs protéomes de référence parmi les protéomes de référence choisis dans le groupe des protéomes de référence énumérés dans le tableau A et/ou le tableau B, ou une version mise à jour dudit protéome de référence.

12. Banque d'acides nucléiques selon l'une quelconque des revendications 1 à 11, lesdites séquences d'acides aminés de la pluralité de peptides codés étant des régions de séquence choisies parmi des séquences d'acides aminés de protéines naturelles (ou de chaînes ou domaines polypeptidiques de celles-ci) avec une structure tridimensionnelle connue ; en particulier ladite protéine naturelle (ou chaîne ou domaine polypeptidique de celle-ci) étant comprise dans la Protein Data Bank, et éventuellement ayant une annotation Pfam ; éventuellement
ladite région de séquence choisie parmi la séquence d'acides aminés de la protéine n'incluant pas d'acide aminé ambigu de cette séquence d'acides aminés comprise dans le protéome de référence ou dans la Protein Data Bank.

13. Banque d'acides nucléiques selon l'une quelconque des revendications précédentes, ladite banque étant destinée à l'expression dans une cellule mammifère, de préférence une cellule humaine ; et/ou éventuellement
ladite banque étant clonée dans un vecteur lentiviral ou un vecteur rétro viral.

14. Banque de peptides codée par la banque d'acides nucléiques selon l'une quelconque des revendications 1 à 13.

15. Méthode d'identification d'une protéine cible qui module un phénotype d'une cellule mammifère, ladite méthode comprenant :
a. l'exposition d'une population de cellules mammifères cultivées in vitro capables de présenter ledit phénotype à une banque d'acides nucléiques selon l'une quelconque des revendications 1 à 13 ou d'une banque de peptides selon la revendication 14,
b. l'identification dans ladite population de cellules d'une modification dans ledit phénotype suite à ladite exposition,
c. la sélection desdites cellules qui subissent le changement phénotypique et l'identification d'un peptide codé par (ou un peptide de) ladite banque qui modifie le phénotype de la cellule,
d. la fourniture dudit peptide et l'identification de la protéine cellulaire qui se lie audit peptide, ladite protéine cellulaire étant une protéine cible qui module le phénotype de la cellule mammifère ;
éventuellement ladite méthode comprenant une étape supplémentaire d'identification d'un composé qui se lie à ladite protéine cible et déplace ou bloque la liaison dudit peptide, ledit composé modulant le phénotype d'une cellule mammifère.

16. Utilisation : (a) d'une banque d'acides nucléiques selon l'une quelconque des revendications 1 à 13 ; et/ou (b) d'une banque de peptides selon la revendication 14, pour identifier un peptide qui se lie à une cible ; éventuellement
ladite cible étant une cible protéique ; et/ou éventuellement
ledit peptide identifié modulant un phénotype d'une cellule mammifère.

17. Utilisation : (a) d'une banque d'acides nucléiques selon l'une quelconque des revendications 1 à 13 ; et/ou (b) d'une banque de peptides selon la revendication 14, pour identifier un composé qui se lie à une cible ; éventuellement
ladite cible étant une cible protéique ; et/ou éventuellement
ledit composé déplaçant ou bloquant la liaison d'un peptide à la cible ; et/ou éventuellement,
ledit peptide et/ou ledit composé modulant un phénotype d'une cellule mammifère.

18. Procédé selon la revendication 15, ou utilisation selon la revendication 16 ou 17, ledit phénotype étant un phénotype associé à la modulation d'une voie de signalisation cellulaire ; éventuellement
ladite méthode ou utilisation comprenant l'identification de peptides qui modulent des voies de signalisation cellulaire et l'identification de cibles protéiques et de sites de surface sur lesdites protéines qui participent à la transduction de signaux ; et/ou éventuellement
ladite voie de signalisation cellulaire étant active ou modifiée dans des cellules cancéreuses.


### SEQUENCE LISTING

<110> Phoremost Limited
<120> Nucleic acid libraries, peptide libraries and uses thereof
<130> P9328WO2
<160> 16
<170> PatentIn version 3.5
<210> 1
<210> 1
   <211> 20
   <212> DNA
   <213> Artificial Sequence
<220>
   <223> Forward amplification sequence used in Example 1
<400> 1
   tgccacctga cgtctaagaa 20
<210> 2
   <211> 20
   <212> DNA
   <213> Artificial Sequence
<220>
   <223> Reverse amplification sequence used in Example 1
<400> 2
   gctcactcaa aggcggtaat 20
<210> 3
   <211> 199
   <212> DNA
   <213> Artificial Sequence
<220>
   <223> Indicative oligonucleotide shown in Example 2
<220>
   <221> misc_feature
   <222> (1)..(20)
   <223> Forward amplification sequence
<220>
   <221> misc_feature
   <222> (21)..(26)
   <223> Restriction enzyme site
<220>
   <221> misc_feature
   <222> (25)..(30)
   <223> Kozak sequence
<220>
   <221> misc_feature
   <222> (33)..(170)
   <223> Coding region
<220>
   <221> misc_feature
   <222> (174)..(179)
   <223> Restriction enzyme site
<220>
   <221> misc_feature
   <222> (180)..(199)
   <223> Backward amplification sequences
<400> 3
   tgccacctga cgtctaagaa ggatccatgg gactcgccca gaccgcctgt gtggtgggca      60
   ggcccggccc ccaccccacc cagttcctcg ccgccaagga aaggaccaag agccacgtgc     120
   ccagcctcct cgacgccgac gtggaaggcc agagcaggga ctacaccgtg taactcgagg     180
   ctcactcaaa ggcggtaat                                                  199
<210> 4
   <211> 20
   <212> DNA
   <213> Artificial Sequence
<220>
   <223> Forward primer used in Example 3
<400> 4
   tgccacctga cgtctaagaa 20
<210> 5
   <211> 20
   <212> DNA
   <213> Artificial Sequence
<220>
   <223> Reverse primer used in Example 3
<400> 5
   attaccgcct ttgagtgagc 20
<210> 6
   <211> 60
   <212> DNA
   <213> Artificial Sequence
<220>
   <223> Cloning site of pMOST25 lentiviral vector
<220>
   <221> misc_feature
   <222> (20)..(25)
   <223> Restriction enzyme site
<220>
   <221> misc_feature
   <222> (38)..(43)
   <223> Restriction enzyme site
<400> 6
   agtagcatcg cattagccgg gatccagcgc tgctaccctc gagtaagtga ctaggcaatc 60
<210> 7
   <211> 195
   <212> DNA
   <213> Artificial Sequence
<220>
   <223> Indicative recombinant construct shown in Example 3
<220>
   <221> misc_feature
   <222> (20)..(25)
   <223> Restriction enzyme site
<220>
   <221> misc_feature
   <222> (24)..(29)
   <223> Kozak sequence
<220>
   <221> misc_feature
   <222> (32)..(169)
   <223> Coding region
<220>
   <221> misc_feature
   <222> (173)..(178)
   <223> Restriction enzyme site
<400> 7
   agtagcatcg cattagccgg gatccatggg actcgcccag accgcctgtg tggtgggcag       60
   gcccggcccc caccccaccc agttcctcgc cgccaaggaa aggaccaaga gccacgtgcc      120
   cagcctcctc gacgccgacg tggaaggcca gagcagggac tacaccgtgt aactcgagta      180
   agtgactagg caatc                                                       195
<210> 8
   <211> 48
   <212> PRT
   <213> Artificial Sequence
<220>
   <223> Indicative expressed peptide shown in Example 3
<400> 8
   Met Gly Leu Ala Gln Thr Ala Cys Val Val Gly Arg Pro Gly Pro His
   1               5                   10                  15
   Pro Thr Gln Phe Leu Ala Ala Lys Glu Arg Thr Lys Ser His Val Pro
               20                  25                  30
   Ser Leu Leu Asp Ala Asp Val Glu Gly Gln Ser Arg Asp Tyr Thr Val
           35                  40                  45
<210> 9
   <211> 193
   <212> DNA
   <213> Artificial Sequence
<220>
   <223> Indicative oligonucleotide shown in Example 5
<220>
   <221> misc_feature
   <222> (1)..(20)
   <223> Forward amplification sequences
<220>
   <221> misc_feature
   <222> (21)..(26)
   <223> Restriction enzyme site
<220>
   <221> misc_feature
   <222> (30)..(167)
   <223> Coding region
<220>
   <221> misc_feature
   <222> (168)..(173)
   <223> Restriction enzyme site
<220>
   <221> misc_feature
   <222> (174)..(193)
   <223> Reverse amplification sequence
<400> 9
   tgccacctga cgtctaagaa ggatccgtgc ctaggtacct gaagggctgg ctgaaggacg      60
   tggtgcagct gagcctgagg aggcctagct tcagggccag caggcagagg cctatcatca     120
   gcctgaacga gaggattctg gagttcaaca agaggaacat cacagccctc gaggctcact     180
   caaaggcggt aat                                                        193
<210> 10
   <211> 60
   <212> DNA
   <213> Artificial Sequence
<220>
   <223> Cloning site of pMOST25A lentiviral vector
<220>
   <221> misc_feature
   <222> (7)..(12)
   <223> Kozak sequence
<220>
   <221> misc_feature
   <222> (12)..(17)
   <223> Restriction enzyme site
<220>
   <221> misc_feature
   <222> (30)..(35)
   <223> Restriction enzyme site
<400> 10
   cattagccat gggatccagc gctgctaccc tcgagtaagt gactaggcaa tctaatctat 60
<210> 11
   <211> 187
   <212> DNA
   <213> Artificial Sequence
<220>
   <223> Indicative recombinant construct shown in Example 5
<220>
   <221> misc_feature
   <222> (7)..(12)
   <223> Kozak sequence
<220>
   <221> misc_feature
   <222> (12)..(17)
   <223> Restriction enzyme site
<220>
   <221> misc_feature
   <222> (21)..(158)
   <223> Coding region
<220>
   <221> misc_feature
   <222> (159)..(164)
   <223> Restriction enzyme site
<400> 11
   cattagccat gggatccgtg cctaggtacc tgaagggctg gctgaaggac gtggtgcagc 60
   tgagcctgag gaggcctagc ttcagggcca gcaggcagag gcctatcatc agcctgaacg 120
   agaggattct ggagttcaac aagaggaaca tcacagccct cgagtaagtg actaggcaat 180
   ctaatct 187
<210> 12
   <211> 52
   <212> PRT
   <213> Artificial Sequence
<220>
   <223> Indicative expressed peptide shown in Example 5
<400> 12
   Met Gly Ser Val Pro Arg Tyr Leu Lys Gly Trp Leu Lys Asp Val Val
   1               5                   10                  15
   Gln Leu Ser Leu Arg Arg Pro Ser Phe Arg Ala Ser Arg Gln Arg Pro
               20                  25                  30
   Ile Ile Ser Leu Asn Glu Arg Ile Leu Glu Phe Asn Lys Arg Asn Ile
           35                  40                  45
   Thr Ala Leu Glu
       50
<210> 13
   <211> 46
   <212> PRT
   <213> Artificial Sequence
<220>
   <223> Indicative peptide described in Example 2
<400> 13
   Leu Ala Gln Thr Ala Cys Val Val Gly Arg Pro Gly Pro His Pro Thr
   1               5                   10                  15
   Gln Phe Leu Ala Ala Lys Glu Arg Thr Lys Ser His Val Pro Ser Leu
               20                  25                  30
   Leu Asp Ala Asp Val Glu Gly Gln Ser Arg Asp Tyr Thr Val
           35                  40                  45
<210> 14
   <211> 46
   <212> PRT
   <213> Artificial Sequence
<220>
   <223> Indicative peptide described in Example 5
<400> 14
   Pro Arg Tyr Leu Lys Gly Trp Leu Lys Asp Val Val Gln Leu Ser Leu
   1               5                   10                  15
   Arg Arg Pro Ser Phe Arg Ala Ser Arg Gln Arg Pro Ile Ile Ser Leu
               20                  25                  30
   Asn Glu Arg Ile Leu Glu Phe Asn Lys Arg Asn Ile Thr Ala
           35                  40                  45
<210> 15
   <211> 48
   <212> PRT
   <213> Artificial Sequence
<220>
   <223> #30-325 hit peptide from Example 4 PTEN synthetic lethality screen
<400> 15
   Met Gly Val Val Ile Ile Ala Val Gly Ala Leu Leu Phe Ile Ile Gly
   1               5                   10                  15
   Leu Ile Gly Cys Cys Ala Thr Ile Arg Glu Ser Arg Cys Gly Leu Ala
               20                  25                  30
   Thr Phe Val Ile Ile Leu Leu Leu Val Phe Val Thr Glu Val Val Val
           35                  40                  45
<210> 16
   <211> 199
   <212> DNA
   <213> Artificial Sequence
<220>
   <223> Oligonucleotide encoding for the #30-325 hit peptide
<400> 16
   tgccacctga cgtctaagaa ggatccatgg gagtggtgat tattgccgtg ggcgccctcc     60
   tcttcattat tggcctcatt ggctgttgtg ccaccattag ggaaagcagg tgtggcctcg    120
   ccaccttcgt gattattctc ctcctcgtgt tcgtgaccga agtggtggtg taactcgagg    180
   ctcactcaaa ggcggtaat                                                 199